# EUROPEAN PATENT SPECIFICATION

(11) **EP 4 192 467 B1**
(45) Date of publication and mention of the grant of the patent: **26.08.2026**
(21) Application number: 21798430.1
(22) Date of filing: 05.08.2021
(51) Int. Cl.: A61K 31/519, A61K 31/555, A61P 35/00, C07D 401/14, C07F 17/02, C07D 471/14

(54) **SYNTHESIS OF NOVEL IMIPRIDONE DERIVATIVES AND THEIR EVALUATION FOR THEIR ANTICANCER ACTIVITY**
SYNTHESE VON NEUEN IMIPRIDONDERIVATEN UND IHRE BEURTEILUNG AUF IHRE ANTIKREBSWIRKUNG
SYNTHÈSE DE NOUVEAUX DÉRIVÉS D'IMIPRIDONE ET LEUR ÉVALUATION CONCERNANT LEUR ACTIVITÉ ANTICANCÉREUSE

(30) Priority: 06.08.2020 HU 2000255
(43) Date of publication of application: 14.06.2023
(73) Proprietor: Eötvös Loránd Tudományegyetem, 1053 Budapest (HU); Semmelweis Egyetem, 1085 Budapest (HU)
(72) Inventor: CSÁMPAI, Antal, 1211 Budapest (HU); BÁRÁNY, Péter, 2119 Pécel (HU); CZUCZI, Tamás, 4031 Debrecen (HU); KOVÁCS, Imre, 4034 Debrecen (HU); ADAMIS, Bálint, 1041 Budapest (HU); NÉMETH, Zsófia, 1183 Budapest (HU); MURÁNYI, József, 2463 Tordas (HU); OLÁHNÉ SZABÓ, Rita, 1135 Budapest (HU); BÖSZE, Szilvia, 1124 Budapest (HU); MEZÖ, Gábor, 1087 Budapest (HU); KÖHIDAI, László, 1125 Budapest (HU); LAJKÓ, Eszter, 1097 Budapest (HU); TAKÁCS, Angéla, 8000 Székesfehérvár (HU); LÁNG, Orsolya, 1125 Budapest (HU); MEZÖ, Diána, 1097 Budapest (HU)
(74) Representative: Szentpéteri, Zsolt
(86) International application number: PCT/HU2021/050047
(87) International publication number: WO 2022/029459

(56) References cited:
- EP-A1- 3 315 498
- WO-A1-2016/123571
- WO-A1-2018/031987

## Description

### FIELD OF THE INVENTION

The present invention relates to compounds of formula (**I**) 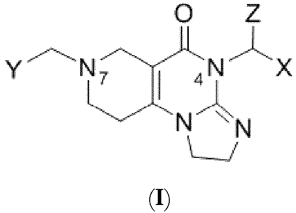 or pharmaceutically acceptable salts and stereoisomers thereof, including single enantiomers, racemic mixtures, mixtures of enantiomers, or combinations thereof, which are applicable for use in treating cancer diseases. The present invention further relates to a pharmaceutical composition comprising the above compounds.

### BACKGROUND OF THE INVENTION

Imipridones, a first-in-class small molecule anti-cancer compounds, comprising an angular tricyclic heterocyclic framework of enhanced basicity with two skeletal nitrogen atoms carrying substituents [cf. general structure represented by formula (**I**)]. This well-defined angularly condensed skeletal structure, comprising suitably positioned ideal number of basic centers and lactam moiety with pending tunable aromatic rings as evenly distributed potential binding sites, render multitargeting character and ideal drug properties to imipridones being essential to their distinct mechanisms of action. Specific G protein-coupled receptors (GPCRs) controlling critical signaling pathways in cancer cells are important targets of ONC201 (Reference Compound 1), the first imipridone implicated in clinical development directly antagonizing a GPCR called DRD2 dopamine receptor [1]. 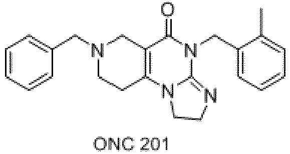 Reference Compound 1

This molecule has also emerged as an efficient activator of the proapoptotic protein tumor necrosis factor-related apoptosis-inducing ligand (TRAIL) and its receptors with a wide therapeutic index [2-4]. *Kline et al.* disclosed that ONC201 (Reference Compound 1) triggered dual inhibition of AKT and extracellular signal-regulated kinase (ERK) pathways in a number of malignant cell lines (e.g. HCT-116, HEPG-2, MCF-7, and MDA-MB-468) and demonstrated that, besides apoptosis measured by sub-G1 fraction and caspase activation, ONC201 (Reference Compound 1) also induced cell cycle arrest in the cell lines which were tested as early as 24 h after treatment [2].

The forgoing cited references [1-4] regard to only ONC201 (Reference Compound 1), and do not disclose other imipridone derivatives.

By means of bromodeoxyuridine (BrdU) labelling experiments the authors also confirmed that the proliferation of the cells was inhibited by ONC201 (Reference Compound 1) and, as a response to the treatment with this imipridone, the early cell cycle arrest caused a significant decrease in a number of viable cells within 48 h, even including those (e.g. A-549 and SNV-449) that did not undergo apoptosis [2]. Preclinical studies have demonstrated its potency as an exceptionally promising apoptotic anticancer agent having pronounced activity against a large variety of cancer cell lines (including e.g. PANC-1, HCT116, MDA-MB-23, U87, HFF, MRC5 and WI-38) [5-8]. Moreover, in phase II clinical trials this compound has proved to be beneficial in the treatment of patients with a wide range of advanced malignances [9].

With respect to the above mentioned references [5-9] it can also be stated that they relate to only ONC201 (Reference Compound 1) and do not disclose other imipridone derivatives. Besides the ONC201 (Reference Compound 1), *Wagner et al.* discloses an isomer of ONC201 (Reference Compound 1), which isomer has a linear [4,3-d] structure, therefore said isomer is not covered by the present invention [5]. *Zhe-Zhu Jin et al.* disclose the uses of ONC201 (Reference Compound 1) in combination with AZD-8055, which is a pyrido[2,3-d]pyrimidine derivative, therefore it does not covered by the present invention [7].

An intense search for analogues [10] identified a trifluoromethylated derivative named as ONC212 (Reference Compound 2) as a more potent imipridone with intensified and selective involvement in GPCR targeting and in tumor cell death. 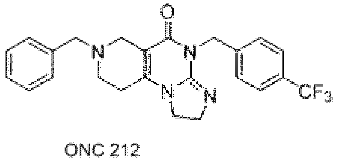 Reference Compound 2

This compound produced significantly enhanced activity at nanomolar concentrations against a number of different malignant cell lines, solid tumours, and hematological malignancies [10]. The cited reference [10] does not disclose such ONC201 (Reference Compound 1) derivatives, which has substituted benzyl group on the N-7 position.

It is also of importance that ONC212 (Reference Compound 2) showed improved preclinical efficacy on pancreatic cancer, melanoma, and hepatocellular carcinoma in a few *in vivo* models including ONC201 (Reference Compound 1)-resistant tumours, e.g. PANC-1 and Capan-2 human pancreatic cancer xenograft models [11]. Said reference [11] regards to only ONC201 (Reference Compound 1) and its derivatives ONC212 (Reference Compound 2) and does not disclose other impiridones. *Graves et al.* demonstrated that ONC201 (Reference Compound 1) and some related analogues are highly potent activators of ClpP [12]. The cited reference [12] does not disclose ONC201 (Reference Compound 1) derivatives having di- or trisubstituted benzyl group on the N-7 position.

Since convincing preclinical evidences have been disclosed about the interplay between TRAIL and redox signalling pathways implicated in cancer [17], the inventors of the present invention in an earlier work [21] attempted to identify antiproliferative imipridones with ferrocene-containing substituents capable of generating reactive oxygen species (ROS) such as nitric oxide, superoxide anion and other forms of free radicals [18, 19] that have been shown to be involved in biological regulatory processes leading to programmed cell death (apoptosis) [20]. Based on the reference [21], starting from ferrocene-containing primary amines and a selection of benzylamines a small library of ferrocene-containing derivatives and purely organic analogues were synthesized and tested, including ONC201 (Reference Compound 1) and ONC212 (Reference Compound 2) to serve as reference models without the potential of ROS-generation [21]. The results of the *in vitro* antiproliferative tests indicate that - although the organometallic imipridones, particularly the compounds with two ferrocene units (e.g. **7de**), display marked cytotoxicity on human malignant cell lines HT-29, HEPG2, PANC1, COLO205, A2058 and EBC1, comparable to that of ONC201 (Reference Compound 1) - their effects are substantially smaller than those exerted by ONC212 (Reference Compound 2). On the other hand, the effects of organic imipridones **7ah** and **7ai** proved to be comparable to those of ONC212 (Reference Compound 2) [21].

U.S. patent No. US 10,239,877 discloses a new class of 4,7-benzyl-substituted imipridone derivatives, including the lead compound known as ONC201 (Reference Compound 1) and some another substituted analogues thereof. The disclosed compounds are potent TRAIL inducers which can be used in cancer therapy.

U.S. patent No. US 9,376,437 discloses new substituted imipridone derivatives of the following formula (Reference Formula 1) 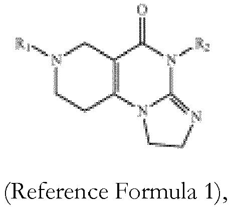 wherein R₁ and R₂ independently represent hydrogen, alkyl, cycloalkyl, cycloalkylalkyl, carboxyl, haloalkyl, alkenyl, cycloalkenyl, alkynyl, aryl, aralkyl, hydroxyalkyl, alkoxy, aryloxy, alkoxyalkyl, alkoxycarbonyl, aralkoxy, aralkylthio, alkanoyl, mercapto, alkylthio, arylthio, alkylsulfinyl, arylsulfinyl, alkylsulfonyl, arylsulfonyl, heteroaryl, acyl, and heterocycle radicals, and wherein when R₁ represents CH₂-Ph, R₂ does not represent CH₂-(2-CH₃-Ph). In the preferred compounds
R₁ is CH₂-Ph and R₂ is CH₂-(2-Cl-Ph),
R₁ is CH₂-Ph and R₂ is CH₂-(2-thienyl),
R₁ is CH₂-Ph and R₂ is CH₂CH₂-Ph,
R₁ is CH₂-Ph and R₂ is CH₂CH₂-(4-N-benzyl-piperazine),
R₁ is CH₂-Ph and R₂ is CH₂-(2,4-di-F-Ph),
R₁ is H and R₂ is CH₂-(2-CH₃-Ph),
R₁ is CH₃ and R₂ is CH₂-(2-CH₃-Ph),
R₁ is CH₂CH₂-Ph and R₂ is CH₂-(2-CH₃-Ph).
U.S. patent No. US 9,843,324 discloses other new substituted imipridone derivatives of above Reference Formula 1, wherein R₁ is selected from the group consisting of H, alkyl, alkylphenyl, alkylphenylketone, benzyl piperazine, alkylthienyl, alkylpyridinyl, alkylisoxazolidinyl, alkylmorpholinyl, alkylthiazolyl and alkylpyrazinyl, wherein alkyl, alkylphenyl, alkylphenylketone, benzyl piperazine, alkylthienyl, alkylpyridinyl, alkylisoxazolidinyl, alkylmorpholinyl, alkylthiazolyl and alkylpyrazinyl are optionally substituted with alkyl, alkoxyl, hydroxyl, perhalogenated alkyl or halogen, and wherein R₂ is a substituted or an unsubstituted heterocycloalkylalkyl, preferably a morpholinoalkyl or a piperazinylalkyl group, or wherein R₂ is a substituted heteroarylalkyl, preferables a pyridylalkyl or isoxazolidinylalkyl group. Furthermore, the cited document discloses the compounds of Reference Formula 2 and Reference Formula 3, as defined in the cited document: 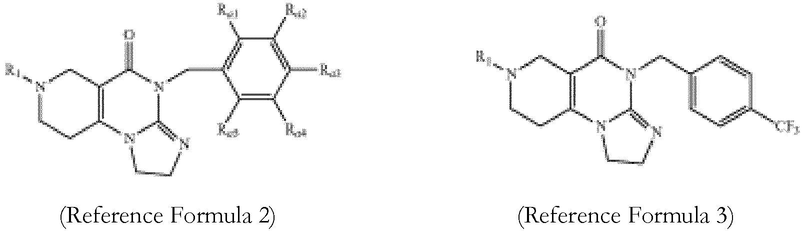
U.S. patent application No. US 2016/0264574 discloses new substituted imipridone derivatives of above Reference Formula 1, wherein R₁ and R₂ independently represent hydrogen, alkyl, cycloalkyl, cycloalkylalkyl, carboxyl, haloalkyl, alkenyl, cycloalkenyl, alkynyl, aryl, aralkyl, hydroxyalkyl, alkoxy, aryloxy, alkoxyalkyl, alkoxycarbonyl, aralkoxy, aralkylthio, alkanoyl, mercapto, alkylthio, arylthio, alkylsulfinyl, arylsulfinyl, alkylsulfonyl, arylsulfonyl, heteroaryl, acyl, and heterocycle radicals, and wherein when R₁ represents CH₂-Ph, R₂ does not represent CH₂-(2-CH₃-Ph). In the preferred compounds
R₁ is CH₂-Ph and R₂ is CH₂-(2-Cl-Ph),
R₁ is CH₂-Ph and R₂ is CH₂-(2-thienyl),
R₁ is CH₂-Ph and R₂ is CH₂CH₂-Ph,
R₁ is CH₂-Ph and R₂ is CH₂CH₂-(4-N-benzyl-piperazine),
R₁ is CH₂-Ph and R₂ is CH₂-(2,4-di-F-Ph),
R₁ is H and R₂ is CH₂-(2-CH₃-Ph),
R₁ is CH₃ and R₂ is CH₂-(2-CH₃-Ph),
R₁ is CH₂CH₂-Ph and R₂ is CH₂-(2-CH₃-Ph),
R₁ is CH₂CH₂NHCOOC(CH₃)₃ and R₂ is CH₂-(2-CH₃-Ph),
R₁ is CH₂CH₂CH₂NH₂ and R₂ is CH₂-(2-CH₃-Ph).

U.S patent No. US 10,266,333 discloses substituted imipridone derivatives of above formulae (Reference Formula 1, Reference Formula 2 and Reference Formula 3), where in the Reference Formula 1 R₁ and R₂ are independently selected from the group consisting of H, alkyl, alkylphenyl, alkylphenylketone, benzyl piperazine, alkylthienyl, alkylpyridinyl, alkylisoxazolidinyl, alkylmorpholinyl, alkylthiazolyl and alkylpyrazinyl, wherein alkyl, alkylphenyl, alkylphenylketone, benzyl piperazine, alkylthienyl, alkylpyridinyl, alkylisoxazolidinyl, alkylmorpholinyl, alkylthiazolyl and alkylpyrazinyl are optionally substituted with alkyl, alkoxy, hydroxyl, perhalogenated alkyl or halogen, and wherein R₂ is a substituted or an unsubstituted heteroarylalkyl; or where in the Reference Formula 2 R₁ is a hydrogen; and Rₐ₁, Rₐ₂, Rₐ₃, Rₐ₄ and Rₐ₅ are each independently selected from the group consisting of hydrogen, X, -CH₃, -NO₂, -OCH₃, -CN, -CXH₂, -CX₂H, C₂-C₄ alkyl, -CX₃,-CH₂(CX₃), -CH(CX₃)₂, -C(CX₃)₃, -CₚX₂ₚ₊₁, -OCX3, -OCₚH₂ₚ₊₁, -OCₚX₂ₚ₊₁, ORₘ, SRₘ, NRₘRₙ, NRₘC(O)Rₙ, SORₘ, SO₂Rₘ, C(O)Rₘ and C(O)ORₘ; where Rₘ and Rₙ are independently selected from hydrogen or a C₁-C₄ alkyl; and X represents a halogen; or derivatives of Reference Formula 4 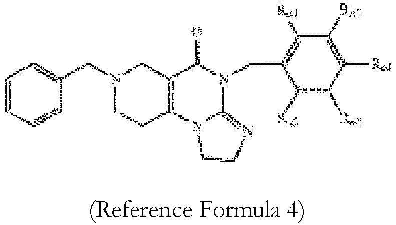 wherein Rₐ₁, Rₐ₄ and Rₐ₅ are each hydrogen; and Rₐ₂ and Rₐ₁ are each chlorine, or
Rₐ₁, Rₐ₃ and Rₐ₅ are each hydrogen; and wherein Rₐ₂ and Rₐ₄ are fluorine, or
Rₐ₂, Rₐ₄ and Rₐ₅ are each hydrogen; and wherein Rₐ₁ and Rₐ₃ are chlorine, or
Rₐ₂, Rₐ₄ and Rₐ₅ are each hydrogen; and wherein Rₐ₁ is a methyl and Rₐ₃ is a fluorine, or
Rₐ₂, Rₐ₄ and Rₐ₅ are each hydrogen; and wherein Rₐ₁ is a fluorine and Rₐ₃ is a CF₃.
U.S. patent No. US 2019/0194201 is a divisional application of U.S patent No. US 10,266,333 and discloses essentially the same substituted imipridone derivatives as its parent application.
U.S. patent No. US 2018/0141946 discloses imidazole pyrimidine ketones of Reference Formula 5 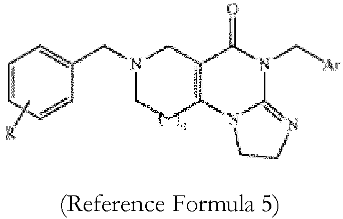 wherein n=0 or 1; R is selected from the group consisting of H, mono- or multi halos, C₁-C₆ alkyl, C₁-C₆ alkoxy, halo-substituted C₁-C₆ alkyl, hetero substituent such as nitrogen or oxygen and six-membered heterocyclic ring with zero, one or two hetero atom substitutions; Ar is selected from the group consisting of mono- or di-substituted aryl groups, with at least one substituent selected from the group consisting of halogen, C₁-C₆ alkyl and halo-substituted C₁-C₄ alkyl group; and wherein when n=1 and R is H, Ar is not phenyl, 2-chlorophenyl, 2,4-difluorophenyl, or o-methyl-phenyl. The preferred meaning of R is F, Cl, Br, methyl, isobutyl, methoxy, trifluromethyl, morpholinyl or piperazinyl group. The cited document discloses the preparation and efficacy data of 58 specific compounds.

International patent application No. WO 2018/031987 discloses further substituted 4,7-dibenzyl- and 4-benzyl-7-(thiophenyl-methyl)-imipridone of formula (Reference Formula 6) 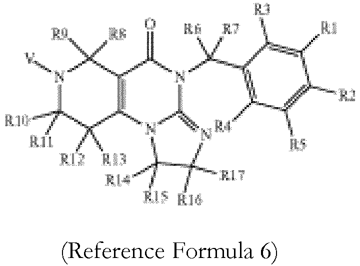 wherein V means substituted benzyl, (thiophen-2-yl)-methyl or (thiophen-3-yl)-methyl group, and the other substituents are as defined in the cited document. The application includes the preparation and efficacy data of 27 specific compounds.

U.S. patent application No. US 2018/016277 relates to novel deuterated imidazo[1,2-a]pyrido[3,4-e]pyrimidin-5(1H)-one compounds, such as ONC201 (Reference Compound 1) and analogues. This document also discloses compositions comprising a compound of the cited document and the use of such compositions alone or in combination with other therapeutics in the treatment of diseases and conditions that are beneficially treated by administering an inducer of the gene encoding tumor necrosis factor (TNF) related apoptosis-inducing ligand (TRAIL) superfamily member 10. Further, the cited patent documents disclose the use of imipridone derivatives in cancer therapies. So, the use of compound ONC201 (Reference Compound 1) in the treatment of genitourinary cancer is disclosed in U.S. patent No. US10,456,402*.*

U.S. patent No. US 9,688,679 discloses the use of ONC 201 in the treatment of leukemia.

International patent application No. WO2017/132661 discloses the use of a compound of above mentioned Reference Formula 1 for treating or preventing a disease, disorder, or condition in a subject in need of selective modulation of the activity of a Class A G protein-coupled receptor (GPCR) or a Class A GPCR signaling pathway, in particular, for treating the cancer is selected from the group consisting of a central nervous system tumor, a brain tumor, a peripheral nervous system tumor, a pheochromocytoma, a paraganglioma, a neuroendocrine tumor, a pancreatic cancer, a prostate cancer, an endometrial cancer, a hematological malignancy, and a lymphatic system tumor. The preferred compounds are ONC 201, 206, 212, 2013 and 236.

U.S. patent No. US 10,172,862 and No. US 10,369,154 disclose the use of compound ONC201 (Reference Compound 1) treatment of midline gliomas.

### THE PROBLEM TO BE SOLVED BY THE INVENTION

There is a need for novel compounds, which has enhanced anticancer activity as compared to the state of the art, which property makes these compounds suitable for use in medicine.

### THE DISCOVERY ACCORDING TO THE PRESENT INVENTION

By means of a close inspection of the structural features of the residual members of imipridones, we have discovered that novel imipridone derivatives, which contain 3,5-disubstituted benzyl group attached to position 7, related to the present invention, have enhanced anticancer activity.

### BRIEF DESCRIPTION OF THE FIGURES

**Figure 1****:** IC₅₀ curves created based on the results of *in vitro* test performed on PC3 human prostate cancer cell line.
**Figure 2****:** IC₅₀ curves created based on the results of *in vitro* test performed on LNCap human prostate cancer cell line.
**Figure 3****:** IC₅₀ curves created based on the results of *in vitro* test performed on BxPC3 pancreatic cancer cell line.
**Figure 4****:** IC₅₀ curves created based on the results of *in vitro* test performed on MiaPaCa2 pancreatic cancer cell line.
**Figure 5****:** IC₅₀ curves created based on the results of *in vitro* test performed on Panc1 pancreatic cancer cell line.
**Figure 6****:** IC₅₀ curves created based on the results of *in vitro* test performed on A549 lung carcinoma cell line.
**Figure 7****:** IC₅₀ curves created based on the results of *in vitro* test performed on HCC827 lung carcinoma cell line.
**Figure 8****:** IC₅₀ curves created based on the results of *in vitro* test performed on H1993 lung carcinoma cell line.
**Figure 9****:** IC₅₀ curves created based on the results of *in vitro* test performed on H520 lung carcinoma cell line.
**Figure 10****:** IC₅₀ curves created based on the results of *in vitro* test performed on MDA-MB-453 breast cancer cell line.
**Figure 11****:** IC₅₀ curves created based on the results of *in vitro* test performed on MDA-MB-231 breast cancer cell line.
**Figure 12****:** Dose-response curve obtained by compound I/1 (ONC 212) (reference) on DU 145, LNCaP and PC-3 cell lines.
**Figure 13****:** Dose-response curve obtained by compound I/7 (ABB-011) on DU 145, LNCaP and PC-3 cell lines.
**Figure 14****:** Dose-response curve obtained by compound I/3 (CZT-021) (reference) on DU 145, LNCaP and PC-3 cell lines.
**Figure 15****:** Dose-response curve obtained by compound I/124 (TBP-333) on DU 145, LNCaP and PC-3 cell lines.
**Figure 16****:** Dose-response curve obtained by compound I/6 (TBP-218) (reference) on DU 145, LNCaP and PC-3 cell lines.
**Figure 17****:** Dose-response curve obtained by compound I/111 (TBP-272) on DU 145, LNCaP and PC-3 cell lines.
**Figure 18****:** Dose-response curve obtained by compound I/149 (TBP-353) (reference) on DU 145, LNCaP and PC-3 cell lines.
**Figure 19****:** Dose-response curve obtained by compound I/133 (TBP-400) on DU 145, LNCaP and PC-3 cell lines.
**Figure 20****:** Dose-response curve obtained by compound I/30 (TBP-301) on DU 145, LNCaP and PC-3 cell lines.
**Figure 21****:** Dose-response curve obtained by compound I/107 (CZT-136) on DU 145, LNCaP and PC-3 cell lines.
**Figure 22****:** Dose-response curve obtained by compound I/1 (ONC 212) (reference) on Panc-1, Capan-1 and MIA PaCa-2 cell lines.
**Figure 23****:** Dose-response curve obtained by compound I/7 (ABB-011) on Panc-1, Capan-1 and MIA PaCa-2 cell lines.
**Figure 24****:** Dose-response curve obtained by compound I/3 (CZT-021) (reference) on Panc-1, Capan-1 and MIA PaCa-2 cell lines.
**Figure 25****:** Dose-response curve obtained by compound I/124 (TBP-333) on Panc-1, Capan-1 and MIA PaCa-2 cell lines.
**Figure 26****:** Dose-response curve obtained by compound I/6 (TBP-218) (reference) on Panc-1, Capan-1 and MIA PaCa-2 cell lines.
**Figure 27****:** Dose-response curve obtained by compound I/111 (TBP-272) on Panc-1, Capan-1 and MIA PaCa-2 cell lines.
**Figure 28****:** Dose-response curve obtained by compound I/149 (TBP-353) (reference) on Panc-1, Capan-1 and MIA PaCa-2 cell lines.
**Figure 29****:** Dose-response curve obtained by compound I/133 (TBP-400) on Panc-1, Capan-1 and MIA PaCa-2 cell lines.
**Figure 30****:** Dose-response curve obtained by compound I/30 (TBP-301) on Panc-1, Capan-1 and MIA PaCa-2 cell lines.
**Figure 31****:** Dose-response curve obtained by compound I/107 (CZT-136) on Panc-1, Capan-1 and MIA PaCa-2 cell lines.
**Figure 32****:** Dose-response curve obtained by compound I/1 (ONC 212) (reference) on Detroit 562, SCC-25 and Fadu cell lines.
**Figure 33****:** Dose-response curve obtained by compound I/7 (ABB-011) on Detroit 562, SCC-25 and Fadu cell lines.
**Figure 34****:** Dose-response curve obtained by compound I/3 (CZT-021) (reference) on Detroit 562, SCC-25 and Fadu cell lines.
**Figure 35****:** Dose-response curve obtained by compound I/124 (TBP-333) on Detroit 562, SCC-25 and Fadu cell lines.
**Figure 36****:** Dose-response curve obtained by compound I/6 (TBP-218) (reference) on Detroit 562, SCC-25 and Fadu cell lines.
**Figure 37****:** Dose-response curve obtained by compound I/111 (TBP-272) on Detroit 562, SCC-25 and Fadu cell lines.
**Figure 38****:** Dose-response curve obtained by compound I/149 (TBP-353) (reference) on Detroit 562, SCC-25 and Fadu cell lines.
**Figure 39****:** Dose-response curve obtained by compound I/133 (TBP-400) on Detroit 562, SCC-25 and Fadu cell lines.
**Figure 40****:** Dose-response curve obtained by compound I/30 (TBP-301) on Detroit 562, SCC-25 and Fadu cell lines.
**Figure 41****:** Dose-response curve obtained by compound I/107 (CZT-136) on Detroit 562, SCC-25 and Fadu cell lines.
**Figure 42****:** Dose-response curve obtained by compound I/1 (ONC 212) (reference) on EBC-1, MDA-MB-231 and MDA-MB-453 cell lines.
**Figure 43****:** Dose-response curve obtained by compound I/7 (ABB-011) on EBC-1, MDA-MB-231 and MDA-MB-453 cell lines.
**Figure 44****:** Dose-response curve obtained by compound I/3 (CZT-021) (reference) on EBC-1, MDA-MB-231 and MDA-MB-453 cell lines.
**Figure 45****:** Dose-response curve obtained by compound I/124 (TBP-333) on EBC-1, MDA-MB-231 and MDA-MB-453 cell lines.
**Figure 46****:** Dose-response curve obtained by compound I/6 (TBP-218) (reference) on EBC-1, MDA-MB-231 and MDA-MB-453 cell lines.
**Figure 47****:** Dose-response curve obtained by compound I/111 (TBP-272) on EBC-1, MDA-MB-231 and MDA-MB-453 cell lines.
**Figure 48****:** Dose-response curve obtained by compound I/149 (TBP-353) (reference) on EBC-1, MDA-MB-231 and MDA-MB-453 cell lines.
**Figure 49****:** Dose-response curve obtained by compound I/133 (TBP-400) on EBC-1, MDA-MB-231 and MDA-MB-453 cell lines.
**Figure 50****:** Dose-response curve obtained by compound I/30 (TBP-301) on EBC-1, MDA-MB-231 and MDA-MB-453 cell lines.
**Figure 51****:** Dose-response curve obtained by compound I/107 (CZT-136) on EBC-1, MDA-MB-231 and MDA-MB-453 cell lines.
**Figure 52****:** Cytotoxic effect of compound I/1 (ONC 212) (reference) on Panc-1 cell line.
**Figure 53****:** Cytotoxic effect of compound I/3 (CZT-021) (reference) on Panc-1 cell line.
**Figure 54****:** Cytotoxic effect of compound I/6 (TBP-218) (reference) on Panc-1 cell line.
**Figure 55****:** Cytotoxic effect of compound I/149 (TBP-353) (reference) on Panc-1 cell line.
**Figure 56****:** Cytotoxic effect of compound I/30 (TBP-301) on Panc-1 cell line.
**Figure 57****:** Cytotoxic effect of compound I/7 (ABB-011) on Panc-1 cell line.
**Figure 58****:** Cytotoxic effect of compound I/124 (TBP-333) on Panc-1 cell line.
**Figure 59****:** Cytotoxic effect of compound I/111 (TBP-272) on Panc-1 cell line.
**Figure 60****:** Cytotoxic effect of compound I/133 (TBP-400) on Panc-1 cell line.
**Figure 61****:** Cytotoxic effect of compound I/107 (CZT-136) on Panc-1 cell line.
**Figure 62****:** Change of the animal weight in the experiment disclosed in Example 23.
**Figure 63****:** Change of tumor volumes [*: p<0.05 (Student's t-test)] in the experiment disclosed in Example 23.
**Figure 64****:** Tumor size after termination (weight) [*: p<0.05 (Student's t-test)] in the experiment disclosed in Example 23.
**Figure 65****:** Tumors after termination in the experiment disclosed in Example 23.

### BRIEF DESCRIPTION OF THE INVENTION

The claimed scope for the disclosed invention is detailed in the enclosed set of claims.

### DETAILED DESCRIPTION OF THE INVENTION

The present invention relates to compounds of formula (**I**) as described above in Item 1, wherein the identifiers in the formula are as defined in Item 1, and pharmaceutically acceptable salts, stereoisomers thereof, including single enantiomers, racemic mixtures, mixtures of enantiomers, or combinations thereof.

A group of compounds of the present invention is a compound of formula (**I**) as described above, wherein the identifiers in the formula are as defined in Item 2, and pharmaceutically acceptable salts, stereoisomers thereof, including single enantiomers, racemic mixtures, mixtures of enantiomers, or combinations thereof.

Another group of compounds of the present invention are compounds of formula (**I**) as described above in Item 3, wherein the identifiers in the formulae are as defined in Item 3, and the pharmaceutically acceptable salts, stereoisomers thereof, including single enantiomers, racemic mixtures, mixtures of enantiomers, or combinations thereof.

A further group of compounds of the present invention is a compound of formula (**I**) as described above in Item 4, wherein the identifiers in the formula are as defined in Item 4, and the pharmaceutically acceptable salts, stereoisomers thereof, including single enantiomers, racemic mixtures, mixtures of enantiomers, or combinations thereof.

A further group of compounds of the present invention is a compound of formula (**I**) as described above in Item 5, and the pharmaceutically acceptable salts, including enantiomers, racemic mixtures, mixtures of enantiomers, (if Z is hydroxymethyl). As noted above, the hydroxymethyl-substituted compounds (if Z is hydroxymethyl) of the present invention may exist as racemic mixtures and as optical isomers, in the form of one enantiomer, which is pure or predominantly present. It will be appreciated that both racemic mixtures and enantiomers in pure form or in the mixture predominantly as compared to the other enantiomer belong to the subject matter of the invention.

The invention also relates to the compounds of the invention for use in the treatment of cancer diseases selected from the group consisting of prostate carcinoma, pancreatic cancer, lung carcinoma, breast cancer, glioma, cancers of head and neck, colon cancer, skin cancer.

By means of a close inspection of the structural features of the residual members of imipridones described in the prior art, it can be established that besides the limited versatility of the substituents bonded to N-4 and N-7, except for ONC201 (Reference Compound 1), the known impiridones do not contain di- or trisubstituted benzyl group attached to position 7 indicating that the accessible chemical space is underexplored. This realization has lead to identify novel lead compounds of more pronounced potential in chemotherapeutic applications which, in addition to enhanced anticancer activity, have additional beneficial properties, e.g. in terms of therapeutic window and bioavailability superior to those of the state of the art collection.

Accordingly, following our convergent synthetic route presented on Reaction Scheme 1 we performed a diversity oriented synthesis of novel imipridones with a variety of mono-, di- and trisubstituted benzyl groups and amine-based molecular fragment on both terminals of the heterocyclic skeleton (see Table 1).

To expand the scope of the functional groups on the pending skeletal substituents, the compounds prepared from commercially available building blocks containing Boc-protected aromatic amine residue(s), were converted into azido derivatives (see Table 1) by a one-pot procedure comprising simultaneous acid-catalyzed N-deprotection and diazotation followed by diazonium→azide exchange (see Reaction Scheme 2). By means of copper(I)-catalysis two azides were coupled with ethynylferrocene and erlotinib to get triazole-tethered hybrids (see Table 1).

In the course of inventive work the results of biological tests, disclosing characteristic structure activity relationships (SAR), were continuously taken into account for the structural refinement in the design and synthesis of the further members of novel imipridones that displayed enhanced cytotoxic effects. Since only a limited set of data resulted from the bioassays of imipridones known from the prior art are accessible (WO 2018031987 A1), besides ONC212 (Reference Compound 2) three additional known halogenated analogues [**I/2** (ONC217 as reference), **I/3** (2185824-99-9P, CZT-021 as reference) and **I/4** (2185824-98-8P, TBP-287 as reference) in Table 1] were also prepared to serve as references in our biological tests.

As a result of stepwise refinement we identified three novel halogenated imipridones [**I/7** (ABB-011), **1/30** (TBP-301) and **I/39** (TBP-302)] as the most potent drug candidates displaying extremely strong cytotoxicity on representative cell lines characterized by IC₅₀ values in a range of ca. 3-8 nM unambiguously indicating their superiority over each on reference compound known from the prior art (Tables 2 and 3) We have also disclosed that the R-enantiomer of a hydroxymethyl substituted imipridone **I/129(R)** [TBP-339(R)] exhibits much stronger antiproliferative effect on PANC1 cell line that does the S-enantiomer **I(129(S)** [(TBP-339(S)] (IC₅₀ = 15 nM vs 265 nM: Table 2).

A clear structure-activity relationship, associated with the stepwise introduction of fluorine substituents in the "*meta*" position(s) of the N-7-benzyl group, can be established from the tendency observed in the gradual increase in the cytotoxicity produced by ONC212 (Reference Compound 2), 2185824-99-9P (CZT-021 as reference) and **1/30** (TBP-301). The very same tendency is discernible in the antiproliferative effects of the series **I/5** (TBP-134), **I/6** (TBP-218 as reference), **I/7** (ABB-011) and **I/41** (TBP-285), 2185824-98-8P **I/39** (TBP-302) the 3,5-difluorobenzyl-substituted derivatives being far the most active ones.

### Preparation of compounds of the invention

The convergent synthesis of the novel imipridones of general formula (**I**) (except for the compounds containing N₃, CN, SCN and SeCN substituents in the side chains pending on positions 4 and 7) (Reaction scheme 1) are based on straightforward coupling reactions and anullations utilizing readily available precursors, such as 2-(methylthio)-4,5-dihydro-1H-imidazole (**I**), methylacrylate and primary amines types **3** and **5.**

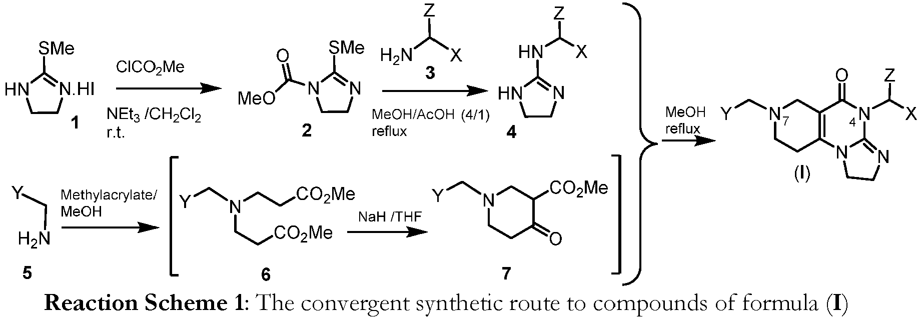

### Materials and Methods

All fine chemicals were obtained from commercially available sources (Merck, Fluorochem, Molar Chemicals, VWR) and used without further purification. Dioxane was distilled from sodium benzophenone. Merck Kieselgel (230-400 mesh, 60 Ǻ) was used for flash column chromatography. Melting points (uncorrected) were determined with a Büchi M-560. The ¹Hand ¹³C NMR spectra of all compounds were recorded in CDCl₃ solution in 5 mm tubes at RT, on a Bruker DRX-500 spectrometer at 500 (¹H) and 125 (¹³C) MHz, with the deuterium signal of the solvent as the lock and TMS as the internal standard. The HSQC, HMBC, COSY and NOESY spectra, which support the exact assignments the of ¹H- and ¹³C NMR signals were obtained by using the standard Bruker pulse programs.

### General procedures of the synthetic steps finally leading to compounds of formula (I) as presented on Reaction Scheme 1

### 1. Methyl 2-(methylthio)-4,5-dihydro-1H-imidazgole-1-carboxylate (2)

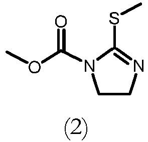

Commercially available 2-methylthio-4,5-dihydroimidazolium iodide (12.21 g, 50 mmol) and triethylamine (TEA, 16 mL, 11.62 g, 115 mmol) were dissolved in DCM (50 mL). Methylchloroformate (5 mL, 6.12 g, 65 mmol) was added dropwise to the solution previously cooled down to 0 °C. The reaction mixture was allowed to warm up to 25 °C and was stirred overnight. After addition of EtOAc (200 mL) and stirring for 15 min, the precipitated ammonium salts were filtered off and washed through with EtOAc (50 mL). The combined solution was evaporated to dryness. The solid residue was triturated with water filtered off and dried under vacuo to obtain **5** as white solid. Yield: 5.55 g (64%).

### 2. N-substituted-4,5-dihydro-1H-imidazol-2-amine (4)

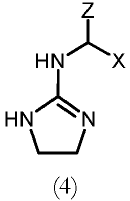

To the solution of primary amine type **3** (2 mmol) dissolved in a mixture of MeOH:AcOH (4 mL:1 mL) methyl 2-(methylthio)-4,5-dihydro-1*H*-imidazole-1-carboxylate **2** (0.47g, 2.4 mmol) was added and the resulting solution was stirred at reflux for 20 h. After cooling down the reaction was concentrated in *vacuo* and the oily residue was dissolved in DCM (30 mL). The solution was washed with 3 M NaOH (10 mL), brine (10 mL), dried over Na₂SO₄ and evaporated to dryness. The colorless oil was crystallised from ether and used without further purification for the cyclisation to imipridone framework.

### 3. Impiridone forming cyclisation

Primary amine **5** (1 mmol), dissolved in MeOH (4 mL), methylacrylate (0.23 mL, 2.5 mmol) was added and the mixture was stirred for 24 h at room temperature, concentrated *in vacuo* and the obtained crude dipropionate type **6** was dissolved in anhydrous THF (4 mL). Under argon atmosphere NaH (0.12 g, 5 mmol,) was added in small portions to the intensively stirred solution that previously was cooled down to 0 °C. The resulting suspension was stirred for additional 2 h at reflux temperature and concentrated to dryness under *vacuo.* The resulting solid residue containing the crude sodium salt of methyl *N*-substituted oxopiperidine-3-carboxylate (**7**) was dissolved in anhydrous MeOH (5 mL). To this solution was added *N*-substituted-4,5-dihydro-1*H*-imidazol-2-amine (**4**) (1 mmol) prepared in separate steps as described above. The basic solution was stirred at a reflux for 12 h, under argon atmosphere then cooled down by ice-water. The cooled reaction mixture was stirred for 1 h and the precipitated solid was collected by filtration, washed with cold methanol and dried to produce the pure imipridone product of formula (**I**).

### 4. Synthesis of amine-containing imipridones.

For the synthesis of the target compounds with pending primary amine or cyclic secondary amine moiety, the corresponding diamine (**3** or **5**) *mono*-Boc-protected either in X or in Y group was used as coupling component in the general procedures described above. The isolated Boc-protected imipridone (2 mmol) was dissolved in cc HCl (5 mL) and the resulting solution was heated at reflux for 5 minutes and cooled down to room temperature. The pH of this solution was then set to ca. 13-14 by concentrated aqueous potassium hydroxide. The precipitated amine product was collected by filtration, thoroughly washed with cold water and dried in desiccator over potassium hydride pellets.

### 5. Synthesis of azidobenzyl imipridones

The corresponding aminobenzyl imipridone (1 mmol) was dissolved in cc HCl (5 mL). To this solution cooled down to 0 °C was dropwise added an aqueous solution of NaNO₂ (137.8 mg, 2 mmol dissolved in 2.5 mL of water). The diazotation reaction was monitored by TLC. After completion, NaN₃ (324 mg, 5 mmol) was added at 0 °C to the reaction mixture which was then stirred at room temperature for 1 h. The pH of the solution was adjusted to 10-11 by a careful addition of solid Na₂CO₃. The resulting mixture was extracted by CH₂Cl₂ (2 × 40 mL) and the organic phase was dried over Na₂SO₄ then evaporated to dryness. The oily residue was crystallized by n-hexane to obtain the product as colourless solid.

### 6. Synthesis of aryltriazolylbenzyl imipridones from azidobenzyl imipridones

The corresponding azidobenzyl imipridone (1 mmol), the terminal alkyne component (1 mmol) and CuI (29.3 mg, 0.15 mmol) were dissolved in DMSO (5 mL). In a closed vessel the reaction mixture was stirred for 24 h at room temperature and poured on water (50 mL). The precipitated solid was collected by filtration, washed with water (100 mL) and suspended in aqueous ammonia solution (20 mL). The suspension was stirred for 20 min and filtered. The residue was washed with water (50 mL), dried and dissolved in a 9:1 mixture of CH₂Cl₂ and MeOH (10 mL). The solution was passed through silica and evaporated. The solid residue was crystallized with ether.

In the following, the invention will be illustrated by means of exemplary embodiments which, however, are not to be construed as limiting the invention.

### EXAMPLES

### Example 1:

### 7-(3,3-Difluorobenzyl)-4-(4-(trifluoromethyl)benzyl)-2,4,6,7,8,9-hexahydroimidazo[1,2-a]pyrido[3,4-e]pyrimidin-5(1H)-one (TBP-301) (Compound I/30)

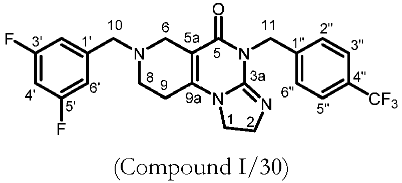

Yield: 387 mg (81%). Mp.: 168.0 °C. ¹H-NMR (CDCl₃): 7.53 and 7.50 (A and B part of an AA'BB' spin system, *J*_{AB}=8.9 Hz, 2x2H, H-3",5" and H-2",6", resp.); 6.84 (br dt, *J*~7 Hz and ~2 Hz, 2H, H-2',6'); 6.67 (tt, *J*=9.0 Hz and 2.3 Hz, 1H, H-4'); 5.06 (s, 2H, H-11); 3.88 (s, 4H, H-1 and H-2); 3.60 (s, 2H, H-10); 3.24 (br s, 2H, H-6); 2.64 (t, 2H, *J*=5.7 Hz, H-8); 2.46 (t, 2H, *J*=5.7 Hz, H-9). ¹³C-NMR (CDCl₃): 163.1 (dd, *J*=250.2 Hz and 15.6 Hz, C-3',5'); 161.3 (C-5); 152.9 (C-3a); 145.8 (C-9a); 142.2 (t, *J*=8.4 Hz, C-1'); 140.8 (C-1"); 129.6 (qa, *J*=32.5 Hz, C-4"); 128.8 (C-2",6"); 125.3 (qa, *J*=3.8 Hz, C-3",5"); 124.4 (qa, *J*=272.5 Hz, CF₃); 111.3 (dd, *J*=19.3 Hz and 4.9 Hz, C-2',6'); 102.8 (t, *J*=25.8 Hz, C-4'); 101.6 (C-5a); 61.4 (C-10); 50.6 (C-2); 49.3 (C-6); 48.4 (C-8); 46.9 (C-1); 45.0 (C-11); 26.8 (C-9).

### Example 2:

### 4-(4-Bromobenzyl)-7-(3,5-difluorobenzyl)-2,4,6,7,8,9-hexahydroimidazo[1,2-a]pyrido[3,4-e]pyrimidin-5(1H)-one (TBP-302) (Compound I/39)

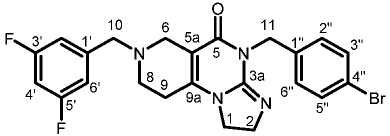

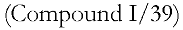

Yield: 271 mg (56%). Mp.: 173.5 °C. ¹H-NMR (CDCl₃): 7.36 (d, *J*=8.3 Hz, 2H, H-3",5"); 7.32 (d, *J*=8.3 Hz, 2H, H-2",6"); 6.84 (br dt, *J*~7 Hz and ~2 Hz, 2H, H-2',6'); 6.66 (tt, *J*=9.0 Hz and 2.3 Hz, 1H, H-4'); 4.95 (s, 2H, H-11); 3.87 (s, 4H, H-1 and H-2); 3.60 (s, 2H, H-10); 3.23 (br s, 2H, H-6); 2.62 (t, 2H, *J*=5.7 Hz, H-8); 2.44 (t, 2H, *J*=5.7 Hz, H-9). ¹³C-NMR (CDCl₃): 163.1 (dd, *J*=250.2 Hz and 15.6 Hz, C-3',5'); 161.3 (C-5); 152.9 (C-3a); 145.8 (C-9a); 142.2 (t, *J*=8.4 Hz, C-1'); 135.9 (C-1"); 131.4 (C-3",5"); 130.6 (C-2",6"); 121.4 (C-4"); 111.3 (dd, *J*=19.3 Hz and 4.9 Hz, C-2',6'); 102.8 (t, *J*=25.7 Hz, C-4'); 101.6 (C-5a); 61.4 (C-10); 50.6 (C-2); 49.4 (C-6); 48.4 (C-8); 46.9 (C-1); 44.8 (C-11); 26.8 (C-9).

### Example 3:

### 4-(4-Fluorobenzyl)-7-(3,5-difluorobenzyl)-2,4,6,7,8,9-hexahydroimidazo[1,2-a]pyrido[3,4-e]pyrimidin-5(1H)-one (ABB-011) (Compound I/7)

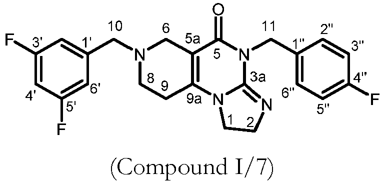

Yield: 290 mg (68%). Mp.: 190.5 °C. ¹H-NMR (CDCl₃): 7.44 (dd, *J*=8.7 Hz and 5.7 Hz, 2H, H-2",6"); 6.92 (t, *J*=8.7 Hz, 2H, H-3",5"); 6.84 (br dt, *J*~7 Hz and ~2 Hz, 2H, H-2',6'); 6.66 (tt, *J*=8.9 Hz and 2.3 Hz, 1H, H-4'); 4.97 (s, 2H, H-11); 3.92-3.83 (m, 4H, H-1 and H-2); 3.59 (s, 2H, H-10); 3.23 (br s, 2H, H-6); 2.62 (t, 2H, *J*=5.7 Hz, H-8); 2.44 (t, 2H, *J*=5.7 Hz, H-9). ¹³C-NMR (CDCl₃): 162.2 (d, J=162.2 Hz, C-4"); 163.1 (dd, *J*=250.0 Hz and 15.2 Hz, C-3',5'); 161.4 (C-5); 152.9 (C-3a); 145.5 (C-9a); 142.2 (t, *J*=8.4 Hz, C-1'); 132.7 (C-1"); 130.7 (d, *J*=7.9 Hz, C-2",6"); 115.0 (d, *J*=21.3 Hz, C-3",5"); 111.4 (dd, *J*=19.3 Hz and 4.9 Hz, C-2',6'); 102.7 (t, *J*=25.7 Hz, C-4'); 101.6 (C-5a); 61.4 (C-10); 50.6 (C-2); 49.4 (C-6); 48.4 (C-8); 46.9 (C-1); 44.7 (C-11); 26.7 (C-9).

### Example 4:

### 4-(4-Chlombenzyl)-7-(3,5-difluorobenzyl)-2,4,6,7,8,9-hexahydmimidazo[1,2-a]pyrido[3,4-e]pyrimidin-5(1H)-one (TBP-333) (Compound I/124)

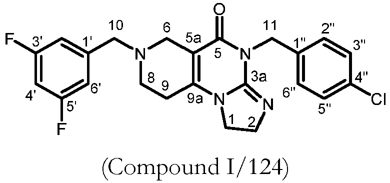

Yield: 332 mg (75%). Mp.: 162.4 °C. ¹H-NMR (CDCl₃): 7.38 (d, *J*=8.3 Hz, 2H, H-2",6"); 7.20 (d, *J*=8.3 Hz, 2H, H-3",5"); 6.84 (br dt, *J*~7 Hz and ~2 Hz, 2H, H-2',6'); 6.66 (tt, *J*=9.0 Hz and 2.3 Hz, 1H, H-4'); 4.97 (s, 2H, H-11); 3.90-3.83 (m, 4H, H-1 and H-2); 3.59 (s, 2H, H-10); 3.22 (br s, 2H, H-6); 2.62 (t, 2H, *J*=5.7 Hz, H-8); 2.44 (t, 2H, *J*=5.7 Hz, H-9). ¹³C-NMR (CDCl₃): 163.1 (dd, *J*=248.2 Hz and 14.6 Hz, C-3',5'); 161.3 (C-5); 152.9 (C-3a); 145.6 (C-9a); 142.2 (t, *J*=8.4 Hz, C-1'); 135.4 (C-1"); 133.2 (C-4"); 130.6 (C-2",6"); 128.4 (C-3",5"); 111.7 (dd, *J*=19.3 Hz and 4.9 Hz, C-2',6'); 102.7 (t, *J*=25.7 Hz, C-4'); 101.6 (C-5a); 61.4 (C-10); 50.6 (C-2); 49.4 (C-6); 48.4 (C-8); 46.9 (C-1); 44.7 (C-11); 26.7 (C-9).

### Example 5:

### 4-(3-Chlorobenzyl)-7-(3,5-difluorobenzyl)-2,4,6,7,8,9-hexahydroimidazo[1,2-a]pyrido[3,4-e]pyrimidin-5(1H)-one (TBP-344) (Compound I/125)

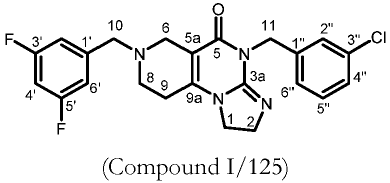

Yield: 302 mg (68%). Mp.: 204.8 °C. ¹H-NMR (CDCl₃): 7.40 (br s, 1H, H-2"); 7.30 (m, 1H, H-5"); 7.18-7.15 (m, 2H, H-4",6"); 6.84 (br dt, *J*~7 Hz and ~2 Hz, 2H, H-2',6'); 6.66 (tt, *J*=8.9 Hz and 2.2 Hz, 1H, H-4'); 4.98 (s, 2H, H-11); 3.87 (br ~s, 4H, H-1 and H-2); 3.59 (s, 2H, H-10); 3.24 (br s, 2H, H-6); 2.63 (t, 2H, *J*=5.6 Hz, H-8); 2.45 (t, 2H, *J*=5.6 Hz, H-9). ¹³C-NMR (CDCl₃): 163.1 (dd, *J*=248.6 Hz and 14.0 Hz, C-3',5'); 161.3 (C-5); 152.9 (C-3a); 145.7 (C-9a); 142.3 (t, *J*=8.4 Hz, C-1'); 138.8 (C-1"); 134.1 (C-3"); 128.4 (two coalesced lines, C-2" and C-4"); 127.6 (C-6"); 126.8 (C-5"); 111.3 (dd, *J*=19.6 Hz and 5.1 Hz, C-2',6'); 102.7 (t, *J*=25.3 Hz, C-4'); 101.6 (C-5a); 61.3 (C-10); 50.6 (C-2); 49.4 (C-6); 48.4 (C-8); 46.9 (C-1); 44.9 (C-11); 26.8 (C-9).

### Example 6:

### rac-4-(4-Chlorobenzyl)-7-(3,5-difluorobenzyl)-2,4,6,7,8,9-hexahydroimidazo[1,2-a]pyrido[3,4-e]pyrimidin-5(1H)-one (TBP-342) (Compound I/128 (racemate))

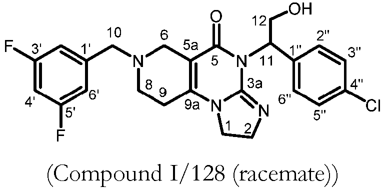

Yield: 38 mg (8%). Mp.: 86 °C. ¹H-NMR (CDCl₃): 7.28 (d, *J*=8.3 Hz, 2H, H-2",6"); 7.23 (d, *J*=8.3 Hz, 2H, H-3",5"); 6.84 (br dt, *J*~7 Hz and ~2 Hz, 2H, H-2',6'); 6.67 (tt, *J*=9.0 Hz and 2.3 Hz, 1H, H-4'); 6.08 (dd, *J*=5.1 Hz and 1.6 Hz, 1H, H-11); 4.31 (dd, *J*=12.8 Hz and 5.1 Hz, 1H, H-12_{A}); 4.06 (dd, *J*=12.8 Hz and 1.6 Hz, 1H, H-12_{B}); 3.93-3.85 (m, 3H, H-1 and H-2_{A}); 3.78 (m, 1H, H-2_{B}); 3.60 (s, 2H, H-10); 3.24 and 3.21 (A and B parts of an AB spin system, *J*_{AB}=14.9 Hz, 2x1H, H-6_{A} and H-6_{B}); 2.66 (m, 2H, H-8); 2.50 (m, 2H, H-9). ¹³C-NMR (CDCl₃): 163.1 (dd, *J*=248.2 Hz and 13.9 Hz, C-3',5'); 161.6 (C-5); 153.5 (C-3a); 146.0 (C-9a); 142.2 (t, *J*=8.4 Hz, C-1'); 135.2 (C-1"); 133.1 (C-4"); 139.0 (C-2",6"); 128.4 (C-3",5"); 111.4 (dd, *J*=19.3 Hz and 4.9 Hz, C-2',6'); 102.8 (t, *J*=25.7 Hz, C-4'); 102.4 (C-5a); 62.9 (C-12); 61.3 (C-10); 57.6 (C-11); 49.5 (C-2); 49.4 (C-6); 48.4 (C-8); 46.5 (C-1); 26.8 (C-9).

### Example 7:

### R-4-(4-Fluorobenzyl)-7-(3,5-difluorobenzyl)-2,4,6,7,8,9-hexahydroimidazo[1,2-a]pyrido[3,4-e]pyrimidin-5(1H)-one (TBP-339) (Compound I/129 (R-enantiomer))

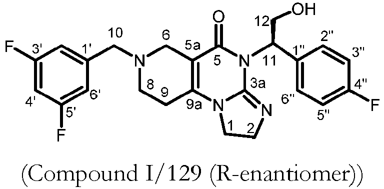

Yield: 51 mg (11%). Mp.: 76 °C. ¹H-NMR (CDCl₃): 7.36 (dd, *J*=8.8 Hz and 5.8 Hz, 2H, H-2",6"); 6.96 (t, *J*=8.3 Hz, 2H, H-3",5"); 6.85 (br dt, *J*~7 Hz and ~2 Hz, 2H, H-2',6'); 6.67 (tt, *J*=9.0 Hz and 2.3 Hz, 1H, H-4'); 6.09 (dd, *J*=5.3 Hz and 1.6 Hz, 1H, H-11); 4.32 (dd, *J*=12.8 Hz and 5.3 Hz, 1H, H-12_{A}); 4.07 (dd, *J*=12.8 Hz and 1.6 Hz, 1H, H-12_{B}); 3.94-3.85 (m, 3H, H-1 and H-2_{A}); 3.81 (m, 1H, H-2_{B}); 3.61 (s, 2H, H-10); 3.25 and 3.21 (A and B parts of an AB spin system, *J*_{AB}=14.9 Hz, 2x1H, H-6_{A} and H-6_{B}); 2.66 (m, 2H, H-8); 2.50 (m, 2H, H-9). ¹³C-NMR (CDCl₃): 163.1 (dd, *J*=248.2 Hz and 13.9 Hz, C-3',5'); 162.0 (d, *J*=245.8 Hz, C-4"); 161.6 (C-5); 153.5 (C-3a); 145.8 (C-9a); 142.1 (t, *J*=8.4 Hz, C-1'); 132.5 (br s, C-1"); 129.4 (d, *J*=7.8 Hz, C-2",6"); 115.1 (d, *J*=21.4 Hz, C-3",5"); 111.3 (dd, *J*=19.6 Hz and 5.2 Hz, C-2',6'); 102.8 (t, *J*=25.6 Hz, C-4'); 102.5 (C-5a); 63.1 (C-12); 61.3 (C-10); 57.6 (C-11); 49.5 (two coalesced lines, C-2 and C-6); 48.3 (C-8); 46.5 (C-1); 26.8 (C-9).

### Example 8:

### 4-(3-(Aminomethyl)benzyl)-7-(3,5-difluorobenzyl)-2,4,6,7,8,9-hexahydroimidazo[1,2-a]pyrido[3,4-e]pyrimidin-5(1H)-one (TBP-324) (Compound I/61)

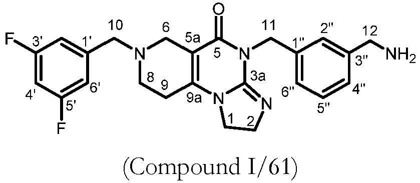

Yield: 376 mg (86%). Mp.: 129.5 °C. ¹H-NMR (CDCl₃): 7.33 (br s, 1H, H-2"); 7.30 (br d,*J*=7.6 Hz, 1H, H-6"); 7.22 (t, *J*=7.6 Hz 1H, H-5"); 7.15 (br d, J=7.6 Hz, 1H, H-4"); 6.84 (br dt, *J*~7 Hz and ~2 Hz, 2H, H-2',6'); 6.66 (tt, *J*=8.9 Hz and 2.3 Hz, 1H, H-4'); 5.00 (s, 2H, H-11); 3.92-3.83 (m, 4H, H-1 and H-2); 3.79 (s, 2H, H-12); 3.59 (s, 2H, H-10); 3.24 (br s, 2H, H-6); 2.63 (t, 2H, *J*=5.7 Hz, H-8); 2.45 (t, 2H, *J*=5.7 Hz, H-9); 1.77 (br s, 2H, NH₂). ¹³C-NMR (CDCl₃): 163.1 (dd, *J*=248.4 Hz and 14.1 Hz, C-3',5'); 161.5 (C-5); 153.2 (C-3a); 145.6 (C-9a); 143.2 (C-3"); 142.3 (t, *J*=8.4 Hz, C-1'); 137.2 (C-1"); 128.6 (C-5"); 127.3 (C-4"); 127.1 (C-2"); 126.2 (C-4"); 111.4 (dd, *J*=20.0 Hz and 5.0 Hz, C-2',6'); 102.7 (t, *J*=25.7 Hz, C-4'); 101.7 (C-5a); 61.4 (C-10); 50.7 (C-2); 49.4 (C-6); 48.4 (C-8); 46.9 (C-1); 46.4 (C-12); 45.4 (C-11); 26.7 (C-9).

### Example 9:

### 4-(3-Aminobenzyl)-7-(3,5-difluorobenzyl)-2,4,6,7,8,9-hexahydroimidazo[1,2-a]pyrido[3,4-e]pyrimidin-5(1H)-one (TBP-346) (Compound I/126)

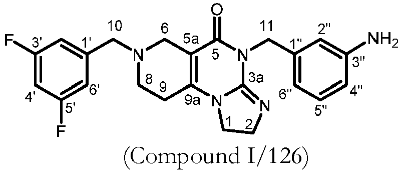

Yield: 347 mg (82%). Mp.: 114.5 °C. ¹H-NMR (CDCl₃): 7.03 (t, *J*=7.9 Hz, 1H, H-5"); 6.85 (br dt, *J*~7 Hz and ~2 Hz, 2H, H-2',6'); 6.82 (br d, *J*=7.9 Hz, 1H, H-6"); 6.75 (br s, 1H, H-2"); 6.66 (tt, *J*=8.9 Hz and 2.3 Hz, 1H, H-4'); 6.52 (dd, *J*=7.6 Hz and 2.2 Hz, 1H, H-4"); 4.93 (s, 2H, H-11); 3.92-3.79 (m, 4H, H-1 and H-2); 3.59 (s, 2H, H-10); 3.24 (br s, 2H, H-6); 2.62 (t, 2H, *J*=5.7 Hz, H-8); 2.44 (t, 2H, *J*=5.7 Hz, H-9); ¹³C-NMR (CDCl₃): 163.1 (dd, *J*=248.4 Hz and 14.1 Hz, C-3',5'); 161.5 (C-5); 153.0 (C-3a); 146.4 (C-3"); 145.3 (C-9a); 142.3 (t, *J*=8.4 Hz, C-1'); 138.0 (C-1"); 129.2 (C-5"); 119.0 (C-6"); 115.2 (C-2"); 114.3 (C-4"); 111.4 (dd, *J*=20.1 Hz and 5.4 Hz, C-2',6'); 102.7 (t, *J*=25.7 Hz, C-4'); 101.7 (C-5a); 61.4 (C-10); 50.6 (C-2); 49.4 (C-6); 48.4 (C-8); 46.8 (C-1); 45.3 (C-11); 26.7 (C-9).

### Example 10:

### 4-(4-Azidobenzyl)-7-(3,5-difluorobenzyl)-2,4,6,7,8,9-hexahydroimidazo[1,2-a]pyrido[3,4-e]pyrimidin-5(1H)-one (CZT-136)

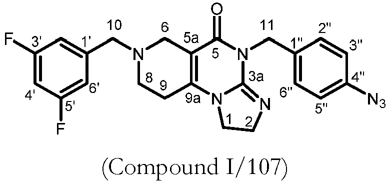

Yield: 270 mg (60%). mp.: 155.5 °C. ¹H-NMR (CDCl₃): 7.44 (d, *J*=8.3 Hz, 2H, H-2",6"); 6.90 (d, *J*=8.3 Hz, 2H, H-3",5"); 6.84 (br ~dt, *J*~7 Hz and ~2 Hz, 2H, H-2',6'); 6.66 (tt, *J*=9.0 Hz and 2.3 Hz, 1H, H-4'); 4.97 (s, 2H, H-11); 3.91-3.84 (m, 4H, H-1 and H-2); 3.59 (s, 2H, H-10); 3.23 (br s, 2H, H-6); 2.63 (t, 2H, *J*=5.7 Hz, H-8); 2.44 (t, 2H, *J*=5.7 Hz, H-9). ¹³C-NMR (CDCl₃): 163.1 (dd, *J*=250.2 Hz and 15.6 Hz, C-3',5'); 161.4 (C-5); 152.9 (C-3a); 145.5 (C-9a); 142.2 (t, *J*=8.4 Hz, C-1'); 139.1 (C-4"); 133.8 (C-1"); 130.4 (C-2",6"); 118.9 (C-3",5"); 111.3 (dd, *J*=19.3 Hz and 4.9 Hz, C-2',6'); 102.7 (t, *J*=25.7 Hz, C-4'); 101.7 (C-5a); 61.4 (C-10); 50.6 (C-2); 49.4 (C-6); 48.4 (C-8); 46.9 (C-1); 44.8 (C-11); 26.8 (C-9).

### Example 11:

### 7-(3-Azidobenzyl)-4-(4-(trifluoromethyl)benzyl)-2,4,6,7,8,9-hexahydroimidazo[1,2-a]pyrido[3,4-e]pyrimidin-5(1H)-one (TBP-272)

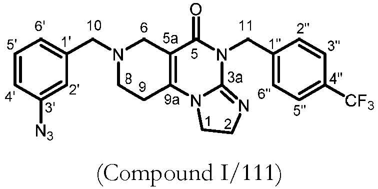

Yield: 313 mg (65%). mp.: 128.8 °C. ¹H-NMR (CDCl₃): 7.52 and 7.50 (A and B part of an AA'BB' spin system, *J*_{AB}=8.9 Hz, 2x2H, H-3",5" and H-2",6", resp.); 7.26 (t, *J*=7.7 Hz, 1H, H-5'); 7.06 (d, *J*=7.7 Hz, 1H, H-6'); 6.99 (t, *J*=1.5 Hz, 1H, H-2'); 6.90 (dd, *J*=7.7 Hz and 1.5 Hz, 1H, H-4'); 5.06 (s, 2H, H-11); 3.87 (s, 4H, H-1 and H-2); 3.61 (s, 2H,H-10); 3.24 (br s, 2H, H-6); 2.64 (t, 2H, *J*=5.7 Hz, H-8); 2.45 (t, 2H, *J*=5.7 Hz, H-9). ¹³C-NMR (CDCl₃): 161.7 (C-5); 153.0 (C-3a); 145.9 (C-9a); 140.8 (C-1"); 140.2 (C-3'); 140.0 (C-1'); 129.8 (C-5'); 129.6 (qa, *J*=32.5 Hz, C-4"); 128.9 (C-2",6"); 125.5 (C-6'); 125.3 (qa, *J*=3.5 Hz, C-3",5"); 124.4 (qa, *J*=272.5 Hz, CF₃); 119,4 (C-2'); 118.1 (C4'); 101.7 (C-5a); 61.9 (C-10); 50.6 (C-2); 49.4 (C-6); 48.4 (C-8); 46.9 (C-1); 45.0 (C-11); 26.8 (C-9).

### Example 12:

### 4-(3-Azidobenzyl)-7-(3,5-difluorobenzyl)-2,4,6,7,8,9-hexahydroimidazo[1,2-a]pyrido[3,4-e]pyrimidin-5(1H)-one (TBP-347) (Compound I/127)

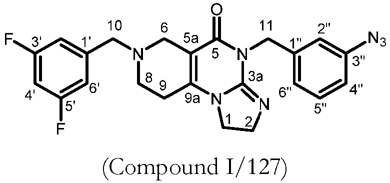

Yield: 355 mg (79%). Mp.: 158 °C. ¹H-NMR (CDCl₃): 7.23 (t, *J*=7.9 Hz, 1H, H-5"); 7.18 (br ~d, *J*~8 Hz 1H, H-6"); 7.10 (br s, 1H, H-2"); 6.87 (br ~d, *J*~8 Hz 1H, H-4"); 6.84 (br dt, *J*~7 Hz and ~2 Hz, 2H, H-2',6');6.66 (tt, *J*=8.9 Hz and 2.3 Hz, 1H, H-4'); 5.00 (s, 2H, H-11); 3.90-3.81 (m, 4H, H-1 and H-2); 3.60 (s, 2H, H-10); 3.24 (br s, 2H, H-6); 2.63 (t, 2H, *J*=5.7 Hz, H-8); 2.45 (t, 2H, *J*=5.7 Hz, H-9); ¹³C-NMR (CDCl₃): 163.1 (dd, *J*=248.4 Hz and 13.7 Hz, C-3',5'); 161.3 (C-5); 153.0 (C-3a); 145.6 (C-9a); 142.3 (t, *J*=9.0 Hz, C-1'); 140.0 (C-3"); 138.9 (C-1"); 129.7 (C-5"); 125.1 (C-6"); 121.2 (C-2"); 118.0 (C-4"); 111.4 (dd, *J*=20.1 Hz and 5.4 Hz, C-2',6'); 102.7 (t, *J*=25.7 Hz, C-4'); 101.7 (C-5a); 61.3 (C-10); 50.6 (C-2); 49.4 (C-6); 48.4 (C-8); 46.9 (C-1); 45.0 (C-11); 26.8 (C-9).

### Example 13:

### 7-Benzyl-4-(4-(4-ferrocenyl-1H-1,2,3-triazol-1-yl)benzyl)-2,4,6,7,8,9-hexahydroimidazo[1,2-a]pyrido[3,4-e]pyrimidin-5(1H)-one (ABB-030)

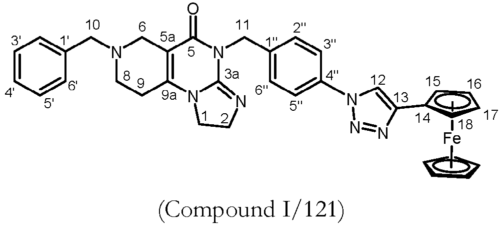

Yield: 443 mg (71%). mp.: 165.4 °C. ¹H-NMR (CDCl₃): 7.81 (s, 1H, H-12); 7.65 (d, *J*=7.9 Hz, 2H, H-3",5"); 7.59 (d, *J*=7.9 Hz, 2H, H-2",6"); 7.31-7.26 (m. 4H, H-2',3',5',6'); 7.22 (m, 1H, H-4'); 5.11 (s, 2H, H-11); 4.74 (t, J*=*1.8 Hz, 2H, H-15,18); 4.29 (t, *J*=1.8 Hz, 2H, H-16,17); 4.07 (s, η⁵-C₅H₅); 3.87 (br s, 4H, H-1 and H-2); 3.63 (s, 2H,H-10); 3.27 (br s, 2H, H-6); 2.63 (t, 2H, *J*=5.7 Hz, H-8); 2.44 (t, 2H, *J*=5.7 Hz, H-9). ¹³C-NMR (CDCl₃): 161.3 (C-5); 153.0 (C-3a); 147.5 (C-4'); 145.9 (C-9a); 130.1 (C-2",6"); 128.4 (C-2',6'); 127.4 (C-4'); 126.1 (C-3',5'); 120.1 (C-3",5"); 116.6 (C-12); 102.2 (C-5a); 75.0 (C-14); 69.6 (η⁵-C₅H₅); 68.8 (C-16,17); 66.8 (C-15,18); 62.3 (C-10); 50.6 (C-2); 49.5 (C-6); 48.3 (C-8); 46.8 (C-1); 44.9 (C-11); 26.8 (C-9).

### Example 14:

### Synthesis of 4-aylmethyl-substituted 7-(3,5-diagidobenzyl)-2,4,6,7,8,9-hexahydro-imidazo[1,2-a]pyride[3,4-e]pyrimidin-5(1H)-ones (Compounds I/138 and I/139)

Sodium azide (0.260 g 4 mmol), sodium ascorbate (0.119 g, 0.60 mmol), N,N-dimethylethylenediamine (0.080 g, 0.9 mmol), NaOH (0.012 g, 0.30 mmol), the corresponding 7-(3,5-dibromobenzyl) imipridone I**/134** or **I/135** (1 mmol) and CuI (0.057 g, 0.30 mmol) are dissolved in 20 mL of a degassed (argon for 30 min) EtOH/H₂O solvent mixture (7:3) and heated at reflux temperature for 3 h. The reaction mixture is concentrated under reduced pressure to ca. third of its original volume and extracted with CH₂Cl₂ (3 × 4 mL). The combined organic phases are washed with water (3 × 50 mL), dried over Na₂SO₄, filtered through paper and concentrated. Final purification is achieved by column chromatography using CH₂Cl₂/MeOH (99:1) as the eluent.

### Example 15:

### Synthesis of 4-aylmethyl-substituted 7-(3,5-dicyanobenzyl)-2,4,6,7,8,9-hexahydro-imidazo[1,2-a]pyrido[3,4-e]pyrimidin-5(1H)-ones (Compounds I/136 and I/137)

To a stirred solution of the corresponding 7-(3,5-dibromobenzyl) imipridone I**/134** or **I/135** (1 mmol) in DMF (2 mL), are sequentially added zinc cyanide (0.181 g, 1.2 mmol), 1,1'-bis(diphenylphosphino)ferrocene ligand (DPPF, 0.065 g, 0.117 mmol) and finally Pd₂dba₃ (45.8 mg, 0.05 mmol). The flask is flushed through with nitrogen and stirred in an oil bath at 110-120°C for 20 h. After cooling down to room temperature, the reaction mixture is evaporated under vacuo. The resulting crude product is subjected to flash silica gel column chromatography (eluent, ethyl acetate:hexane (1:4)) to obtain the dicyanobenzyl-substituted imipridone as a pure product.

### Example 16:

### Synthesis of 4-arylmethyl-substituted 7-(3-thiocyanatobenzyl)-2,4,6,7,8,9-hexahydro-imidazo[1,2-a]pyrido[3,4-e]pyrimidin-5(1H)-ones (Compounds I/142 and I/143)

Under an argon atmosphere, a mixture of the corresponding 7-(3-iodobenzyl) imipridone **(I/140** or **1/141)** (1.0 mmol), CuSCN (0.12 g, 1.0 mmol), KSCN (0.095 g, 1.0 mmol) and DMF (3 mL) is heated with stirring in an oil bath and maintained at 140 °C for 12 h. After cooling, the mixture is diluted with toluene (5 mL) and water (5 mL) then filtered through a Celite bed. The aqueous phase is extracted with toluene (2×5 mL) and the combined organic phase is washed with water, dried over Na₂S0₄, and concentrated. The residue is chromatographed over silica gel (hexane as eluent) affording the thiocyanate product that is further purified by recrystallization from hexane.

### Example 17:

### Synthesis of 4-arylmethyl-substituted 7-(3-selenocyanatobenzyl)-2,4,6,7,8,9-hexahydro-imidazo[1,2-a]pyrido[3,4-e]pyrimidin-5(1H)-ones (Compounds I/145 and I/146)

To a solution of the corresponding 7-(3-iodobenzyl) imipridone (**I/79** and I**/144**) (5 mmol) in dioxane (4 mL) is added 6 N HCl (10 mL). The resulting suspension is cooled to 0 °C, then NaNO₂ (0.415 g, 6 mmol) in water (2 mL) is slowly added. After stirring for 30 min, saturated NaOAc solution (ca. 30 mL) is added in portions to adjust the pH of the reaction mixture to 5-6. The resulting suspension is poured into a solution of KSeCN (0.793 g, 5.5 mmol) in water (25 mL) at 0 °C. After stirring for 30 min, the reaction mixture was warmed to room temperature and extracted with ether. The organic layer was washed with water and brine, dried over Na₂SO₄, and concentrated. The residue is purified by column chromatography over silica using ethyl acetate - hexane (1:5) as eluent.

### Example 18:

In this example we list the compounds of formula (**I**) according to the present invention and demonstrate the results of their *in vitro* antiproliferative assays for representative compounds investigated during our systematic experimental work along with relevant references known from prior art.

### Cell Cultures for long treatment experiments

The PANC-1 (human pancreatic carcinoma of ductal origin), COLO 205 (human colorectal adenocarcinoma), A2058 (human metastatic melanoma) obtained from European Collection of Authenticated Cell Cultures (ECACC, Salisbury, UK) and EBC-1 (human lung squamous cell carcinoma) purchased from Japanese Research Resources Bank (Tokyo, Japan) were used to determine the tumor growth inhibitory effects of the imipridone derivatives. PANC-1 cells were maintained in Dulbecco's Modified Eagle Medium (DMEM, Lonza, Basel, Switzerland); for the culturing of COLO-205 cell line DMEM medium formulated with 4500 mg/L d-glucose was used; EBC-1 cells were cultured in DMEM medium containing 1% non-essential amino acids (NEAA, Gibco^{®}/Invitrogen Corporation, New York, NY, USA), 1 mM sodium pyruvate (Sigma-Aldrich, St. Louis, MO, USA), while A2058 cell line was grown in RPMI 1640 (Lonza, Basel, Switzerland). In case of all cell lines, the aforementioned basal media were supplemented with 10% fetal bovine serum (FBS, Gibco^{®}/Invitrogen Corporation, New York, NY, USA), L-glutamine (2 mmol/L) (Lonza, Basel, Switzerland) and 100 µg/mL penicillin/streptomycin (Gibco^{®}/Invitrogen Corporation, New York, NY, USA). (Gibco^{®}/Invitrogen Corporation, New York, NY, USA). All cell lines were cultivated under standard conditions (37 °C, humidified 5% CO₂ atmosphere) in plastic culture dishes (Sigma-Aldrich, St. Louis, MO, USA or Eppendorf AG, Hamburg, Germany).

### Viability Assays (long treatment experiments)

### Impedance-Based Assay

The cytotoxicity experiments on PANC-1 cells was conducted using the impedance-based xCELLigence SP System (ACEA Biosciences, San Diego, CA, USA). A more detailed description of the basis of impedimetric measurement is given in our previous paper [22]. Monitoring the impedance change, which is proportional to the number of adhered cells on an electrode surface, provides a sensitive way for cytotoxicity studies [23]. The change in the impedance is expressed as in the form of Cell Index (CI) calculated by the software (RTCA 2.0, ACEA Biosciences, San Diego, CA, USA) integrated to xCELLigence System. For determination of IC₅₀ (a concentration that decreases the cell viability by 50%) values the tested imipridones were solved in DMSO and further diluted in supplemented DMEM medium to prepare a concentration range from 2.5×10⁻⁴ to 5×10⁻⁷ M. The steps of our impedimetric experiment proceeded in the same way as what was indicated in [24]. In brief, after gaining a constant CI value during the background measurement, the PANC-1 cells (1.5×10⁴ cells/well) were added to the so-called E-plate, and their adhesion/spreading was monitored for 24 h in order to settle the plateau phase of cell culture. In the last step, the cells in this balanced state were treated with the test compounds (final concentrations: 2.5×10⁵ to 5×10⁻⁸M) and the changes in CI were monitored for at least 72 hours at 10 kHz. In case of the control wells, the adequate volume ratio of DMSO was added. Three parallels were measured for each measurement. The CI values of each concentration obtained at 72 h after the treatment were normalized to that of the DMSO control. The IC₅₀ value was calculated for these normalized CI values by fitting a sigmoidal dose-response curve with the nonlinear regression function of OriginPro 8 (OriginLab Corporation, Northampton, MA, USA).

### Colorimetric Assay

The antiproliferative/cytotoxic effects of the imipridones on COLO-205 and A2058 cell lines were measured by the alamarBlue-assay. This colometric assay proved to be the more suitable method for the analysis of these cell lines than the xCELLigence System, because COLO-205 cells show weak/negligible adhesion, while A2058 cell line fails to establish a stable plateau phase during the impedimetric analysis. The inoculation of the cells and the procedure of alamarBlue-assay were analogous to the description reported in our previous paper [24]. The main steps are the following: (i) cell seeding on 96-well plates (Sarstedt AG, Nümbrecht, Germany) at 10⁴ cells/well concentration, (ii) treatment with the test substances at 2.5×10⁻⁵ to 5×10⁻⁸M final concentrations for 72 h, (iii) addition of alamarBlue reagent (0.15 mg/mL, Sigma-Aldrich, St. Louis, MO, USA) solved in PBS (phosphate-buffered saline, pH = 7.2) and (iv) reading the fluorescence intensity of the samples after 6-8 h incubation with alamarBlue reagent. LS-50B Luminescence Spectrometer (Perkin Elmer Ltd., Buckinghamshire United Kingdom) was applied for the fluorescence measurements with the following settings: excitation wavelength = 560 nm and emission wavelength = 590 nm. Each measurement was done in triplicates. Wells containing adequate volume ratio of DMSO served as control. The fluorescence intensity of each sample was expressed as a ratio of the fluorescence of DMSO control. The nonlinear regression function of OriginPro 8 (OriginLab Corporation, Northampton, MA, USA) was used for fitting sigmoidal dose-response curves to the normalized fluorescence intensities in order to calculate the IC₅₀ values.

Statistical evaluation of data Evaluation of the results was performed using RTCA 2.0 (ACEA Biosciences, San Diego, CA, USA), MS Excel, OriginPro 8 (OriginLab Corporation, Northampton, MA, USA) software. Data obtained from each experiment represent mathematical averages. The standard deviations of IC₅₀ parameters were also obtained with the sigmoidal curve fitting.

### Short term cytotoxicity studies using MTT assay

For the short term cytotoxicity studies the A-431 (human squamous carcinoma) and U-87 (human primary glioblastoma) cells were cultured in RPMI-1640 medium supplemented with 10% FCS (fetal calf serum, Sigma Ltd.), 2 mM L-glutamine, and 160 mg/mL gentamicin. Cell cultures were maintained at 37 °C in a humidified atmosphere with 5% CO₂. The cells were grown to confluency and were distributed into 96-well plate with initial cell number of 5.0×10³ per well. After 24 h incubation at 37 °C, the cells were treated with the compounds in 200 µL final volume containing 1.0 v/v% DMSO. The cells were incubated with the compounds at 10⁻⁴-10² µM concentration range for 1 h. Control cells were treated with serum free medium (RPMI-1640) only or with DMSO (c = 1.0 v/v %) at 37 °C for 1 h. After incubation the cells were washed twice with serum free (RPMI-1640) medium. To determine the in vitro cytostatic effect, the cells were cultured for a further 72 h in 10% serum containing medium. MTT-solution (45 mL, 2 mg/mL, final concentration: 0.37 mg/mL) was added to each well. The respiratory chain [26, 27] and other electron transport systems [28] reduce MTT and thereby form non-watersoluble violet formazane crystals within the cell [29]. The amount of these crystals can be determined spectrophotometrically and serves as an estimate for the number of mitochondria and hence the number of living cells in the well [30]. After 4 h of incubation the cells were centrifuged for 5 min (900 g) and the supernatant was removed. The obtained formazane crystals were dissolved in DMSO (100 mL) and optical density (OD) of the samples was measured at λ = 540 and 620 nm, respectively, using ELISA Reader (iEMS Reader, Labsystems, Finland). OD620 values were substracted from OD540 values. The percent of cytostasis was calculated by using the following equation: Cytostatic effect (%) = [1-(ODtreated/ODcontrol)]x100 Values ODtreated and ODcontrol correspond to the optical densities of the treated and the control cells, respectively. In each case two independent experiments were carried out with 4 parallel measurements. The 50% inhibitory concentration (IC₅₀) values were determined from the dose-response curves. The curves were defined using Microcal TM Origin1 (version 7.5) software: cytostasis was plotted as a function of concentration, fitted to a sigmoidal curve, and based on this curve, the half maximal inhibitory concentration (IC₅₀) value was determined. IC₅₀ represents the concentration of a compound that is required for 50% inhibition in vitro.

### High-throughput screening on EBC-1 and H2228 lung cancer cell lines using CellTiter-Glo luminescent cell viability assay.

EBC-1 (obtained from JRCB, https://cellbank.nibiohn.go.jp/english/) and H2228 (obtained from ATCC https://www.lgcstandards-atcc.org/) cell lines were maintained according to the instructions provided by JRCB and ATCC in a 5% CO₂ humidified incubator maintained at 37 °C temperature.. Effect of compounds on cell viability was measured via CellTiter-Glo^{®} luminescent cell viability assay (Promega, Madison, WI, USA). Cells were plated at 1000 cells/well onto a flat-bottomed, white 96 well plate (BRANDplates, cat. no.: 781965). After 24 h, cells were treated for 72 h with the compounds at 100 nM concentration. After the treatment, medium was removed and CellTiter-Glo^{®} reagent was added. Untreated cells were used as control. The luminescence signal was recorded using a microplate reader (BioTek Synergy 2 Multi-Mode Reader, BioTek, Winooski, VT, USA). Cell viability data (% of untreated control cells) was evaluated with Microsoft Excel.

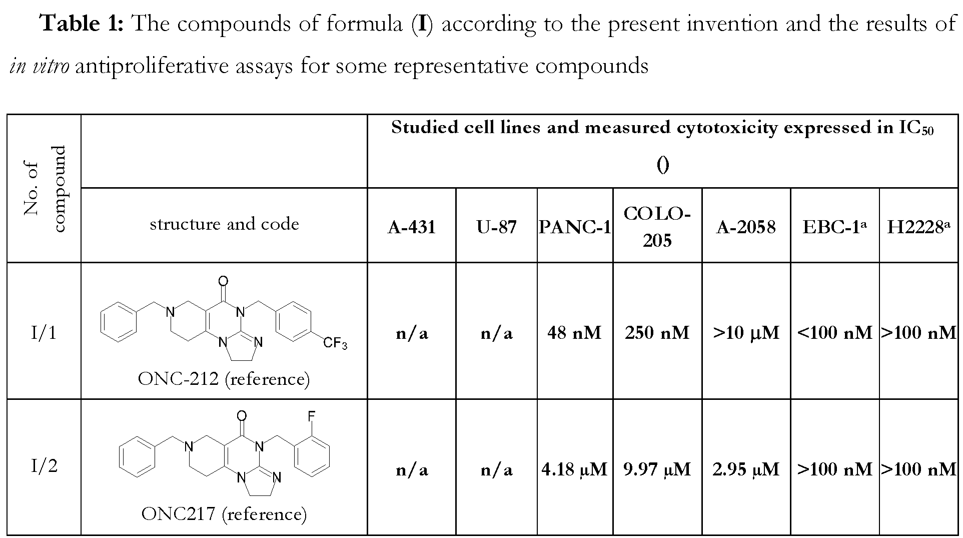

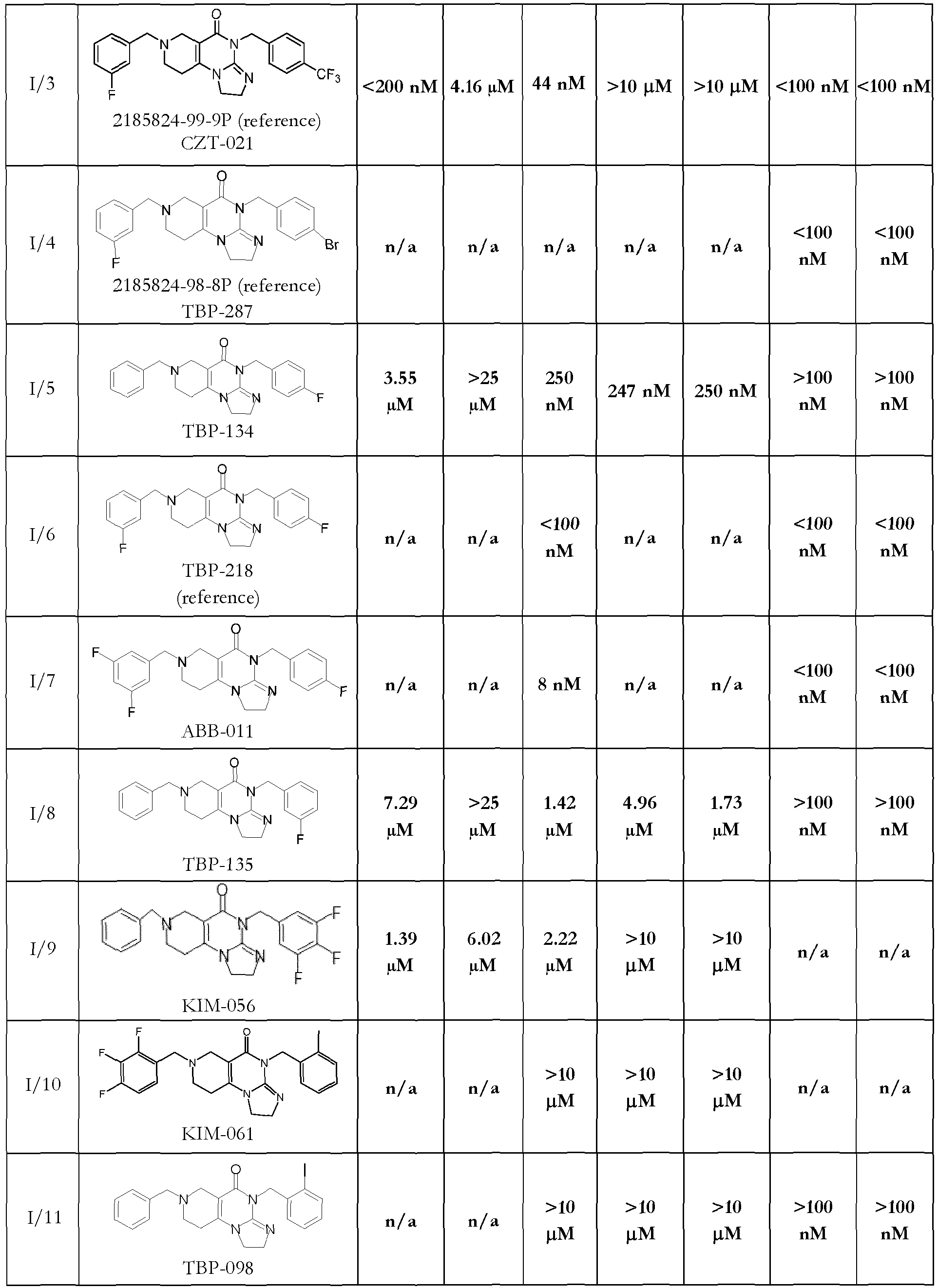

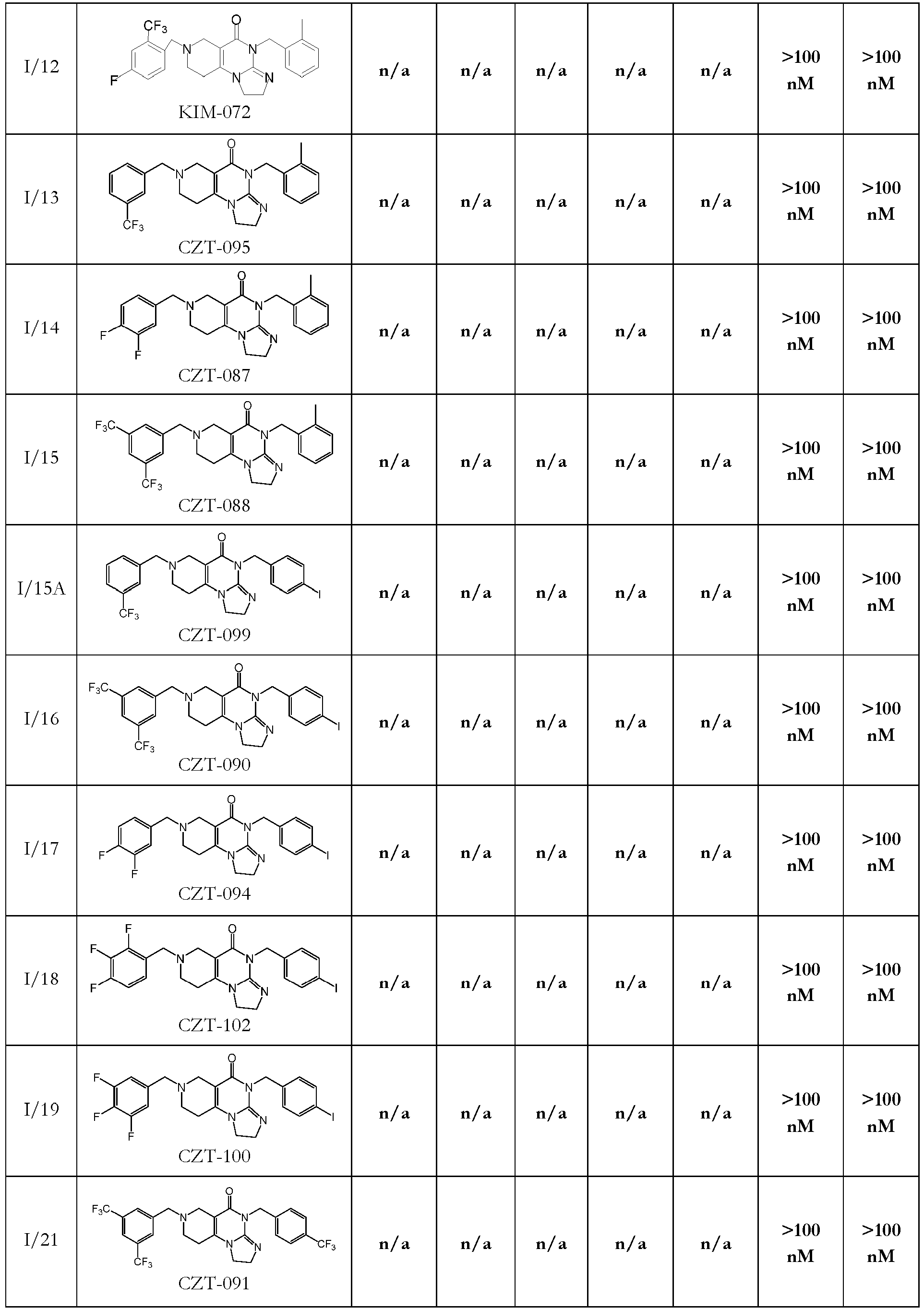

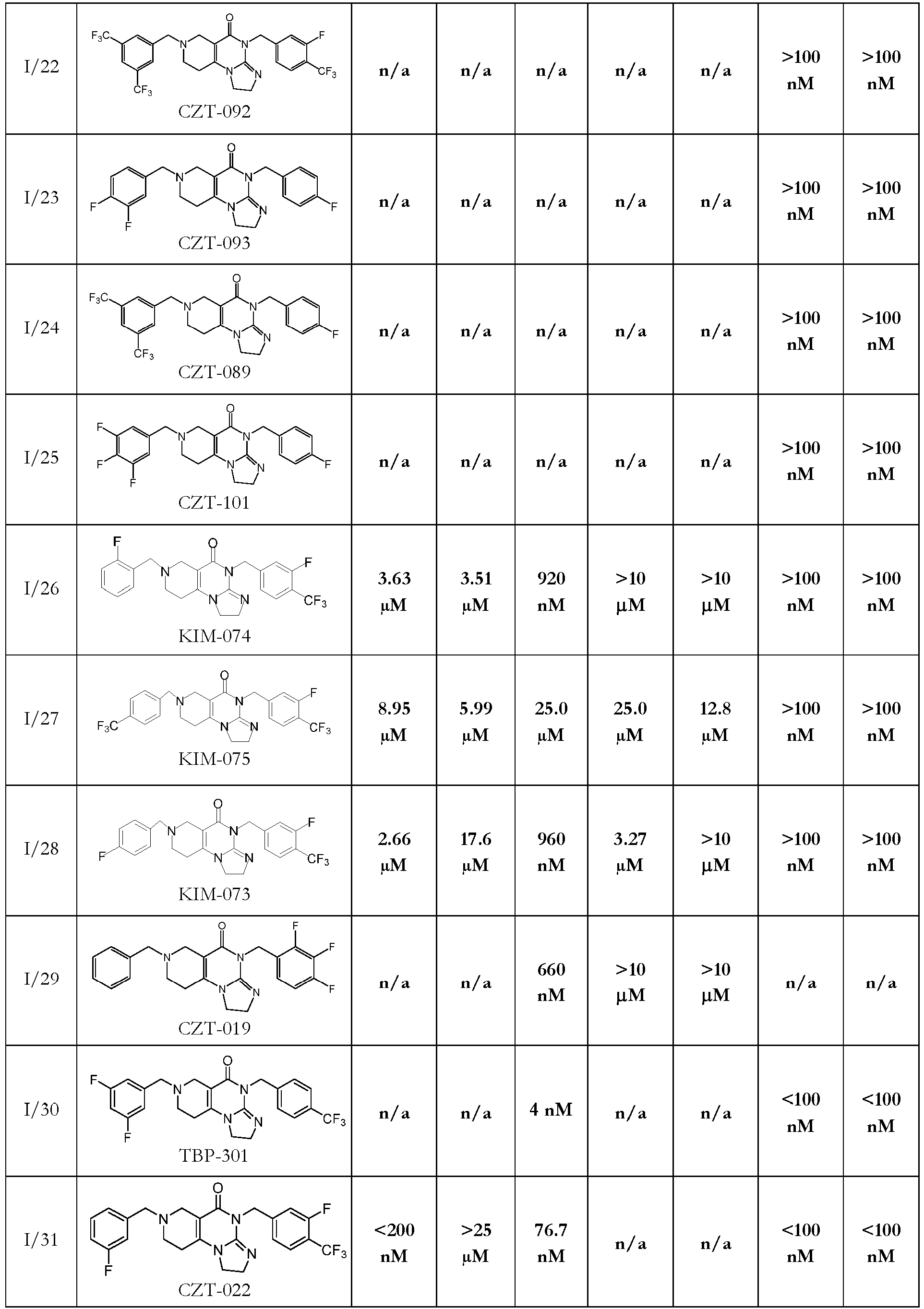

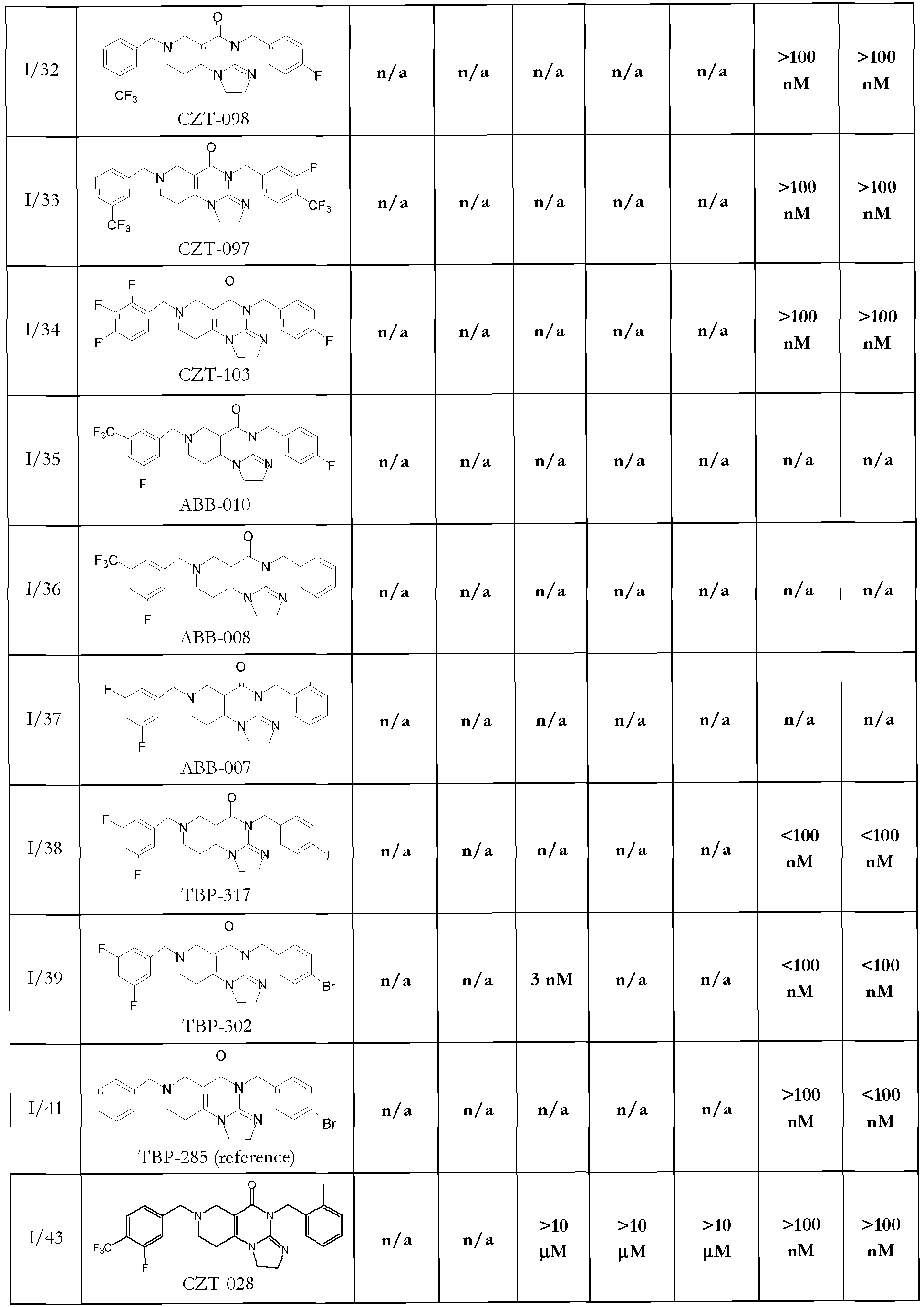

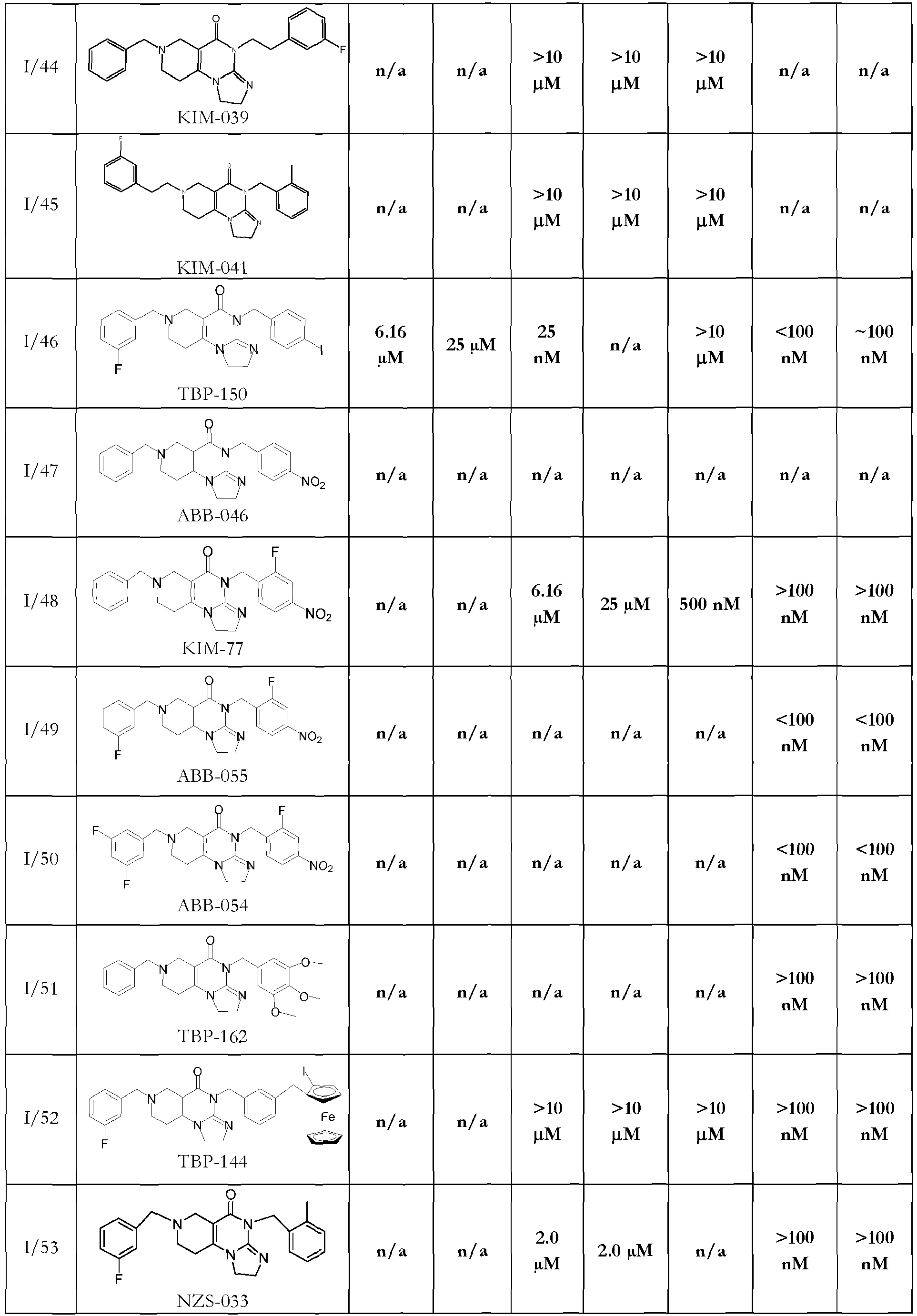

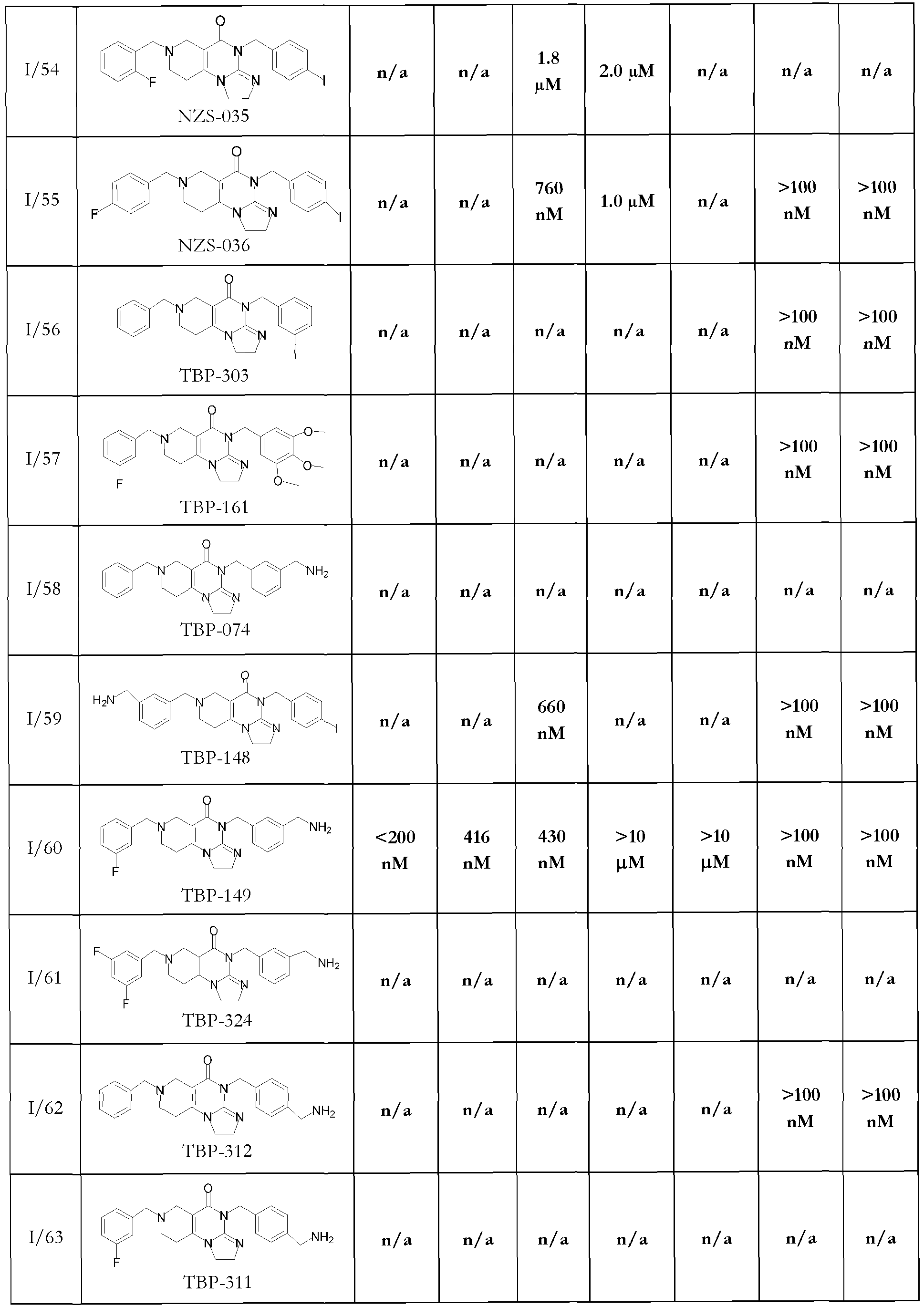

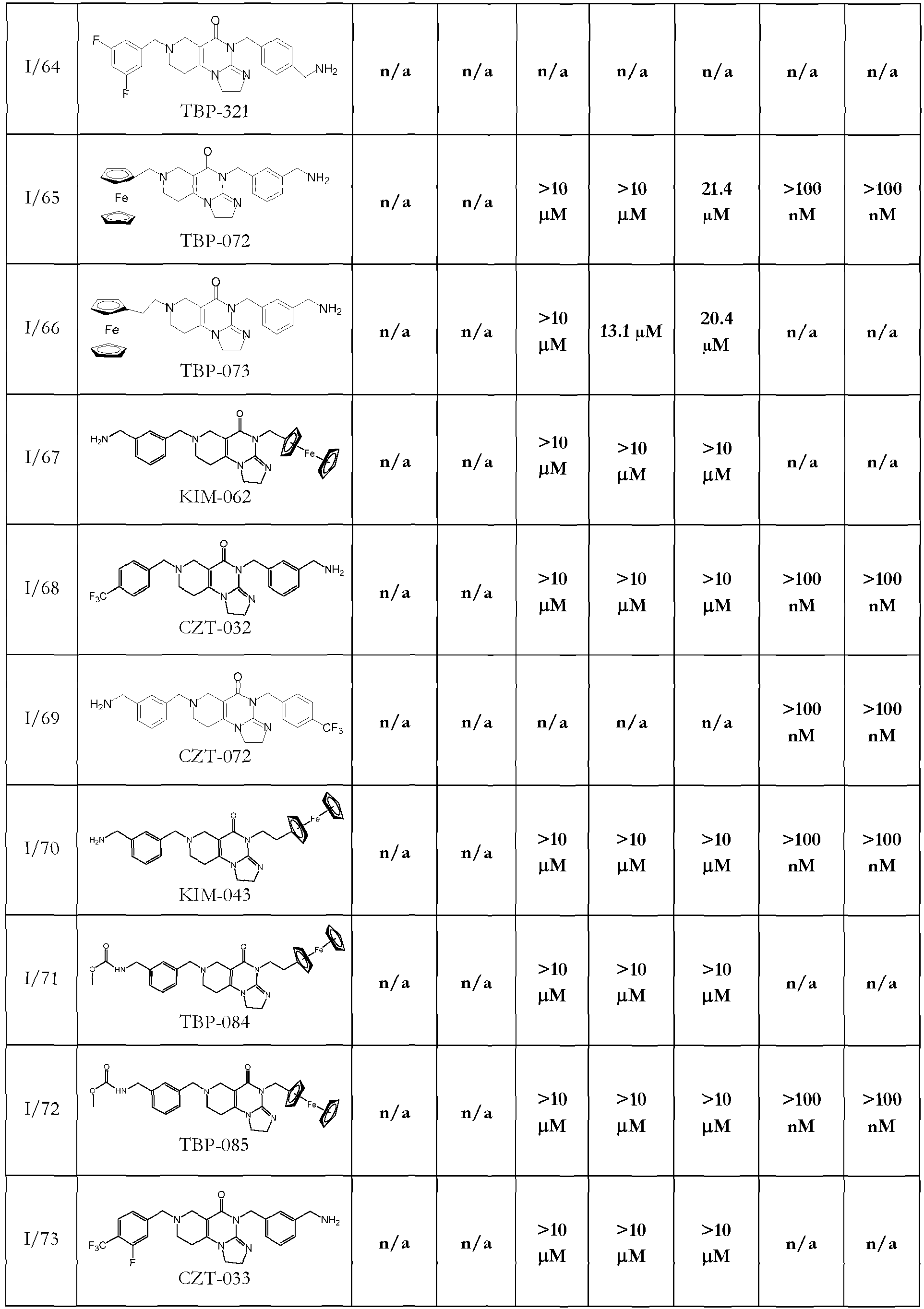

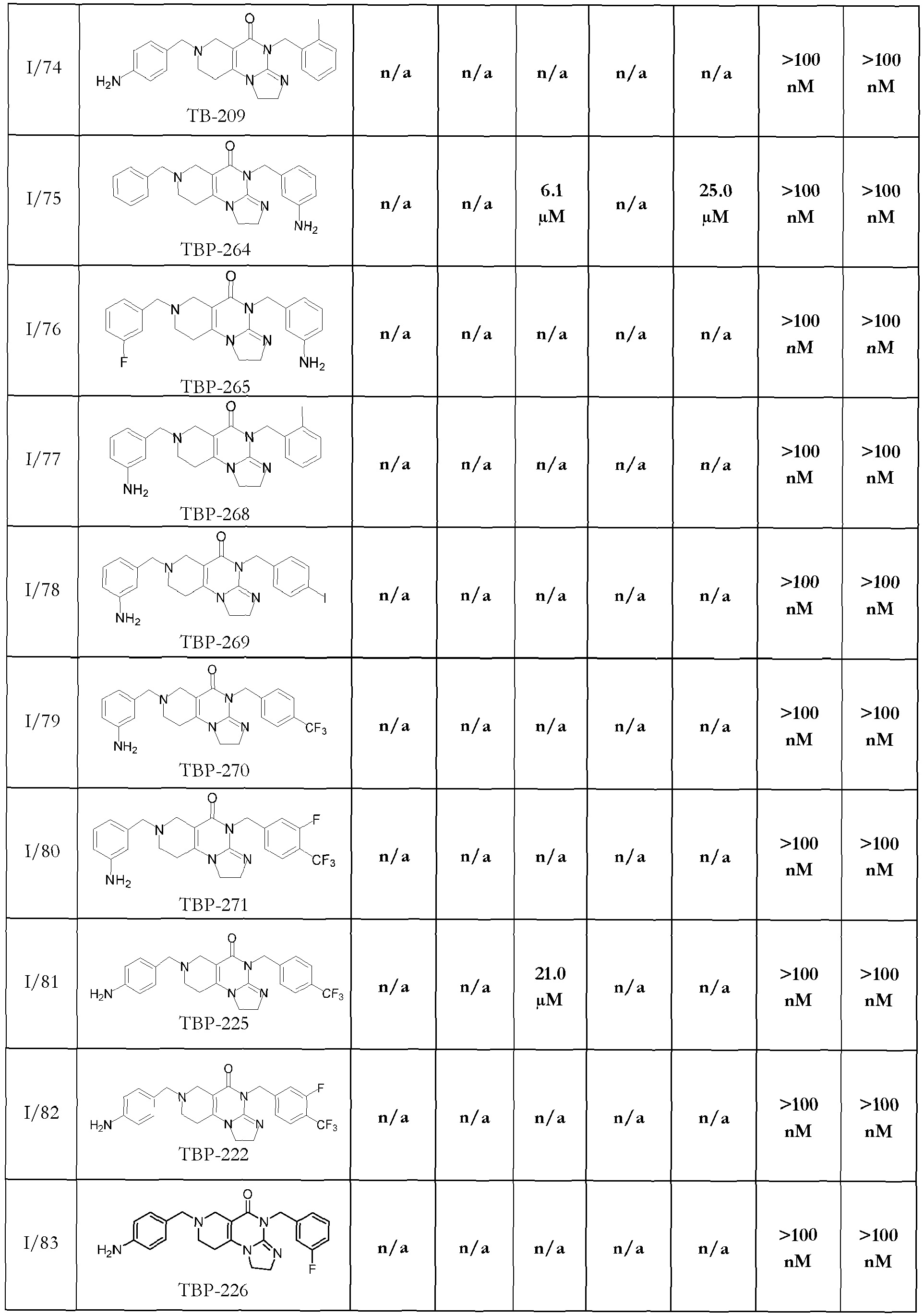

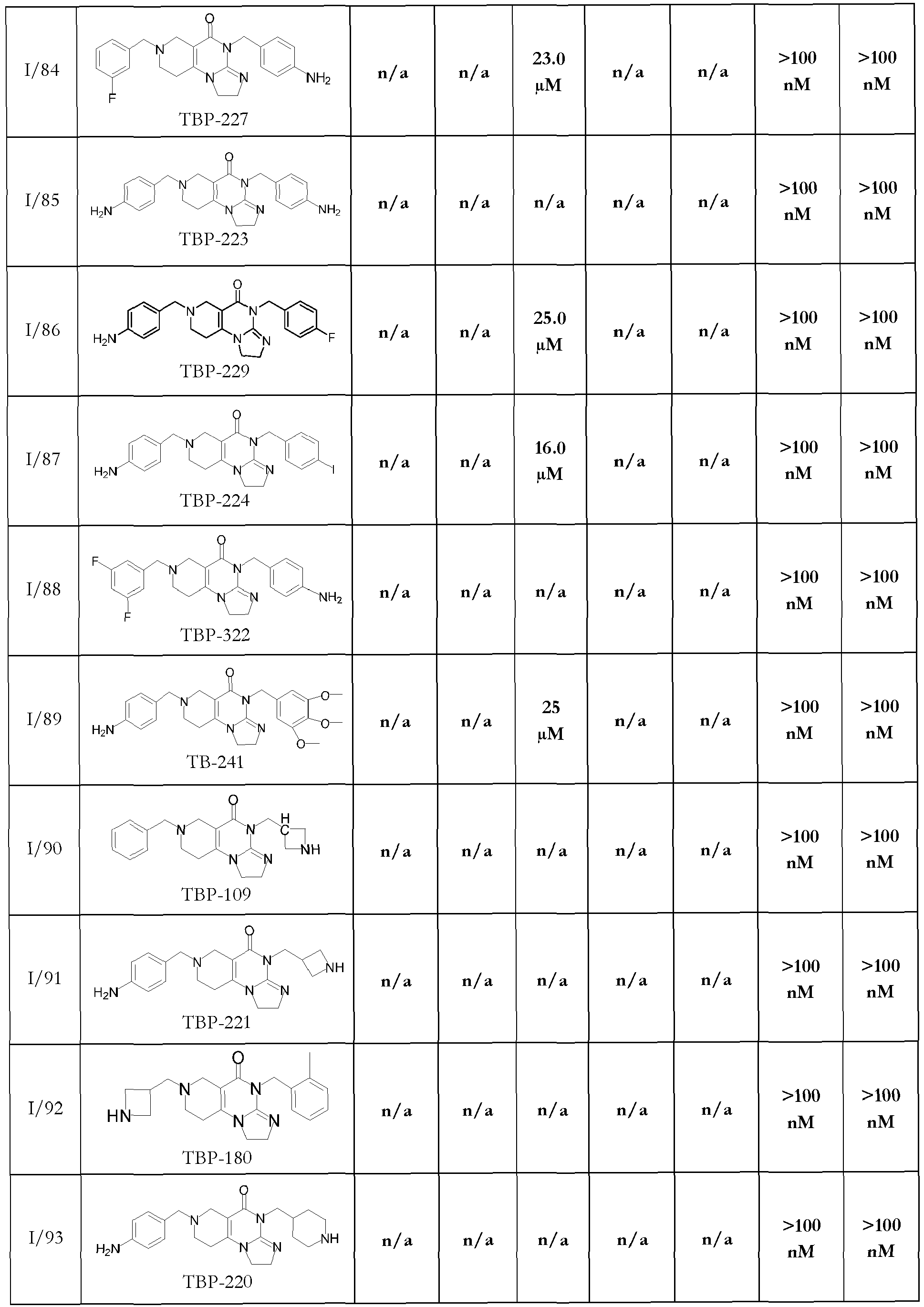

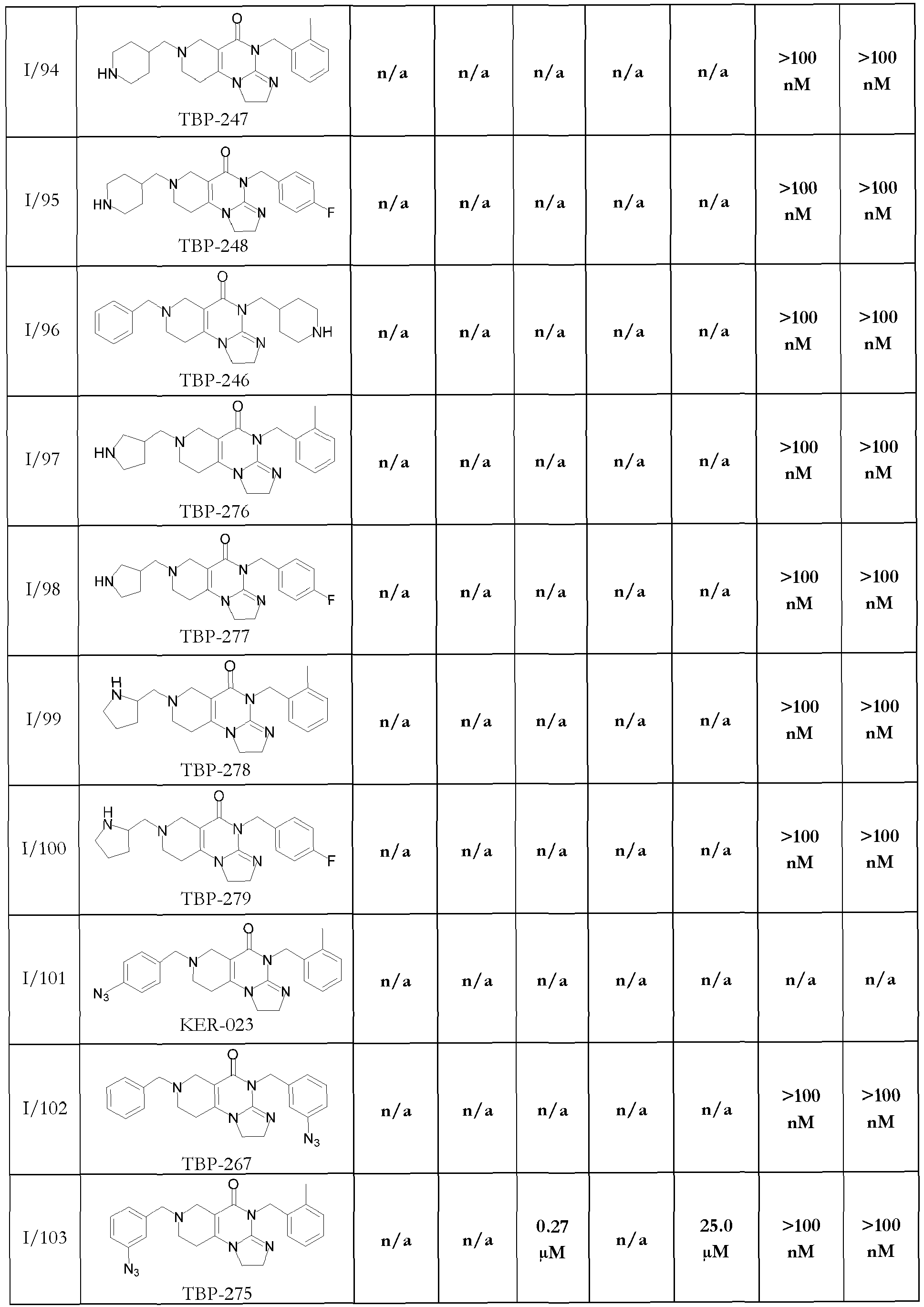

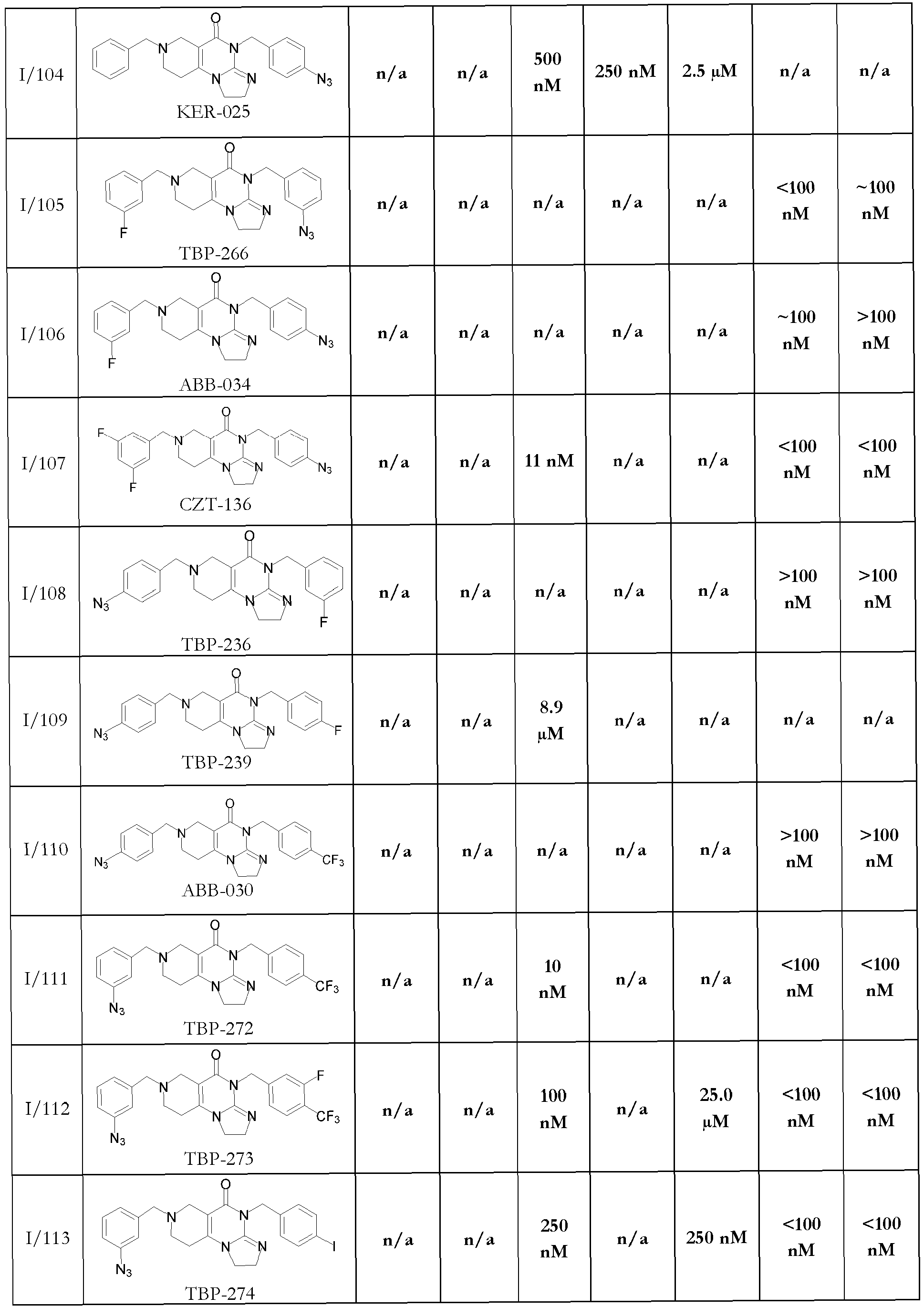

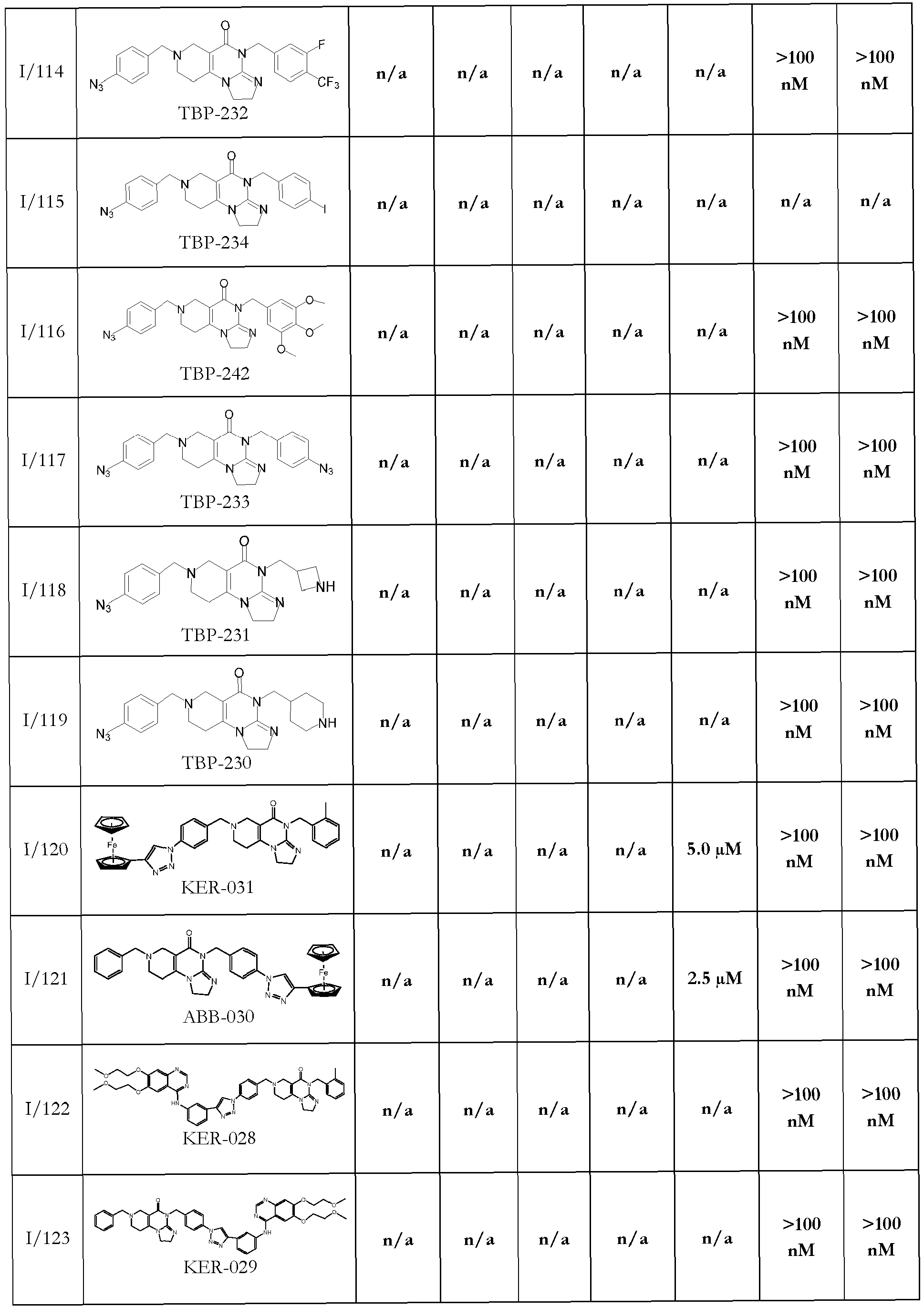

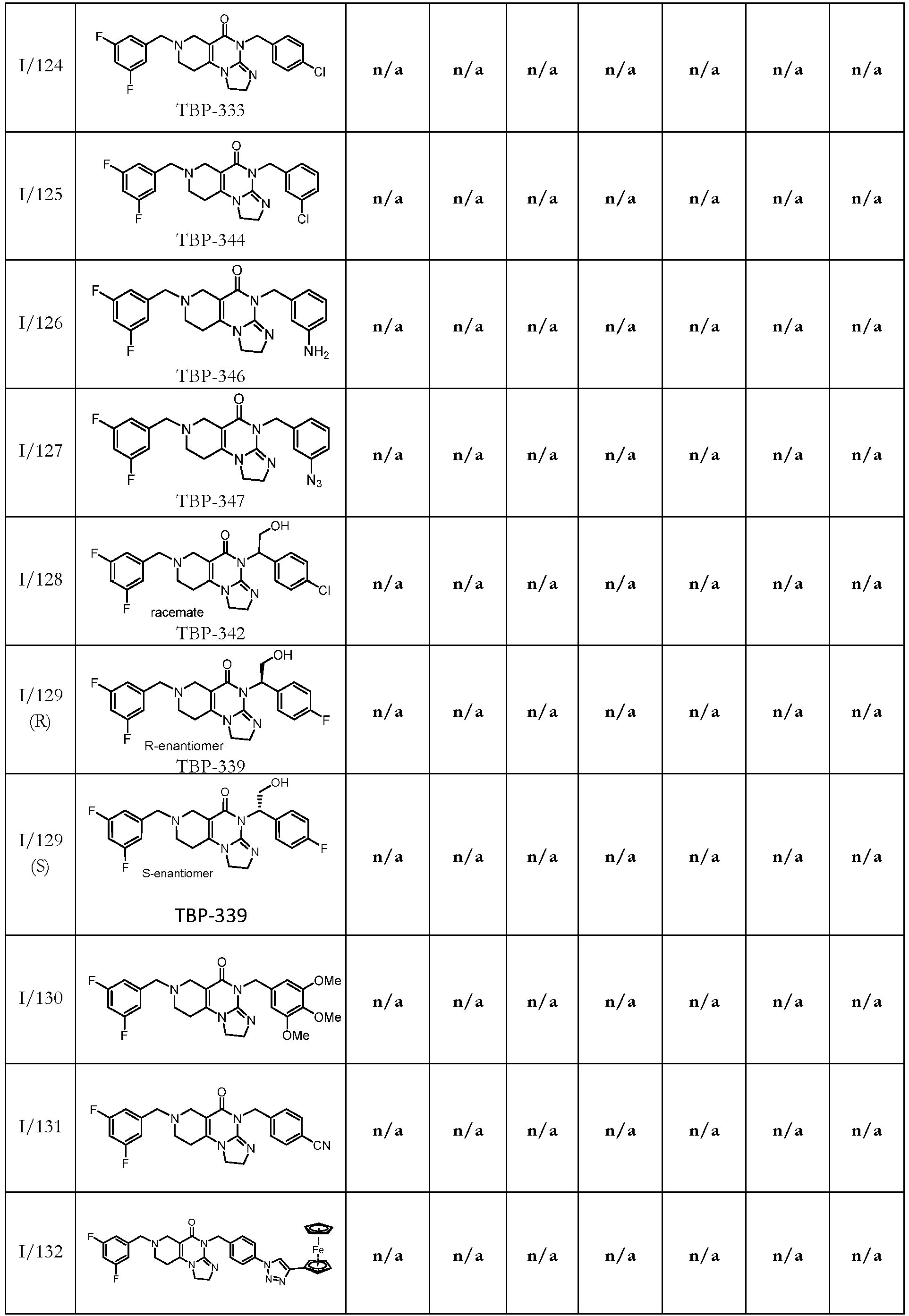

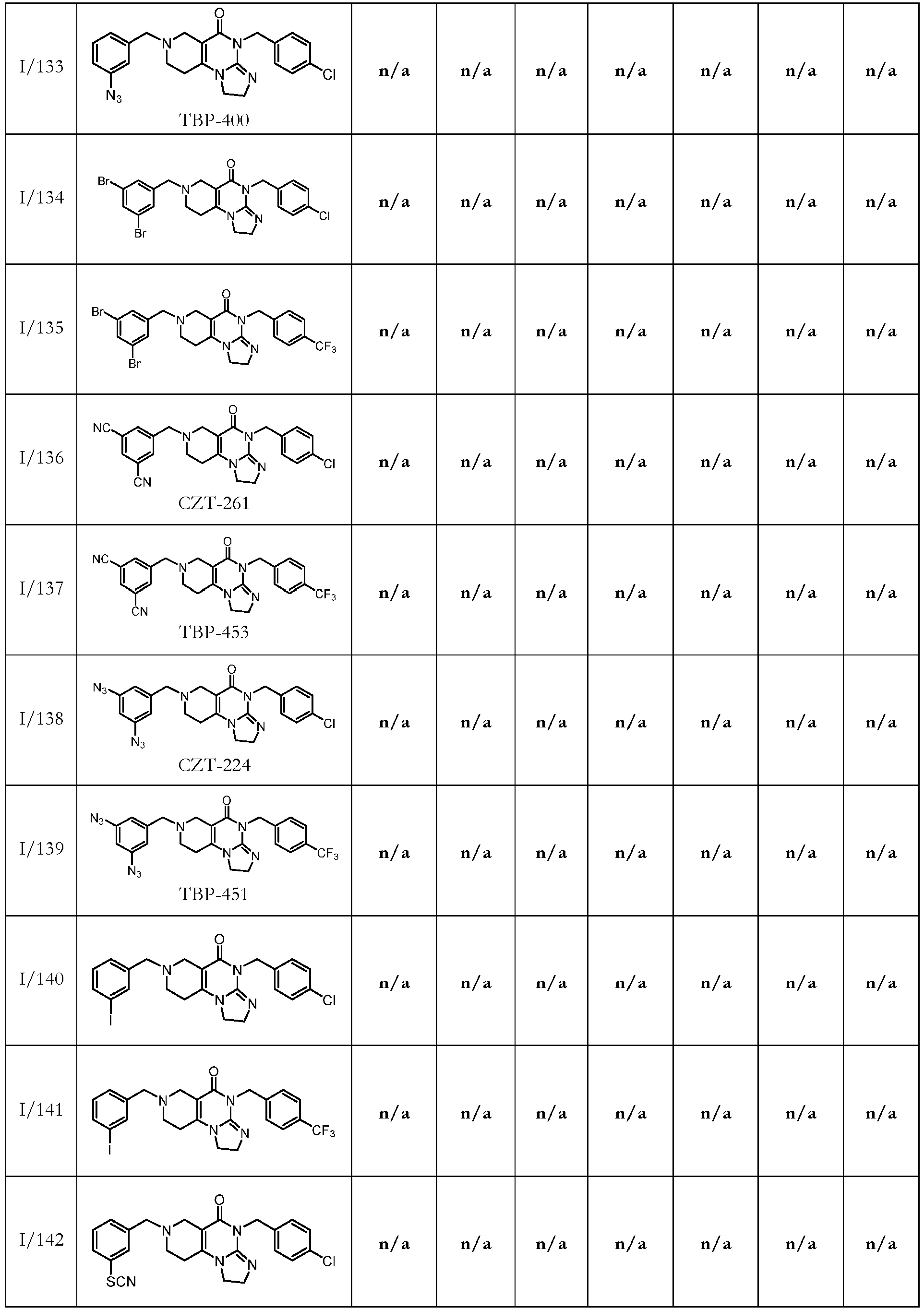

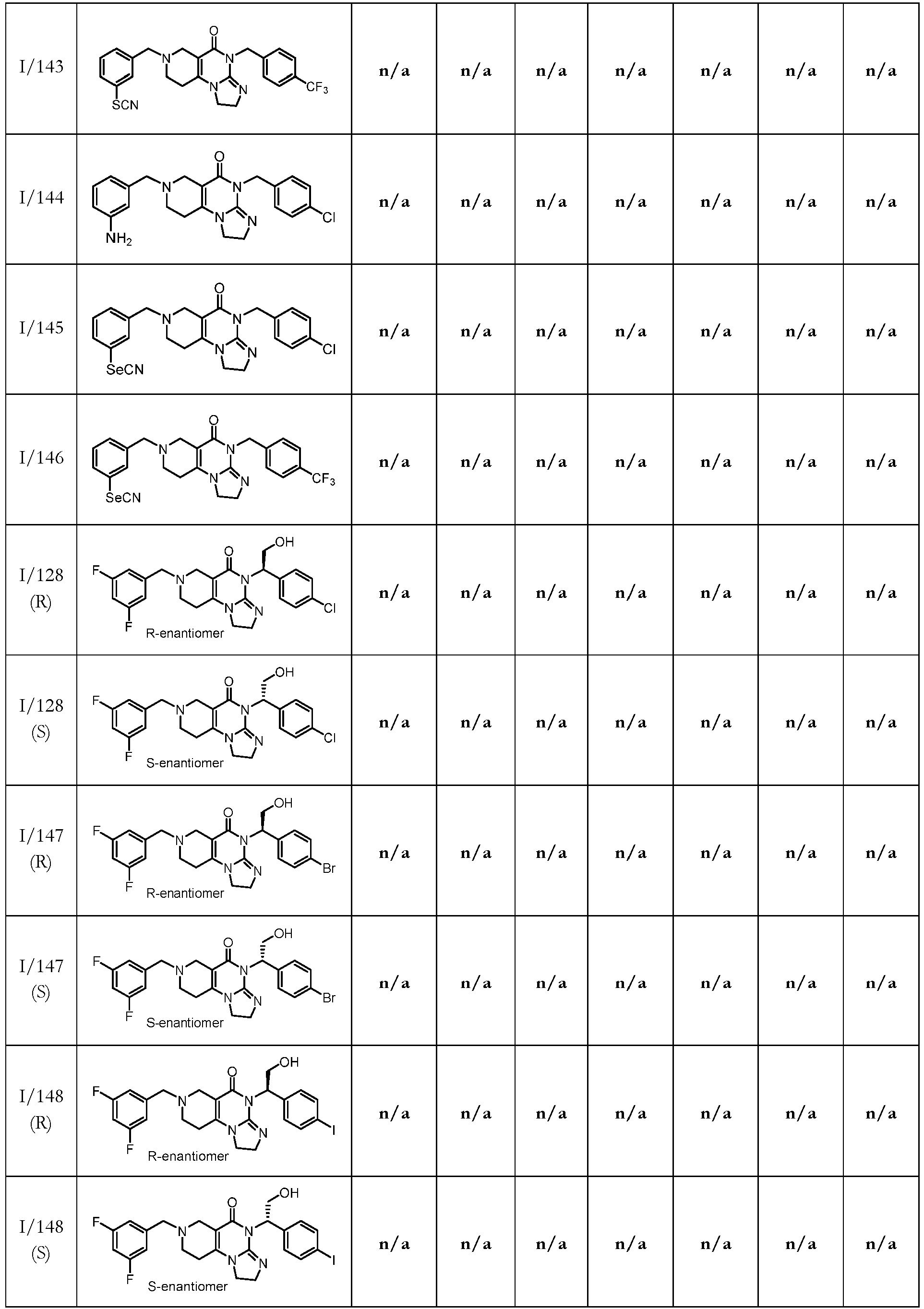

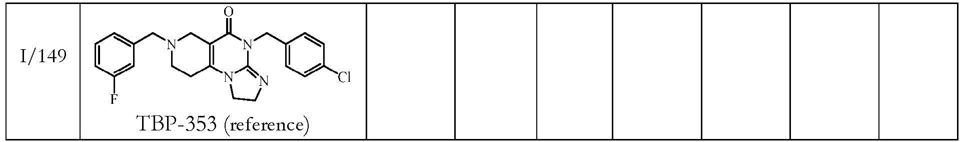

### ^{a} The results of High throughput screening (HTS) are presented on lung cancer cell lines EBC-1 and H2228. In subsequent experiments IC₅₀ values were determined for the compounds proved to be potent in the HTS (Table 3).

### Example 19:

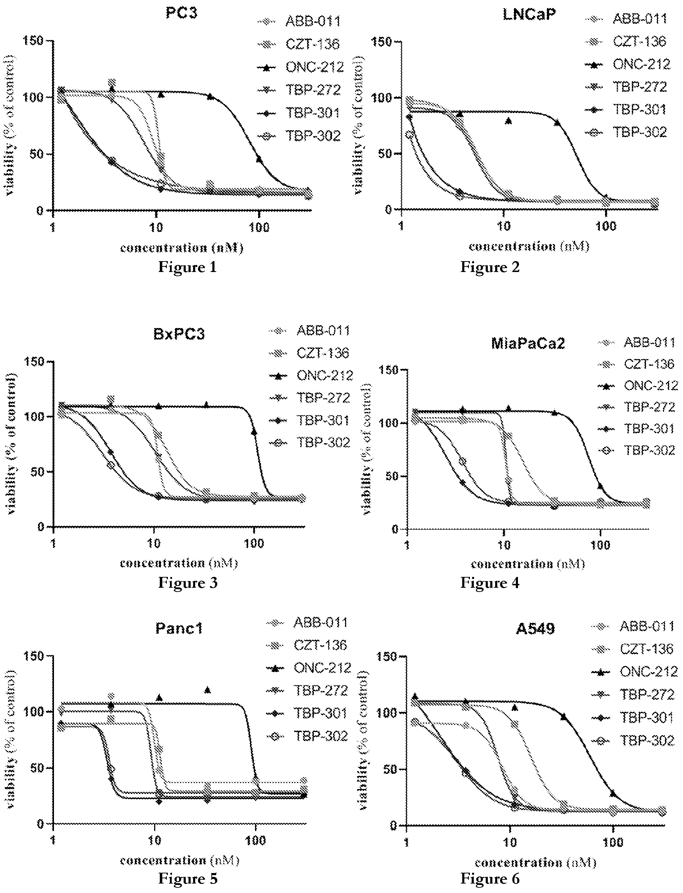

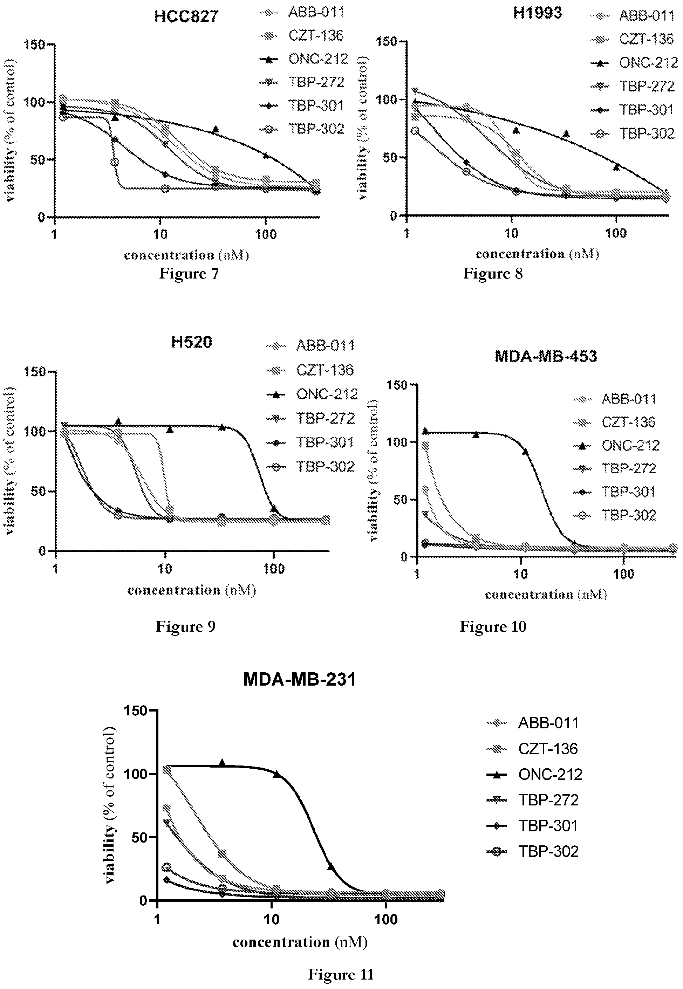

Some representative imipridones and ONC212 (Reference Compound 2) as reference were further tested on the following human malignant cell lines: PC3 and LNCap (prostate carcinoma); BxPC3, MiaPaCa2 and Panc1 (pancreatic cancer); A549, HCC827, H1993 and H520 (lung carcinoma); MDA-MB-453 and MDA-MB-231 (breast cancer) (see Table 2 and Figures 1-11).

Cell lines were maintained according to the instructions provided by ATCC (https://www.lgcstandards-atcc.org/ ) in a 5% CO₂ humidified incubator maintained at 37 °C temperature. Effects of compounds on cell viability were measured via CellTiter-Glo^{®} luminescent cell viability assay (Promega, Madison, WI, USA) Cells were plated at 1000 cells/well onto a flat-bottomed, white 96 well plate (BRANDplates, cat. no.: 781965). After 24 h, cells were treated for 72 h with 3-fold serial diluted compound concentrations (300 nM, 100 nM, 33.3 nM, 11.1 nM, 3.7 nM, 1.2 nM). After the treatment, medium was removed and CellTiter-Glo^{®} reagent was added. The luminescence signal was recorded using a microplate reader (BioTek Synergy 2 Multi-Mode Reader, BioTek, Winooski, VT, USA).

Cell viability data (% of untreated control cells) was evaluated with Microsoft Excel. Dose-response curves (using a non-linear regression model, log (inhibitor) vs response, variable slope) were generated and IC₅₀ values were determined using Graph Pad Prism 5.02 software (GraphPad Software, San Diego, CA, USA).

In these tests our compounds proved to be extremely efficient antiproliferative agents characterized by IC50 values in low nanomolar range far superior to ONC212 (Reference Compound 2). Particularly I/30 (TBP-301) and I/39 (TBP-302) can be regarded as exceptionally potent anticancer agents. On the other hand, the potent azide derivatives I/107 (CZT-136) and I/111 (TBP-272) provide unique possibility to identify cellular targets, thus to disclose certain mechanisms of action indispensable to drug-approval.

**Table 2: The results of in vitro tests (IC₅₀ [nM]) of some representative compounds of the invention and ONC212 (as a reference) on a set of human malignant cell lines**

| No. of compound (code) | **PC3** | **LNCaP** | **BxPC3** | **MiaPaCa2** | **Panc1** | **A549** | **HCC827** | **H1993** | **H520** |
|---|---|---|---|---|---|---|---|---|---|
| I/1 (ONC212) (reference) | 80 | 52 | 110 | 76 | 74 | 60 | 105 | 90 | 73 |
| I/7 (ABB-011) | 9 | 5 | 12 | 11 | 11 | 8 | 13 | 10 | 7 |
| I/107 (CZT-136) | 11 | 5 | 14 | 16 | 14 | 16 | 15 | 12 | 8 |
| I/111 (TBP-272) | 7 | 5 | 13 | 10 | 9 | 8 | 12 | 6 | 6 |
| I/30 (TBP-301) | 2 | 2 | 4 | 3 | 3 | 4 | 5 | 4 | 3 |
| I/39 (TBP-302) | 2 | 2 | 3 | 4 | 4 | 4 | 4 | 3 | 3 |
| I/124 (TBP-333) | | | | | 2 | | | | |
| I/61 TBP-324 | | | | | 300 | | | | |
| 1/129 (R) TBP-339 (R) | | | | | 15 | | | | |
| I/129 (S) TBP-339 (S) | | | | | 265 | | | | |
| I/128 TBP-342 | | | | | 10 | | | | |

### Example 20:

Additional data for demonstrating the effectiveness of compounds of the invention.

**Table 3: The results of additional in vitro tests (IC₅₀ [nM]) of representative compounds of invention and ONC212 (as a reference) on a set of human malignant cell lines**

| No. of compound (code) | **MDA-MB-453** | **MDA-MB-231** | **EBC-1** | **H2228** | **SCC-25** | **Detroit 562** | **Fadu** |
|---|---|---|---|---|---|---|---|
| I/1 (ONC212) (reference) | 16 | 24 | 76 | 105 | 166 | 32 | 90 |
| I/7 (ABB-011) | 1 | 2 | 9 | 10 | 13 | 3 | 10 |
| I/107 (CZT-136) | 2 | 3 | 17 | 24 | 31 | 7 | 19 |
| I/111 (TBP-272) | <1 | 1 | 8 | 13 | 15 | 3 | 11 |
| I/30 (TBP-301) | <1 | <1 | 5 | 5 | 6 | 2 | 5 |
| I/39 (TBP-302) | <1 | <1 | 4 | 6 | 7 | 3 | 5 |
| I/124 (TBP-333) | | | 3 | 3 | 4 | <1 | 2 |
| I/61 (TBP-324) | | | 29 | 104 | 89 | 84 | 22 |
| I/126 (TBP-346) | | | 288 | 269 | >300 | 103 | 290 |
| I/127 (TBP-347) | | | 16 | 15 | 21 | 10 | 16 |
| I/129 (S) (TBP-339 (S)) | | | >300 | 300 | >300 | 120 | 150 |
| I/128 (TBP-342) | | | 13 | 12 | 19 | 5 | 12 |

### Example 21: Cell Viability Assay Protocols

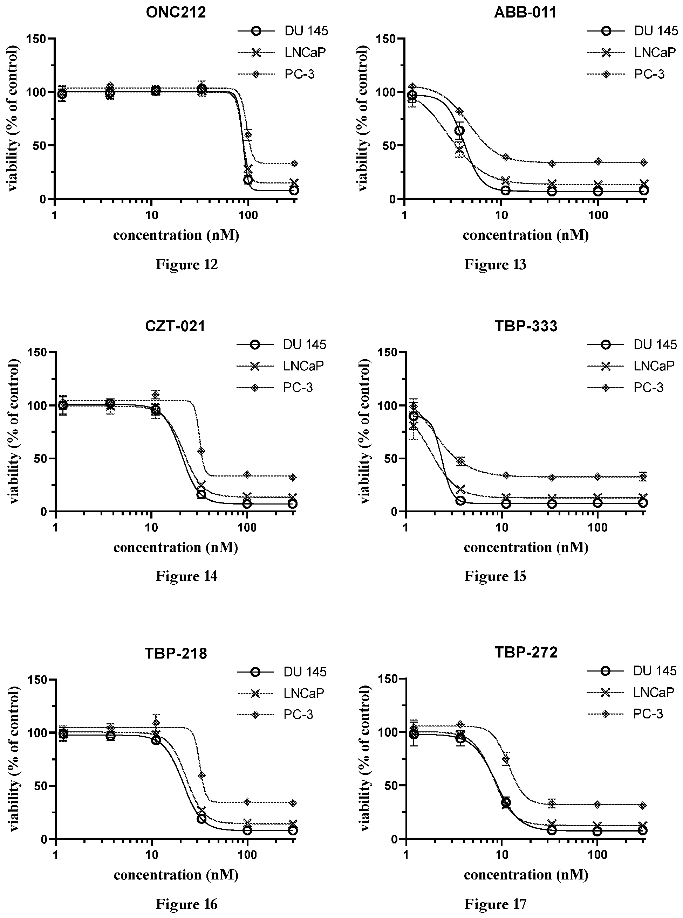

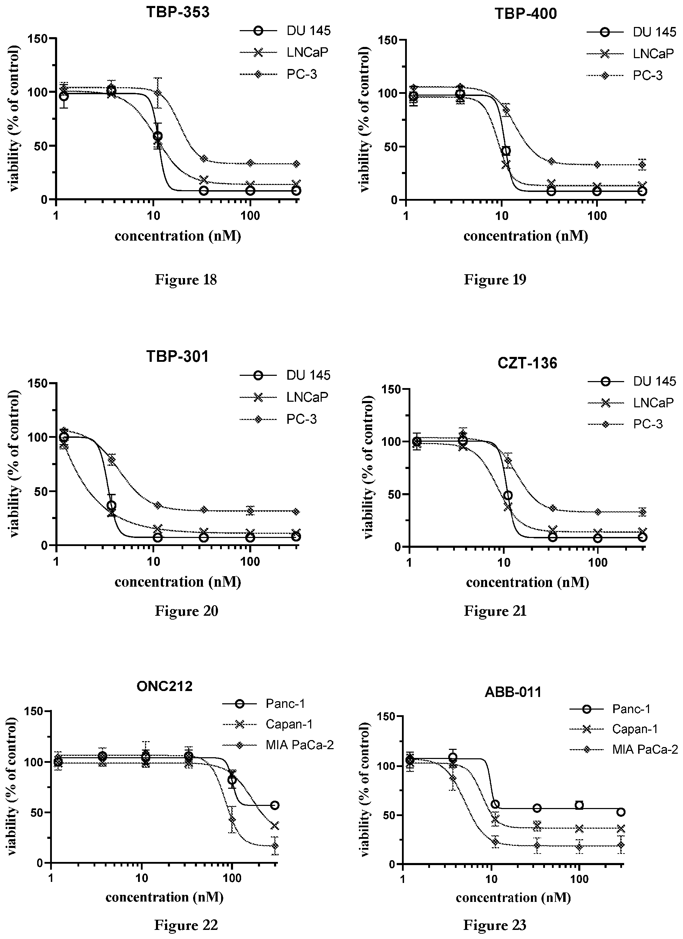

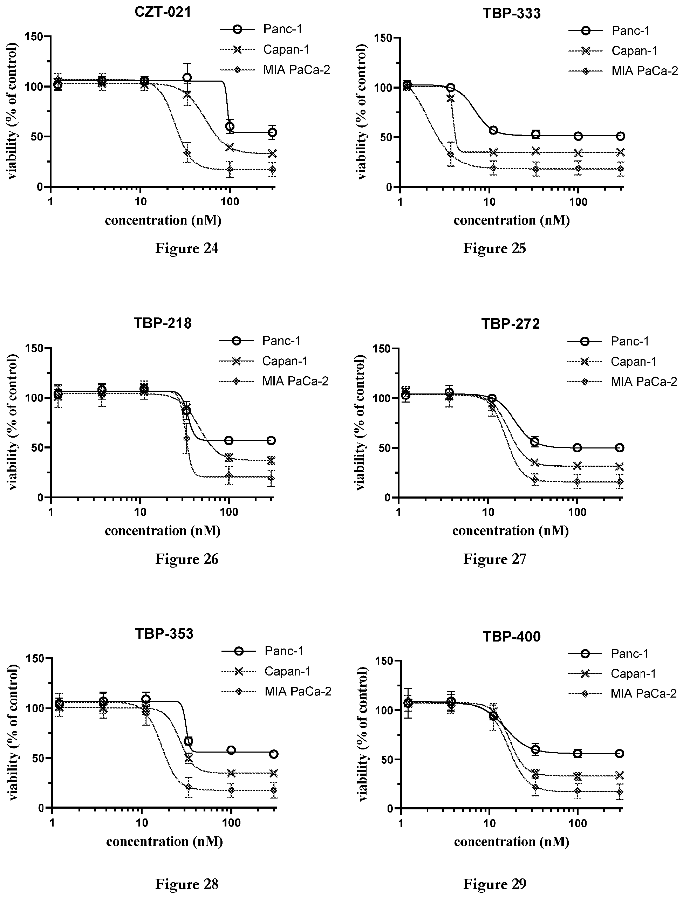

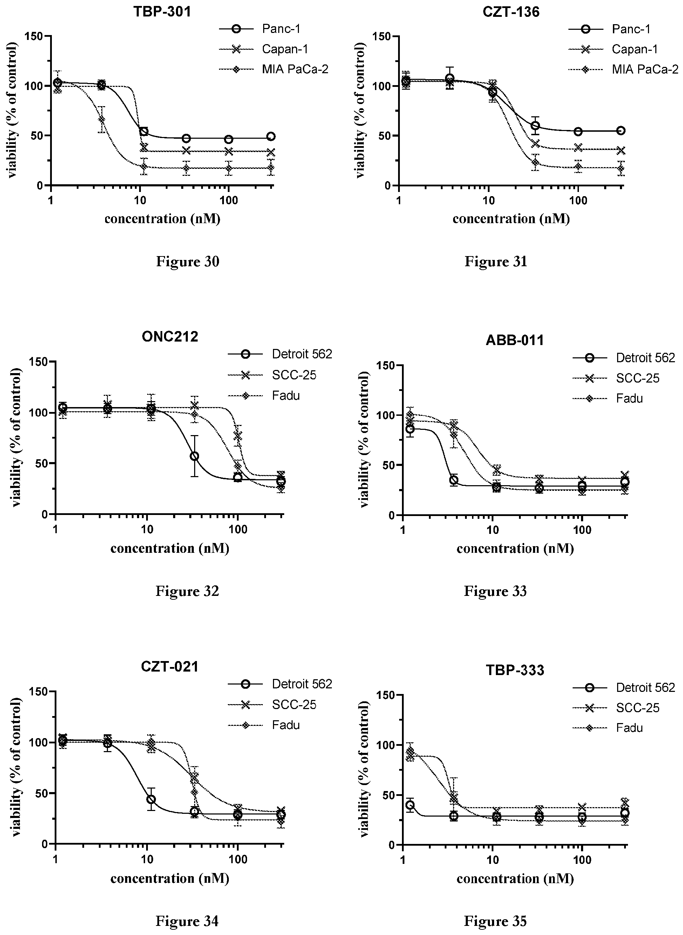

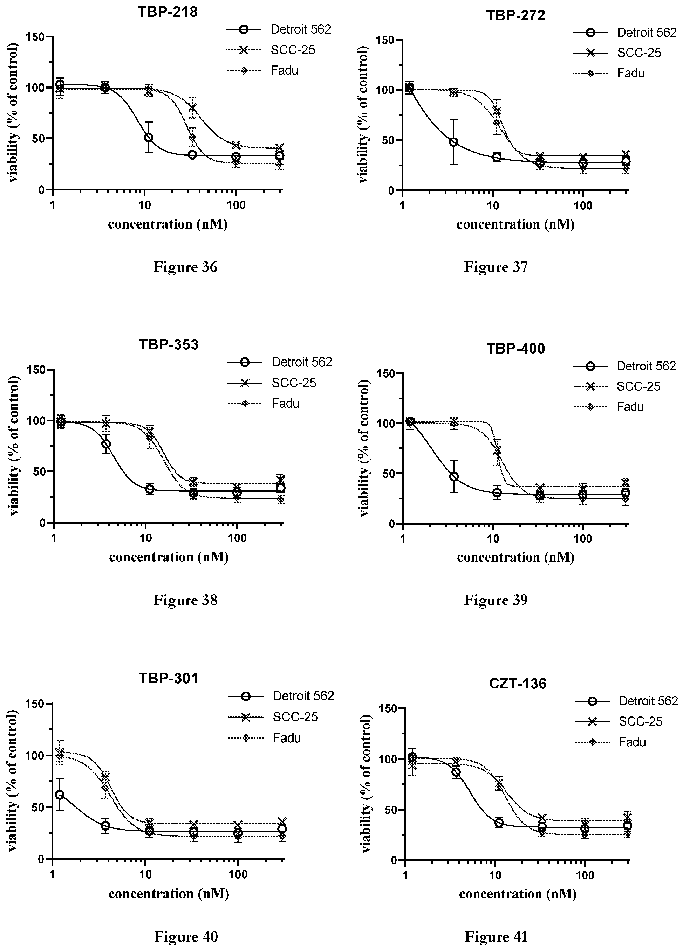

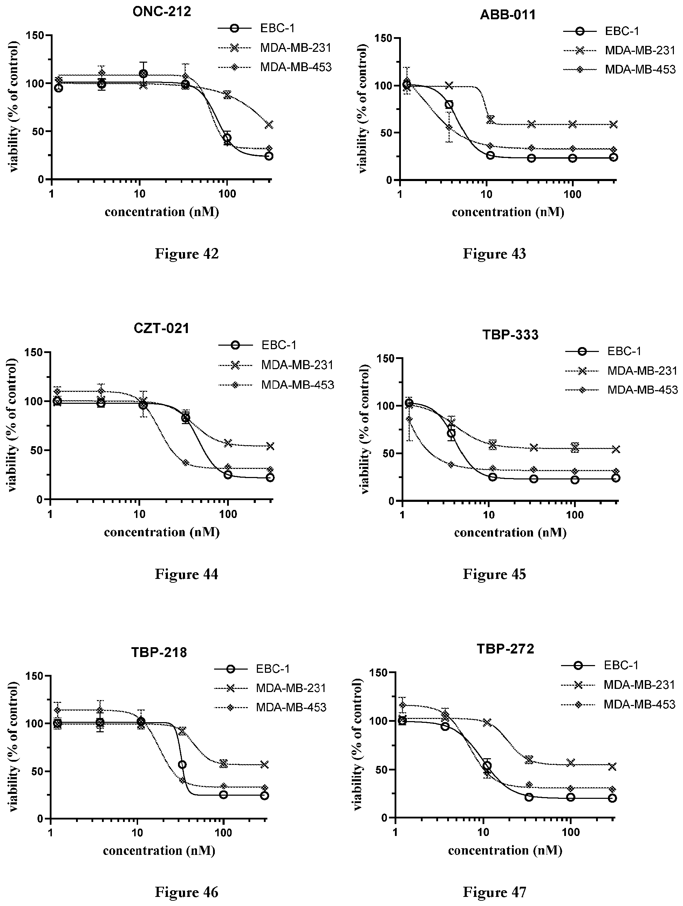

Effect of the selected compounds on cancer cell viability was measured via CellTiter-Glo^{®} luminescent cell viability assay (Promega, Madison, WI, USA) according to the manufacturer's instructions. Cells were plated onto a flat-bottomed, white 96 well plate (BRANDplates^{®}, cat. no.: 781965). Density of the seeded cells was optimized based on their size and growth rate, as the follows: Panc-1:750 cells/well; DU 145, PC-3, Capan-1, MIA PaCa-2, SCC-25, FaDu and EBC-1: 1000 cells/well; LNCaP, Detroit 562, MDA-MB-231 and MDA-MB-453 1500 cells/well. After 48 h incubation, cells were treated for 72 h with 3-fold serial diluted compound concentrations (300 nM - 1.2 nM range) Untreated cells (incubated during the 72 h treatment in the corresponding cell culture medium) were used as control. After the treatment, the luminescence signal was recorded using a microplate reader (BioTek Synergy 2 Multi-Mode Reader, BioTek, Winooski, VT, USA). Dose-response curves were generated by GraphPad Prism 8.4.2. software using a non-linear regression model (variable slope, four parameters), (see Figures 12-51).

**Table 4: Cell viability measurements on DU 145 cell line**

| **DU 145** (prostate, carcinoma) cell viability (% of control) | | | | | | |
|---|---|---|---|---|---|---|
| | **300 nM** | **100 nM** | **33.3 nM** | **11.1 nM** | **3.7 nM** | **1.2 nM** |
| I/1 (ONC-212) (ref.) | **8.1** ± 2.2 | **17.5** ± 4.2 | **103.1** ± 2.5 | **101.3** ± 4.3 | **99.0 ± 4.6** | **98.2** ± 6.9 |
| I/3 (CZT-021) (ref.) | **7.2** ± 1.2 | **7.1** ± 1.6 | **15.7** ± 4.1 | **95.9** ± 5.1 | **102.0** ± 3.5 | **100.3** ± 7.5 |
| I/6 (TBP-218) (ref.) | **7.9** ± 1.6 | **8.0** ± 1.4 | **18.7** ± 2.8 | **93.4** ± 2.2 | **96.8** ± 3.7 | **99.0** ± 6.3 |
| I/149 (TBP-353) (ref.) | **8.1** ± 0.2 | **8.0** ± 1.6 | **7.8 ±** 1.2 | **59.1** ± 11.9 | **101.0** ± 4.1 | **95.6** ± 11.3 |
| I/30 (TBP-301) | **8.0** ± 2.9 | **7.0** ± 1.1 | **6.6** ± 1.0 | **7.0** ± 0.9 | **37.2** ± 10.4 | **99.6** ± 6.9 |
| I/7 (ABB-011) | **8.5** ± 2.4 | **7.2** ± 2.3 | **7.4** ± 1.4 | **7.5** ± 0.6 | **64.1** ± 8.0 | **97.3** ± 7.2 |
| I/124 (TBP-333) | **7.7** ± 1.8 | **8.0** ± 1.0 | **7.0** ± 0.9 | **7.0** ± 1.1 | **10.0 ±** 1.4 | **90.2** ± 12.9 |
| I/111 (TBP-272) | **8.1** ± 1.9 | **7.1** ± 2.0 | **7.7 ±** 1.3 | **33.8** ± 5.0 | **93.6** ± 6.9 | **97.7** ± 10.8 |
| I/133 (TBP-400) | **8.1** ± 2.6 | **8.0** ± 2.0 | **7.6 ±** 1.1 | **45.7** ± 4.4 | **98.8** ± 6.7 | **97.1** ± 9.4 |
| I/107 (CZT-136) | **9.0** ± 2.9 | **8.3** ± 2.9 | **8.7** ± 1.9 | **48.7 ±** 1.7 | **101.2** ± 6.5 | **100.2** ± 7.7 |

**Table 5: Cell viability measurements on LNCaP cell line**

| **LNCaP** (prostate, carcinoma) cell viability (% of control) | | | | | | |
|---|---|---|---|---|---|---|
| | **300 nM** | **100 nM** | **33.3 nM** | **11.1 nM** | **3.7 nM** | **1.2 nM** |
| I/1 (ONC-212) (ref.) | **15.2** ± 1.7 | **28.1** ± 1.7 | **100.6** ± 4.5 | **101.4** ± 3.3 | **98.6** ± 5.7 | **99.4** ± 6.7 |
| I/3 (CZT-021) (ref.) | **12.7** ± 1.2 | **13.8** ± 1.7 | **24.9** ± 2.2 | **95.0** ± 7.4 | **98.7** ± 7.4 | **99.7** ± 8.8 |
| I/6 (TBP-218) (ref.) | **14.4** ± 0.7 | **15.4** ± 2.0 | **26.6** ± 2.3 | **97.9** ± 7.3 | **101.8** ± 5.6 | **99.1** ± 7.2 |
| I/149 (TBP-353) (ref.) | **13.7 ±** 1.1 | **13.4** ± 0.8 | **18.0** ± 1.4 | **54.5** ± 6.4 | **98.4** ± 3.5 | **99.7** ± 1.9 |
| I/30 (TBP-301) | **11.3** ± 1.1 | **11.4** ± 0.6 | **11.8** ± 0.2 | **15.0** ± 0.4 | **29.6** ± 2.5 | **93.6** ± 5.0 |
| I/7 (ABB-011) | **13.9** ± 1.0 | **13.1** ± 0.8 | **13.8** ± 0.9 | **17.2** ± 1.9 | **45.6** ± 7.0 | **95.0** ± 8.5 |
| I/124 (TBP-333) | **13.0** ± 0.7 | **12.7** ± 0.8 | **12.4** ± 1.2 | **13.3** ± 1.6 | **20.8** ± 2.0 | **80.9** ± 12.5 |
| I/111 (TBP-272) | **11.7** ± 0.7 | **11.8** ± 0.7 | **13.6** ± 0.4 | **32.4** ± 2.3 | **97.1** ± 2.6 | **99.6** ± 3.9 |
| I/133 (TBP-400) | **13.3** ± 1.2 | **12.3** ± 1.1 | **14.7 ±** 1.4 | **33.4** ± 3.1 | **95.9** ± 6.1 | **95.8** ± 5.1 |
| I/107 (CZT-136) | **13.6** ± 0.6 | **13.5** ± 0.7 | **16.3** ± 1.9 | **37.9** ± 3.3 | **95.3** ± 2.3 | **98.1** ± 3.4 |

**Table 6: Cell viability measurements on PC-3 cell line**

| **PC-3** (prostate, adenocarcinoma) cell viability (% of control) | | | | | | |
|---|---|---|---|---|---|---|
| | **300 nM** | **100 nM** | **33.3 nM** | **11.1 nM** | **3.7 nM** | **1.2 nM** |
| I/1 (ONC-212) (ref.) | **33.3**±3.3 | **60.4**±5.2 | **104.5**±5.2 | **102.4**±3.7 | **106.0**±2.8 | **100.9** ± 4.2 |
| I/3 (CZT-021) (ref.) | **32.3**±2.6 | **34.7**±1.0 | **56.5**±3.3 | **110.2**±4.1 | **100.8**±4.0 | **102.9** ± 5.1 |
| I/6 (TBP-218) (ref.) | **34.1**±2.0 | **35.3**±0.5 | **60.0**±3.3 | **108.7**±7.7 | **103.6**±4.4 | **100.8** ± 2.4 |
| I/149 (TBP-353) (ref.) | **33.1**±3.3 | **33.6**±2.2 | **38.4**±1.0 | **98.6**±13.6 | **104.6**±6.5 | **102.7** ± 6.5 |
| I/30 (TBP-301) | **31.3**±2.5 | **32.1**±3.6 | **33.0**±1.9 | **36.6**±1.8 | **78.9**±4.6 | **106.0** ± 2.9 |
| I/7 (ABB-011) | **34.3**±3.4 | **34.6**±3.0 | **32.5**±3.1 | **38.9**±2.1 | **82.1**±1.6 | **104.6** ± 3.3 |
| 1/124 (TBP-333) | **32.7**±4.4 | **32.8**±3.2 | **32.1**±3.4 | **33.9**±3.3 | **46.9**±4.2 | **99.4** ± 7.1 |
| I/111 (TBP-272) | **31.3**±3.0 | **32.0**±3.2 | **33.2**±3.5 | **75.5**±5.9 | **106.8**±2.4 | **103.7** ± 7.0 |
| 1/133 (TBP-400) | **32.9**±4.6 | **32.6**±3.3 | **36.2**±2.2 | **83.5**±6.4 | **105.6**±1.8 | **105.4** ± 2.9 |
| I/107 (CZT-136) | **32.8**±4.1 | **32.7**±2.6 | **37.2**±1.9 | **82.3**±7.0 | **107.1**±5.9 | **100.3** ± 3.0 |

**Table 7: Cell viability measurements on Panc-1 cell line**

| **Panc-1** (pancreas, carcinoma) cell viability (% of control) | | | | | | |
|---|---|---|---|---|---|---|
| | **300 nM** | **100 nM** | **33.3 nM** | **11.1 nM** | **3.7 nM** | **1.2 nM** |
| I/1 (ONC-212) (ref.) | **56.9**±2.4 | **81.8**±8.1 | **105.8**±5.8 | **104.6** ± 7.2 | **105.8**±0.5 | **100.1**±2.0 |
| I/3 (CZT-021) (ref) | **53.9**±6.5 | **59.9**±7.3 | **109.4**±13.5 | **105.7** ± 4.2 | **105.8**±4.0 | **101.8**±5.8 |
| I/6 (TBP-218) (ref.) | **57.2**±3.2 | **57.1**±0.1 | **86.8**±8.7 | **109.1** ± 4.2 | **107.8**±6.0 | **104.4**±3.8 |
| I/149 (TBP-353) (ref.) | **54.0**±3.1 | **58.3**±2.5 | **66.7**±3.6 | **109.1** ± 6.9 | **107.1**±9.0 | **103.6**±5.7 |
| I/30 (TBP-301) | **49.1**±0.2 | **46.4**±3.1 | **46.6**±2.4 | **54.4** ± 3.5 | **100.8**±3.4 | **103.3**±3.3 |
| I/7 (ABB-011) | **53.5**±0.6 | **60.0**±3.6 | **57.0**±2.0 | **61.1** ± 3.4 | **108.6**±7.6 | **105.9**±8.4 |
| I/124 (TBP-333) | **51.2**±0.4 | **50.9**±0.3 | **52.7**±4.3 | **56.9** ± 0.2 | **99.7**±3.0 | **102.6**±4.0 |
| I/111 (TBP-272) | **49.7**±1.2 | **50.1**±2.0 | **56.1**±4.9 | **99.6** ± 1.4 | **106.1**±7.3 | **103.3**±7.0 |
| I/133 (TBP-400) | **55.7**±2.5 | **56.1**±3.7 | **60.2**±6.0 | **94.1** ± 4.0 | **108.9**±10.2 | **107.0**±14.9 |
| I/107 (CZT-136) | **55.0**±3.2 | **54.2**±0.6 | **60.1**±9.1 | **93.6** ± 8.1 | **107.9**±11.5 | **104.6**±7.6 |

**Table 8: Cell viability measurements on Capan-1 cell line**

| **Capan-1** (pancreas, adenocarcinoma) cell viability (% of control) | | | | | | |
|---|---|---|---|---|---|---|
| | **300 nM** | **100 nM** | **33.3 nM** | **11.1 nM** | **3.7 nM** | **1.2 nM** |
| I/1 (ONC-212) (ref.) | **36.8**±3.4 | **88.1**±4.2 | **98.9 ±** 5.4 | **99.2 ±** 3.8 | **100.3** ± 4.0 | **99.0**±7.1 |
| I/3 (CZT-021) (ref) | **33.0**±2.2 | **38.7**±2.8 | **91.6** ± 10.6 | **101.5** ± 5.5 | **102.7** ± 6.9 | **104.9**±8.1 |
| I/6 (TBP-218) (ref) | **37.4**±3.5 | **40.1**±3.7 | **90.2** ± 5.9 | **106.0** ± 8.0 | **105.9** ± 7.8 | **100.8**±11.1 |
| I/149 (TBP-353) (ref.) | 3**5.3**±3.3 | **34.8**±1.9 | **49.6** ± 5.0 | **99.6** ± 6.1 | **99.9** ± 9.6 | **101.0**±8.6 |
| I/30 (TBP-301) | **33.1**±1.7 | **34.0**±2.8 | **34.7 ±** 2.0 | **38.4** ± 4.5 | **100.8** ± 5.3 | **98.5**±5.3 |
| I/7 (ABB-011) | **35.5**±3.3 | **36.1**±2.8 | **38.5** ± 5.3 | **46.0** ± 7.5 | **101.9** ± 3.8 | **102.9**±9.2 |
| I1/124 (TBP-333) | **34.6**±2.4 | **33.6**±1.1 | **36.1** ± 1.7 | **35.3** ± 3.1 | **88.5** ± 1.8 | **100.8**±3.8 |
| I/111 (TBP-272) | **30.8**±3.3 | **31.7**±1.7 | **34.5** ± 3.3 | **95.1** ± 8.2 | **102.5** ± 4.0 | **107.5**±2.2 |
| I/133 (TBP-400) | **34.0**±2.8 | **32.6**±4.0 | **36.4 ±** 3.8 | **98.8** ± 7.9 | **108.3** ± 8.3 | **107.0**±8.1 |
| I/107 (CZT-136) | **34.7**±2.9 | **37.6**±2.3 | **42.2** ± 3.0 | **101.2 ±** 5.2 | **103.5** ± 3.0 | **105.0**±4.7 |

**Table 9: Cell viability measurements on MIA PaCa-2 cell line**

| **MIA PaCa-2** (pancreas, carcinoma) cell viability (% of control) | | | | | | |
|---|---|---|---|---|---|---|
| | **300 nM** | **100 nM** | **33.3 nM** | **11.1 nM** | **3.7 nM** | **1.2 nM** |
| I/1 (ONC-212) (ref.) | **17.3**±8.8 | **43.4**±13.5 | **106.1**± 8.9 | **108.5**±11.2 | **105.7**±8.5 | **105.1**±4.9 |
| I/3 (CZT-021) (ref.) | **17.2**±7.5 | **17.5**±7.9 | **34.2**±10.0 | **105.2**±3.4 | **107.1**±6.4 | **107.4**±2.5 |
| I/6 (TBP-218) (ref.) | **19.5**±7.5 | **21.6**±9.1 | **58.7**±14.1 | **110.7**±6.4 | **101.6**±10.8 | **107.0**±5.9 |
| I/149 (TBP-353) (ref.) | **18.2**±8.3 | **17.8**±6.8 | **21.1**±10.1 | **95.0**±11.8 | **104.7**±9.9 | **106.9**±7.5 |
| I/30 (TBP-301) | **17.6**±7.6 | **16.7**±6.7 | **16.8**±7.1 | **19.1**±8.0 | **65.7**±12.6 | **105.3**±10.4 |
| I/7 (ABB-011) | **19.8**±8.6 | **18.5**±6.8 | **18.9**±8.3 | **23.1**±6.3 | **87.4**±12.0 | **107.3**±4.0 |
| I/124 (TBP-333) | **18.0**±7.3 | **18.7**±7.1 | **17.8**±7.0 | **18.9**±7.1 | **33.5**±12.1 | **102.7**±2.6 |
| I/111 (TBP-272) | **16.2**±6.9 | **16.5**±6.7 | **18.0**±5.9 | **90.6**±9.3 | **100.2**±8.8 | **106.6**±5.1 |
| I/133 (TBP-400) | **17.4**±7.9 | **18.4**±7.8 | **21.6**±8.9 | **92.1**±13.2 | **104.0**±7.3 | **110.2**±2.5 |
| I/107 (CZT-136) | **17.4**±7.1 | **18.8**±6.2 | **23.1±**7.8 | **91.5**±6.6 | **102.5**±5.4 | **106.6**±3.0 |

**Table 10: Cell viability measurements on Detroit 562 cell line**

| **Detroit 562** (pharynx, carcinoma) cell viability (% of control) | | | | | | |
|---|---|---|---|---|---|---|
| | **300 nM** | **100 nM** | **33.3 nM** | **11.1 nM** | **3.7 nM** | **1.2 nM** |
| I/1 (ONC-212) (ref.) | **32.0**±5.0 | **35.8**±4.4 | **57.0**±20.1 | **104.0**±7.2 | **104.1**±4.8 | **104.6**±4.5 |
| I/3 (CZT-021) (ref.) | **28.7**±4.1 | **28.9**±5.3 | **31.8**±4.9 | **44.3**±11.0 | **99.5**±8.4 | **101.7**±5.1 |
| I/6 (TBP-218) (ref.) | **33.0**±3.4 | **31.6**±2.1 | **34.2**±3.2 | **50.9**±15.2 | **99.8**±6.4 | **103.1**±7.4 |
| I/149 (TBP-353) (ref.) | **34.1**±4.4 | **29.9**±4.5 | **29.3**±4.6 | **33.4**±5.2 | **76.7**±9.1 | **99.2**±5.8 |
| I/30 (TBP-301) | **28.7**±2.5 | **24.7**±2.9 | **26.1**±3.8 | **27.0**±5.5 | **31.9**±6.7 | **61.9**±14.5 |
| I/7 (ABB-011) | **33.0**±4.8 | **28.6**±3.6 | **27.0**±4.9 | **28.4**±4.2 | **35.1**±5.8 | **85.6**±7.7 |
| I/124 (TBP-333) | **31.5**±3.7 | **28.3**±3.6 | **28.1**±3.9 | **27.8**±3.6 | **29.4**±4.9 | **40.4**±6.5 |
| I/111 (TBP-272) | **28.8**±3.8 | **26.8**±2.8 | **27.3**±2.7 | **32.8**±3.7 | **48.0**±21.7 | **102.3**±5.9 |
| I/133 (TBP-400) | **31.3**±4.1 | **28.8**±3.6 | **28.5**±3.9 | **31.1**±6.5 | **47.1**±16.0 | **102.1**±2.3 |
| I/107 (CZT-136) | **34.1**±6.5 | **31.4**±2.9 | **32.9**±3.6 | **36.8**±5.1 | **86.6**±5.7 | **101.7**±3.0 |

**Table 11: Cell viability measurements on SCC-25 cell line**

| **SCC-25** (tongue, squamous cell carcinoma) cell viability (% of control) | | | | | | |
|---|---|---|---|---|---|---|
| | **300 nM** | **100 nM** | **33.3 nM** | **11.1 nM** | **3.7 nM** | **1.2 nM** |
| I/1 (ONC-212) (ref.) | **37.6**±4.3 | **76.7**±9.9 | **106.7**±9.3 | **105.5**±12.7 | **108.5**±8.6 | **101.3**±6.8 |
| I/3 (CZT-021) (ref.) | **33.1**±1.7 | **34.9**±3.9 | **64.7**±4.6 | **95.1**±5.1 | **101**.**0**±4.9 | **103.8**±2.7 |
| I/6 (TBP-218) (ref.) | **41.5**±3.1 | **42.5**±1.8 | **80.1**±10.1 | **96.2**±4.8 | **101.8**±4.3 | **98.7**±6.6 |
| I/149 (TBP-353) (ref.) | **42.3**±4.7 | **35.4**±2.0 | **40.2**±4.0 | **89.0**±5.5 | **97.5**±7.7 | **99.4**±7.0 |
| I/30 (TBP-301) | **36.3**±2.6 | **32.9**±2.1 | **32.6**±2.0 | **35.0**±4.5 | **78.7**±5.0 | **103.4**±11.6 |
| I/7 (ABB-011) | **39.7**±2.7 | **35.1**±2.2 | **36.5**±3.7 | **45.1**±4.7 | **88.7**±5.6 | **93.6**±5.7 |
| I/124 (TBP-333) | **42.3**±5.2 | **37.5**±1.4 | **34.8**±3.6 | **34.3**±2.0 | **47.2**±5.8 | **89.3**±5.4 |
| I/111 (TBP-272) | **36.4**±0.9 | **32.5**±1.2 | **34.4**±2.7 | **79.0**±11.0 | **98.3**±4.0 | **102.1**±4.0 |
| I/133 (TBP-400) | **40.9**±3.7 | **34.7**±4.9 | **35.5**±1.7 | **72.9**±3.0 | **102.3**±4.2 | **102.3**±3.4 |
| I/107 (CZT-136) | **42.4**±5.7 | **35.4**±5.7 | **41.7**±2.1 | **75.6**±6.8 | **97.3**±3.8 | **94.2**±9.5 |

**Table 12: Cell viability measurements on FaDu cell line**

| **FaDu** (pharynx, squamous cell carcinoma) cell viability (% of control) | | | | | | |
|---|---|---|---|---|---|---|
| | **300 nM** | **100 nM** | **33.3 nM** | **11.1 nM** | **3.7 nM** | **1.2 nM** |
| I/1 (ONC-212) (ref.) | **25.6**±5.3 | **46.9**±5.6 | **97.7 ± 8.3** | **98.8**±4.7 | **102.4** ± 7.4 | **101.7**±2.4 |
| I/3 (CZT-021) (ref.) | **22.4**±5.9 | **26.2**±7.9 | **51.4** ± 25.3 | **100.9**±5.9 | **101.6** ± 3.4 | **98.3**±4.2 |
| I/6 (TBP-218) (ref.) | **25.2**±4.6 | **27.3**±5.2 | **50.7** ± 8.5 | **97.8**±5.2 | **97.9** ± 3.9 | **98.8**±10.4 |
| I/149 (TBP-353) (ref.) | **23.4**±3.6 | **25.0**±5.4 | **27.9** ± 5.1 | **83.1**±9.5 | **98.2** ± 3.2 | **99.5**±4.9 |
| I/30 (TBP-301) | **22.3**±5.2 | **21.4**±4.5 | **22.4** ± 5.4 | **24.7**±3.7 | **69.5** ± 11.0 | **99.4**±5.4 |
| I/7 (ABB-011) | **26.1**±4.5 | **24.0**±3.8 | **24.6** ± 3.5 | **29.1**±5.6 | **80.1** ± 13.3 | **100.8**±6.6 |
| I/124 (TBP-333) | **24.7**±5.0 | **24.5**±4.6 | **23.8** ± 4.1 | **26.1**±6.2 | **45.6** ± 20.7 | **95.3**±7.0 |
| I/111 (TBP-272) | **22.1**±4.8 | **21.7**±5.4 | **24.8** ± 3.6 | **66.8**±12.0 | **97.7** ± 3.9 | **100.7**±2.4 |
| I/133 (TBP-400) | **24.6**±6.8 | **25.3**±6.4 | **26.5** ± 6.4 | **70.7**±13.1 | **100.0** ± 6.2 | **100.3**±5.8 |
| I/107 (CZT-136) | **25.5**±4.3 | **24.6**±3.6 | **27.1** ± 4.0 | **74.3**±4.2 | **98.6** ± 1.9 | **102.2**±7.8 |

**Table 13: Cell viability measurements on EBC-1 cell line**

| **EBC-1** (lung, squamous cell carcinoma) cell viability (% of control) | | | | | | |
|---|---|---|---|---|---|---|
| | **300 nM** | **100 nM** | **33.3 nM** | **11.1 nM** | **3.7 nM** | **1.2 nM** |
| I/1 (ONC-212) (ref.) | **24.2** ± 2.1 | **43.4**±6.8 | **99.2**±0.9 | **110.1**±11.9 | **98.8**±5.8 | **95.3**±2.2 |
| I/3 (CZT-021) (ref.) | **22.2** ± 0.1 | **25.1**±0.2 | **82.6**±6.4 | **96.3**±1.4 | **97.8**±4.0 | **99.7**±0.8 |
| I/6 (TBP-218) (ref.) | **24.1** ± 2.4 | **25.1**±0.3 | **56.6**±0.5 | **101.7**±3.3 | **101.0**±9.5 | **100.2**±1.5 |
| I/149 (TBP-353) (ref.) | **23.6** ± 1.3 | **23.2**±0.7 | **25.8**±1.0 | **89.0**±5.6 | **97.7**±13.2 | **102.7**±15.3 |
| I/30 (TBP-301) | **20.1** ± 0.0 | **21.3**±0.6 | **20.8**±0.5 | **24.8**±1.1 | **76.4**±2.2 | **103.9**±7.1 |
| I/7 (ABB-011) | **23.8** ± 0.6 | **23.2**±0.9 | **23.4**±0.2 | **25.6**±0.1 | **79.8**±4.1 | **101.3**±0.5 |
| I/124 (TBP-333) | **23.5** ± 1.3 | **22.4**±0.5 | **22.6**±1.6 | **25.3**±0.7 | **70.9**±8.3 | **103.3**±1.5 |
| I/111 (TBP-272) | **20.0** ± 0.0 | **20.6**±0.6 | **21.4**±0.5 | **54.0**±6.5 | **94.1**±3.0 | **99.7**±3.8 |
| I/133 (TBP-400) | **22.2** ± 1.3 | **23.2**±0.7 | **23.1**±2.1 | **60.1**±6.7 | **97.1**±6.0 | **100.8**±4.3 |
| I/107 (CZT-136) | **23.5** ± 2.5 | **23.5**±1.0 | **23.7**±1.3 | **70.3**±0.9 | **99.9**±2.5 | **100.0**±5.9 |

**Table 14: Cell viability measurements on MDA-MB-231 cell line**

| **MDA-MB-231** (breast, adenocarcinoma) cell viability (% of control) | | | | | | |
|---|---|---|---|---|---|---|
| | **300 nM** | **100 nM** | **33.3 nM** | **11.1 nM** | **3.7 nM** | **1.2 nM** |
| I/1 (ONC-212) (ref.) | **56.8**±2.1 | **87.9**±4.4 | **98.5**±1.6 | **97.9**±1.1 | **101.0**±4.4 | **99.9** ± 6.2 |
| I/3 (CZT-021) (ref.) | **53.7**±3.1 | **56.5**±3.4 | **84.2**±7.0 | **99.8**±1.8 | **101.4**±1.4 | **99.3** ± 3.4 |
| I/6 (TBP-218) (ref.) | **57.4**±1.7 | **57.7**±3.8 | **91.8**±4.3 | **99.4**±4.1 | **100.5**±5.4 | **98.9** ± 4.7 |
| I/149 (TBP-353) (ref.) | **57.5**±3.8 | **59.1**±3.5 | **63.0**±4.3 | **100.0**±1.3 | **101.6**±4.0 | **100.9** ± 2.2 |
| I/30 (TBP-301) | **52.6**±2.5 | **52.1**±1.8 | **54.6**±1.1 | **59.9**±2.1 | **100.0**±4.5 | **99.8** ± 3.6 |
| I/7 (ABB-011) | **59.4**±3.0 | **58.6**±3.0 | **58.8**±1.9 | **63.6**±4.4 | **99.6**±2.2 | **98.4** ± 2.1 |
| I/124 (TBP-333) | **54.2**±2.4 | **55.9**±5.3 | **56.5**±2.8 | **59.4**±4.5 | **82.4**±7.2 | **100.8** ± 0.9 |
| I/111 (TBP-272) | **52.8**±2.7 | **56.9**±2.2 | **59.8**±3.6 | **98.5**±2.1 | **102.7**±2.8 | **102.2** ± 3.2 |
| I/133 (TBP-400) | **56.4**±3.5 | **58.2**±2.5 | **60.8**±2.4 | **100.4**±2.9 | **101.4**±2.0 | **102.1** ± 2.5 |
| I/107 (CZT-136) | **55.7**±0.9 | **57.3**±1.8 | **59.6**±3.0 | **98.6**±0.8 | **102.1**±3.5 | **100.6** ± 5.8 |

**Table 15: Cell viability measurements on MDA-MB-453 cell line**

| **MDA-MB-453** (breast, carcinoma) cell viability (% of control) | | | | | | |
|---|---|---|---|---|---|---|
| | **300 nM** | **100 nM** | **33.3 nM** | **11.1 nM** | **3.7 nM** | **1.2 nM** |
| I/1 (ONC-212) (ref.) | **32.5**±0.5 | **37.9**±1.3 | **107.4**±12.7 | **110.8** ± 2.3 | **111.0**±7.3 | **104.0**±3.3 |
| I/3 (CZT-021) (ref.) | **29.8**±1.5 | **33.2**±2.0 | **37.3**±0.5 | **97.0** ± 13.3 | **110.3**±7.7 | **109.9**±5.4 |
| I/6 (TBP-218) (ref.) | **32.0**±1.1 | **33.9**±1.3 | **39.8**±1.9 | **103.7** ± 10.4 | **113.6**±9.8 | **113.6**±8.3 |
| I/149 (TBP-353) (ref.) | **31.2**±0.9 | **33.3**±2.6 | **34.1**±2.4 | **57.0** ± 18.5 | **108.6**±13.2 | **110.0**±3.0 |
| I/30 (TBP-301) | **28.9**±1.0 | **31.1**±1.8 | **32.1**±2.0 | **34.8** ± 3.3 | **58.0**±24.4 | **109.5**±10.7 |
| I/7 (ABB-011) | **31.6**±0.9 | **33.1**±1.1 | **34.0**±1.9 | **35.8** ± 1.3 | **55.8**±16.5 | **104.7**±14.5 |
| I/124 (TBP-333) | **30.9**±1.4 | **31.2**±2.2 | **33.0**±1.0 | **33.5** ± 1.8 | **37.9**±3.2 | **86.3**±23.2 |
| I/111 (TBP-272) | **28.8**±0.4 | **30.3**±1.0 | **34.1**±2.4 | **45.7** ± 5.0 | **107.2**±5.9 | **115.8**±7.5 |
| I/133 (TBP-400) | **31.7**±0.5 | **31.9**±1.3 | **35.0**±1.7 | **50.1** ± 10.6 | **108.6**±9.7 | **110.3**±8.8 |
| I/107 (CZT-136) | **32.2**±1.3 | **32.3**±1.3 | **35.2**±2.1 | **47.8** ± 6.2 | **107.2**±9.1 | **108.1**±7.7 |

### Example 22:

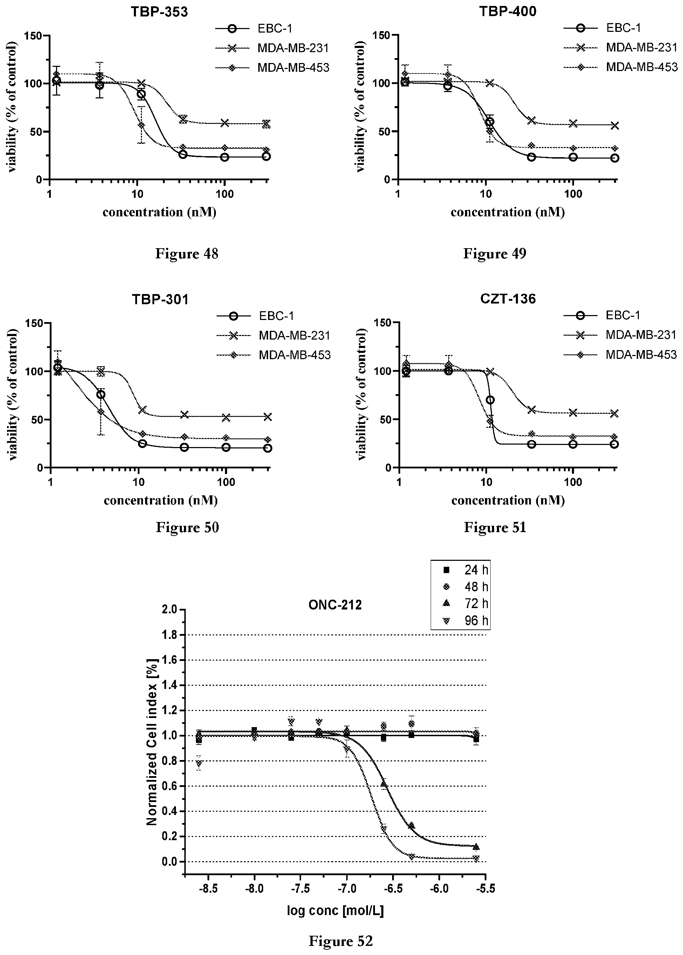

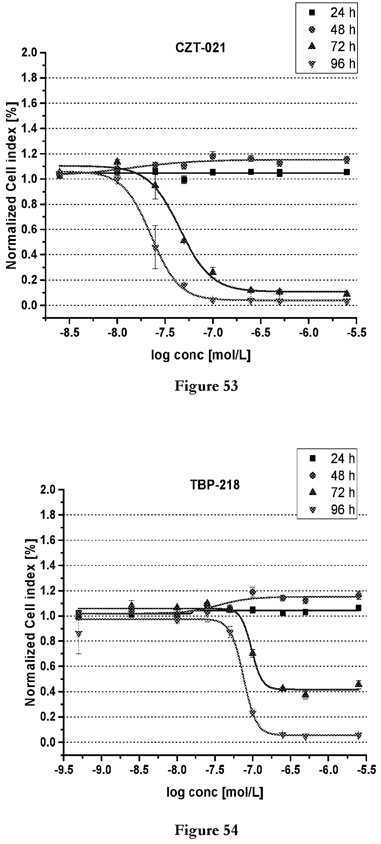

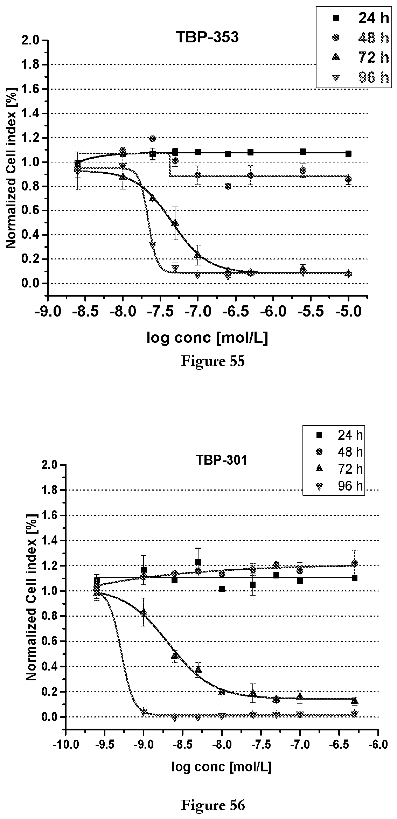

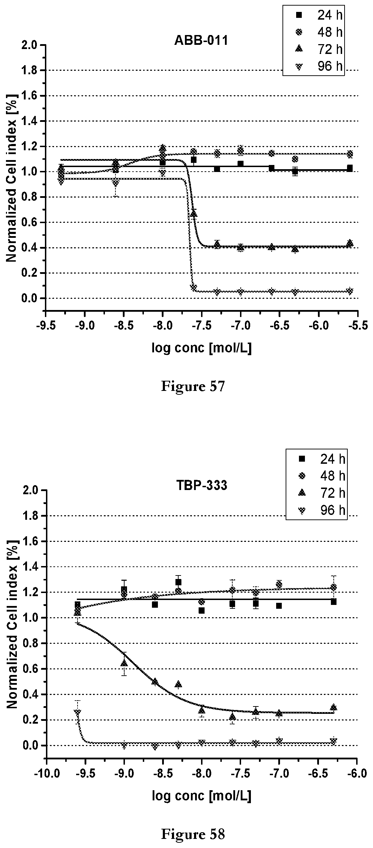

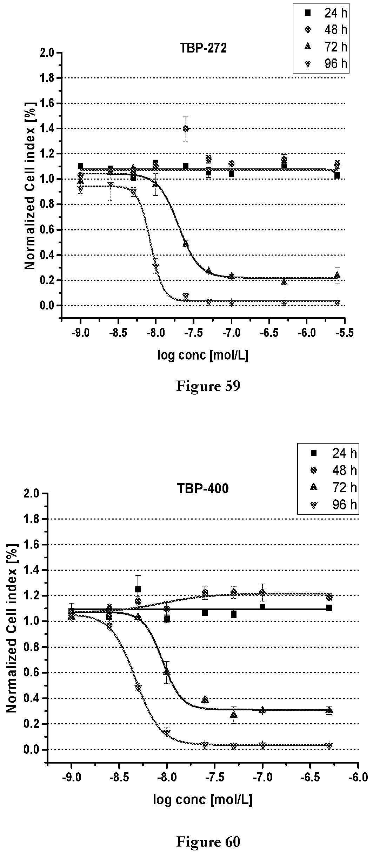

This example demonstrates the cytotoxic effect of some compounds according to the invention on Panc-1 human cell line. The IC₅₀ values were determined in xCELLigence SP instrument (see Figures 52-61). The following table shows the IC₅₀ values obtained after 24 h, 48 h, 72 h and 96 h treating.

**Table 16: Results of cytotoxicity measurements on Panc-1 cell line**

| No. of compound (code) | IC₅₀ (24 h) [nM] | IC₅₀ (48 h) [nM] | IC₅₀ (72 h) [nM] | IC₅₀ (96 h) [nM] |
|---|---|---|---|---|
| I/1 (ONC-212) (ref.) | ND | ND | 274 ± 15.9 | 185 ± 44.4 |
| I/3 (CZT-021) (ref.) | ND | ND | 45.3 ± 3.34 | 22.6 ± 0.856 |
| I/6 (TBP-218) (ref.) | ND | ND | 126 ± 5.96 | 75.5 ± 6.76 |
| I/149 (TBP-353) (ref.) | ND | ND | 45.0 ± 27.3 | 21.8 ± 5.29 |
| I/30 (TBP-301) | ND | ND | 2.08 ± 0.267 | 0.517 ± 0.0232 |
| I/7 (ABB-011) | ND | ND | 27.3 ± 15.4 | 22.1 ± 5.98 |
| I/124 (TBP-333) | ND | ND | 1.31 ± 0.286 | <0.25 |
| I/111 (TBP-272) | ND | ND | 20.1 ± 1.68 | 8.58 ± 0.256 |
| I/133 (TBP-400) | ND | ND | 9.05 ± 0.669 | 4.73 ± 0.109 |
| I/107 (CZT-136) | ND | ND | 102 ± 24.4 | 49.6 ± 17.0 |

| | | | | |
|---|---|---|---|---|
| ND: not detectable | | | | |

### Example 23:

This example demonstrates the antitumoral effects of compounds I/1 (ONC 212 as Reference Compound 2), I/7 (ABB-011), I/124 (TBP-333) and I/107 (CZT-136) on subcutaneously growing MDA-MB-231 tumor xenografts in SCID mice (immunsuprimized mice).

MDA-MB-231 human triple-negative breast cancer xenografts were formed by subcutaneous inoculation of tumor cells into the back of the immunodeficient (SCID) mice. The investigated materials were injected into the animals intraperitoneal every two-three days for 3 weeks.

The experimental results showed that all of the investigated compounds reduced the tumor volume, however, this effect was significant only in the case of I/124 (TBP-333).

### Experimental design:

The tested compounds:

| | |
|---|---|
| I/1 (ONC 212) as reference: | 0.022 mg/animal/treatment; dose: 0.88 mg/kg |
| | 0.036 mg/animal/treatment; dose: 1.466 mg/kg |
| I/7 (ABB-011): | 0.0213 mg/animal/treatment, dose: 0.85 mg/kg |
| | 0.0355 mg/animal/treatment, dose: 1.416 mg/kg |
| I/124 (TBP-333): | 0.022 mg/animal/treatment dose: 0.89 mg/kg |
| | 0.036 mg/animal/treatment; dose: 1.466 mg/kg |
| I/107 (CZT-136): | 0.022 mg/animal/treatement, dose: 0.89 mg/kg |
| | 0.036 mg/animal/treatment; dose: 1.466 mg/kg |

and physiological saline solution with 1% DMSO as control.

Forty SCID mice were included in the experiment. Each animal group was kept in separate cage during the experiments. On each cage, there was an identification card with birth date, the date of tumor cell injection, number and gender of animals. The injection of each substance was written on the identification card. Eight animals per substance were used and identified by ear cutting.

The animals used in these studies were cared for according to the "Guiding Principles for the Care and Use of Animals" based upon the Helsinki declaration, and the studies were approved by the local ethics committee.

Animals were housed in IVC (Individually Ventilated Cages) system in sterilized cages, with a controlled cycle of 12 hours light and 12 hours dark, the light phase being between 07:00h and 19:00h. Temperature and humidity were recorded daily during the whole experiment. Sterilized rodent special quality control diet (VRF1, Special Diets Services Ltd, Witham, UK) and acidified (pH=3) sterilized distilled water were available ad libitum throughout the study periods.

Each batch of diet was delivered with an accompanying certificate of analysis detailing nutritional composition. The health status of the mice was assessed by animal house staff.

MDA-MB-231 human triple-negative breast cancer cells were grown in RPMI-1640 Medium (Sigma Chemical Co., St. Louis, MO), supplemented with 10% fetal bovine serum (Sigma) and 1% penicillin-streptomycin (Sigma) at 37°C in a humidified atmosphere of 5% CO₂. Cells from monolayer culture were detached with 0.02% EDTA (Sigma), washed twice with serum-free medium, and one-cell suspension was inoculated subcutaneously into the back of SCID mice with a number of 1.3x10⁶ cells/animal. When the tumor volumes reached the detectable size (~100-200 mm³, 12 days after tumor cell inoculation) the test products were inoculated intraperitoneal.

All of the test compounds were provided as powder. The powders were dissolved in DMSO. For the final treatment concentration, the compounds were diluted in physiological saline solution 1:100 (1% DMSO).

All of the test compounds were planned to be administered by intraperitoneal injection in a 0.3 ml volume in physiological saline solution with 1% DMSO three times per week. However, during the experiment this schedule was modified: from 07.19 intraperitoneal injection in a 0.5 ml volume every day was executed (Table 17).

**Table 17: Treatment schedule**

| **Treatment schedule** | | | | |
|---|---|---|---|---|
| 2021.06.14 | 2021.06.15 | 2021.06.16 | 2021.06.17 | 2021.06.18 |

| | | **Tumor inoculation*** | Tumor growth | |
|---|---|---|---|---|
| 2021.06.21 | 2021.06.22 | 2021.06.23 | 2021.06.24 | 2021.06.25 |
| | | | | |
| 2021.06.28 | 2021.06.29 | 2021.06.30 | 2021.07.01 | 2021.07.02 |
| | | | | |
| 2021.07.05 | 2021.07.06 | 2021.07.07 | 2021.07.08 | 2021.07.09 |
| First treatment 300 µl ≈0.88 mg/kg | | 300µl ≈0.88 mg/kg | | 300µl ≈0.88 mg/kg |
| 2021.07.12 | 2021.07.13 | 2021.07.14 | 2021.07.15 | 2021.07.16 |
| 300µl ≈0.88 mg/kg | | 300µl ≈0.88 mg/kg | 300µl ≈0.88 mg/kg | 300µl ≈0.88 mg/kg |
| 2021.07.19 | 2021.07.20 | 2021.07.21 | 2021.07.22 | 2021.07.23 |
| 500 µl ≈1.4 mg/kg | 500 µl ≈1.4 mg/kg | 500 µl ≈1.4 mg/kg | 500 µl ≈1.4 mg/kg | **Termination** |

| | | | | |
|---|---|---|---|---|
| *: Number of inoculated cell: 1.3*10⁶ | | | | |

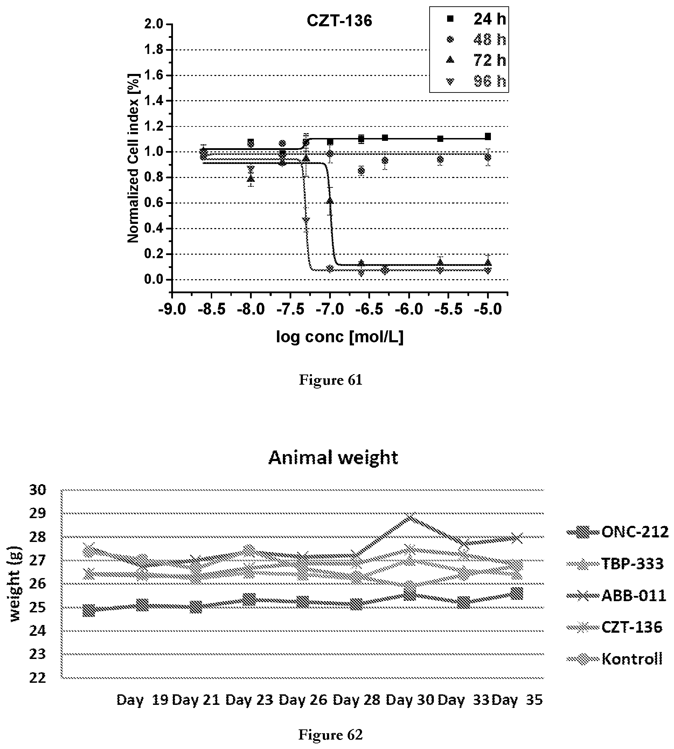

During the experiment weight of the animals and size of the tumors were recorded. The investigated compounds did not cause weight loss (Figure 62).

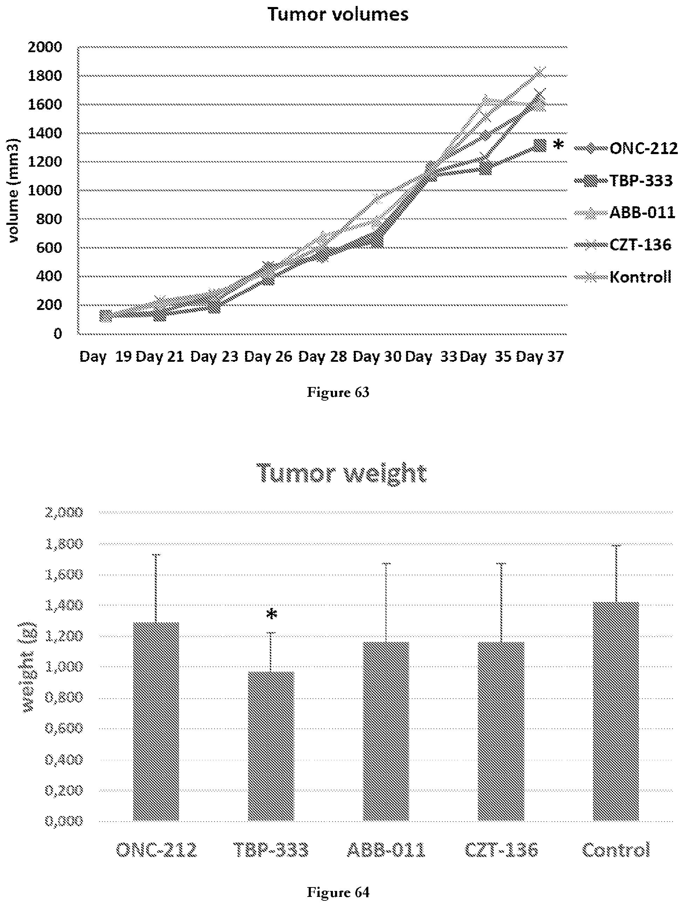

Tumor volume was determined three times a week during the experiment. All of the investigated compounds reduced the tumor growth, but the effect was significant only in the case of I/124 (TBP-333) (Figure 63).

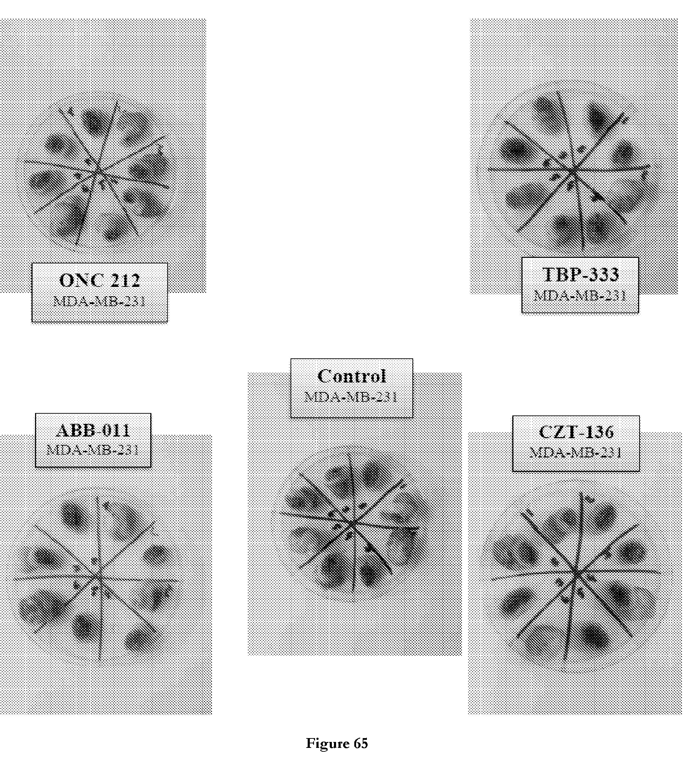

Anti-tumor effect of compounds was evaluated also measuring tumor weight in each group after termination of experiment (Figures 64 and 65). Based on tumor weight we could obtain that the highest, significant inhibition showed I/124 (TBP-333), but all of the investigated compounds decreased the tumor weight in comparison to control.

The experimental results showed that the tested drugs have an antitumor effect, which was shown both in the volumes and in the tumor masses at the end of the experiment, however, this effect was significant only in the case of I/124 (TBP-333). It can be assumed that if the solubility of the materials can be improved, or the amount delivered can be increased in any other way, even more significant effects can be achieved.

Based on the forgoing results it should be noted the following:
As for the effect of the substitution pattern of the benzyl groups attached to 4 and 7 positions of the imipridone skeletone, we have unambiguously established that [7-(3",5"-difluorobenzyl)]-substituted compounds with identical substituent in position 4' (in benzyl group on N-4 atom) exhibit significantly stronger antiproliferative activity than their [7-(3"-fluorobenzyl)]-substituted counterparts (I/30 (TBP-301) vs. I/3 (CZT-021 as reference), I/7 (ABB-011) vs. I/6 (TBP-218 as reference) and I/124 (TBP-333) vs. I/149 (TBP-353 as reference)) as clearly reflected from the IC₅₀ values (Table 16) and cell viability data demonstrated in Example 21 obtained by *in vitro* assays performed on human malignant cell lines by different methods. It must be emphasized that even all [7-(3"-fluorobenzyl)]-imipridones including our original azidobenzyl derivatives are far more active than ONC-212 (Reference Compound 2) in each comparative *in vitro* test of this invention. The original monoazido derivatives also demonstrated an exceptionally outstanding activity profile against the investigated cell lines. It is of pronounced significance and novelty in efficiency profile that [7-(3"-azidobenzyl)]imipridones (I/111 (TBP-272) and 1/133 (TBP-400)) show higher degree of cytotoxicity than the corresponding [7-(3"-fluorobenzyl)]imipridones I/3 (CZT-021 as reference) and I/149 (TBP-353 as reference), respectively, as demonstrated by the IC₅₀ values measured on PANC-1 cell line (Table 16). This tendency was also manifested in the assays carried out by CellTiter-Glo^{®} luminescent cell viability assay on a series of cell lines (see Example 21).

Finally, taking all the measured IC₅₀ and cell viability data into account it can be stated that at *in vitro* level imipridone 4-(4-chlorobenzyl)-7-(3,5-difluorobenzyl)-2,4,6,7,8,9-hexahydroimidazo[1,2-*a*]pyrido[3,4*-e*]pyrimidin-5(1*H*)-one (I/124 (TBP-333)), prepared and investigated by us for the first time, is the most potent representative of the members of small molecule anticancer imipridone family phisically identified so far.

### INDUSTRIAL APPLICABILITY

The compounds of the invention has anticancer activity, thus these compounds are suitable for use in medicine.

### REFERENCES CITED IN THE DESCRIPTION:

[1] Arrillaga-Romany, I.; Andrew S. Chi, A,S.; Allen, J.E.; Oster, W.; Patrick Y. Wen P.Y.; Batchelo, T.T. A phase 2 study of the first imipridone ONC201, a selective DRD2 antagonist for oncology, administered every three weeks in recurrent glioblastoma. Oncotarget. 2017, 8, 79298-79304. https://doi.org/10.18632/oncotarget.17837
[2] Prabhu, V.V.; Allen, J.E.; Dicker, D.T.; El-Deiry, W.S. Small-Molecule ONC201/TIC10 Targets Chemotherapy-Resistant Colorectal Cancer Stem-like Cells in an Akt/Foxo3a/TRAIL-Dependent Manner. Cancer Res. 2015, 75, 1423-1432, doi:10.1158/0008-5472.CAN-13-3451.
[3] Kline, C.L.; Van den Heuvel, A.P.; Allen, J.E.; Prabh, V.V.; Dicker, D.T.; El-Deiry, W.S. ONC201 kills solid tumor cells by triggering an integrated stress response dependent on ATF4 activation by specific eIF2α kinases. Sci. Signal 2016, 9, ra18, doi:10.1126/scisignal.aac4374.
[4] Allen, J.E.; Krigsfeld, G.; Mayes, P.A.; Patel, L.; Dicker, D.T.; Patel, A.S.; Dolloff, N.G.; Messaris, E.; Scata, K.A.; Wang, W.; et al. Dual inactivation of Akt and ERK by TIC10 signals Foxo3a nuclear translocation, TRAIL gene induction, and potent antitumor effects. Sci. Transl. Med. 2013, 5, 171ra17, doi:10.1126/scitranslmed.3004828.
[5] Wagner, J.; Kline, C.L.; Pottorf, R.S.; Nallaganchu, B.R.; Olson, G.L.; Dicker, D.T.; Allen, J.E.; El-Deiry, W.S. The angular structure of ONC201, a TRAIL pathway-inducing compound, determines its potent anti-cancer activity. Oncotarget 2014, 5, 12728-12737, doi:10.18632/oncotarget.2890.
[6] Zhang, Q.; Wang, H.; Ran, L.; Zhang, Z.; Jiang, R. The preclinical evaluation of TIC10/ONC201 as an anti-pancreatic cancer agent. Biochem. Biophys. Res. Commun. 2016, 476, 260-266, doi:10.1016/j.bbrc.2016.05.106.
[7] Jin, Z.Z.; Wang, W.; Fang, D.L.; Jin, Y.J. mTOR inhibition sensitizes ONC201-induced anti-colorectal cancer cell activity. Biochem. Biophys. Res. Commun. 2016, 478, 1515-1520, doi:10.1016/j.bbrc.2016.08.126.
[8] Feng, Y.; Zhou, J.; Li, Z.; Jiang, Y.; Zhou, Y. Small Molecular TRAIL Inducer ONC201 Induces Death in Lung Cancer Cells: A Preclinical Study. PLoS ONE 2016, 11, e0162133, doi:10.1371/journal.pone.0162133.
[9] Stein, M.N.; Bertino, J.R.; Kaufman, H.L.; Mayer, T.; Moss, R.; Silk, A.; Chan, N.; Malhotra, J.; Rodriguez, L.; Aisner, J.; et al. First-in-human Clinical Trial of Oral ONC201 in Patients with Refractory Solid Tumors. Clin. Cancer Res. 2017, doi:10.1158/1078-0432.ccr-16-2658.
[10] Wagner, J.; Kline, C.L.; Ralff, M.D.; Lev, A.; Lulla, A.; Zhou, L.; Olson, G.L.; Nallaganchu, B.R.; Benes, C.H.; Allen, J.E.; et al. Preclinical evaluation of the imipridone family, analogs of clinical stage anti-cancer small molecule ONC201, reveals potent anti-cancer effects of ONC212. Cell Cycle 2017 16, 1790-1799, doi:10.1080/15384101.2017.1325046.
[11] Lev, A.; Lulla, A.R.; Wagner, J.; Ralff, M.D.; Kiehl, J.B.; Zhou, Y.; Benes, C.H.; Prabhu, V.V.; Oster, W.; Astsaturov, I.; et al. Anti-pancreatic cancer activity of ONC212 involves the unfolded protein response (UPR) and is reduced by IGF1-R and GRP78/BIP. Oncotarget 2017, 8, 81776-81793, doi:10.18632/oncotarget.20819.
[12] Graves, P. R. et. al. Mitochondrial Protease ClpP is a Target for the Anticancer Compounds ONC201 and Related Analogues. ACS Chem. Biol., 2019, 14, 1020-1029. https://doi.org/10.1021/acschembio.9b00222.
[13] Xu, Ruo; Liu, Yunyong. Imidazopyrimidone compounds as antitumor agents and their preparation, pharmaceutical compositions and use in the treatment of cancer. PCT Int. Appl. (2016), WO 2016184437 A1 20161124-
[14] Allen, Joshua E.; Stogniew, Martin; Prabhu, Varun Vijay, Preparation of imipridone compounds as G protein-coupled receptor modulators for treatment of cancer, infection, and psychiatric disorders. PCT Int. Appl. (2017), WO 2017132661 A2 20170803
[15] Allen, Joshua E.; Stogniew, Martin; Prabhu, Varun Vijay, Imipridones for treatment of gliomas. U.S. Pat. Appl. Publ. (2018), US 20180221375 A1 20180809.
[16] Iwanowicz, Edwin J. Preparation of hexahydroimidazo pyridopyrimidinones as protein kinase regulators useful in mono- and combination therapy of diseases. PCT Int. Appl. (2018), WO 2018031987 A1 20180215
[17] Voltan, R.; Secchiero, P.; Casciano, F.; Milani, D.; Zauli, G.; Tisato, V. Redox signalling and oxidative stress: Cross talk with TNF-related apoptosis inducing ligand activity. International Journal of Biochemistry & Cell Biology 2016, 81, 364-374, https://doi.org/10.1016/j.biocel.2016.09.019.
[18] Tamura, H.; Miwa, M. DNA Cleaving Activity and Cytotoxic Activity of Ferricenium Cations Chem. Lett., 1997, 26, 1177-1178, https://dot.org/10.1246/cl.1997.1177.
[19] Osella, D.; Ferrali, M.; Zanello, P.; Laschi, F.; Fontani, M.; Nervi, C. Cavigiolio, G. On the mechanism of the antitumor activity of ferrocenium derivatives. Inorg. Chim. Acta, 2000, 306, 42-48, https://doi.org/10.1016/S0020-1693(00)00147-X.
[20] Simon, H.U.; Haj-Yehia, A.; Levi-Schaffer, F. Role of reactive oxygen species (ROS) in apoptosis induction. Apoptosis 2000 5: 415-418. https://doi.org/10.1023/A:1009616228304.
[21] Bárány, P.; Oláh, R.S.; Kovács, I.; Czuczi, T.; Szabó, C.L.; Takács, A.; Lajkó, E.; Láng, O.; Kőhidai, L.; Schlosser,G.;etal. Ferrocene-Containing Imipridone (ONC201) Hybrids: Synthesis, DFT Modelling, In Vitro Evaluation, and Structure-Activity Relationships. Molecules, 2018, 23, 2248. https://doi.org/10.3390/molecules23092248.
őhidai, L. Investigation on chemotactic drug targeting (chemotaxis and adhesion) inducer e_ect of GnRH-III derivatives in Tetrahymena and human leukemia cell line. J. Pept. Sci. 2013, 19, 46-58.
[23] Urcan, E.; Haertel, U.; Styllou, M.; Hickel, R.; Scherthan, H.; Reichl, F.X. Real-time xCELLigence impedance analysis of the cytotoxicity of dental composite components on human gingival fibroblasts. Dent. Mater. 2010, 26, 51-58.
[24] Bárány, P.; Oláh, R.S.; Kovács, I.; Czuczi, T.; Szabó, C.L.; Takács, A.; Lajkó, E.; Láng, O.; Kőhidai, L.; Schlosser, G.; Mez6, G.; Hudecz, G.; Csámpai, A. Ferrocene-Containing Imipridone (ONC201) Hybrids: Synthesis, DFT Modelling, In Vitro Evaluation, and Structure-Activity Relationships. Molecules 2018, 23, 2248.
[25] Lajkó, E.; Spring, S.; Hegedüs, R.; Biri-Kovács, B.; Ingebrandt, S.; Mez6, G.; Kőhidai, L. Comparative cell biological study of in vitro antitumor and antimetastatic activity on melanoma cells of GnRH-III-containing conjugates modified with short-chain fatty acids. Beilstein J. Org. Chem. 2018, 26, 2495-2509.
[26] Slater, T.F.; Sawyer, B.; Strauli, U. Studies on succinate-tetrazolium reductase systems: III. Points of coupling of four different tetrazolium salts III. Points of coupling of four different tetrazolium salts. Biochim Biophys Acta. 1963, 77, 383-393.
[27] Mosmann, T.J. Rapid colorimetric assay for cellular growth and survival: application to proliferation and cytotoxicity assays.Immunol. Methods. 1983, 65, 55-63.
[28] Liu, Y.B.; Peterson, D.A.; Kimura, H.; Schubert, D.J. Mechanism of Cellular 3-(4,5-Dimethylthiazol-2-yl)-2,5-Diphenyltetrazolium Bromide (MTT) Reduction. Neurochem. 1997, 69, 581-593.
[29] Altman, F.P. Tetrazolium salts and formazans. Prog. Hystochem. Cytochem. 1976, 9, 1-56
[30] Denizot, F.; Lang, R. Rapid colorimetric assay for cell growth and survival. Modifications to the tetrazolium dye procedure giving improved sensitivity and reliability. J. Immunol Methods. 1986, 89, 271-277.

## Claims

1. Compounds of formula (**I**) 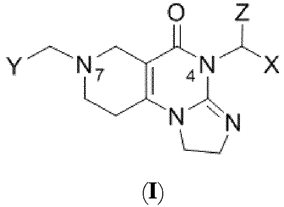 wherein
**if Y is phenyl and Z is H,** then X is 3-azidophenyl (Compound I/102) or 4-azidophenyl (Compound I/104); or
**if Y is 3-fluorophenyl and Z is H,** then X is 3-azidophenyl (Compound I/105) or 4-azidophenyl (Compound I/106); or
**if Y is 3,5-difluorophenyl and Z is H,** then X is 3-(aminomethyl)phenyl (Compound I/61), 4-azidophenyl (Compound I/107) or 3-azidophenyl (Compound I/127); or
**if Y is 4-azidophenyl and Z is H,** then X is 2-methylphenyl (Compound I/101); 3-fluorophenyl (Compound I/108), 4-fluorophenyl (Compound I/109), 4-(trifluoromethyl)phenyl (Compound I/110), 3-fluoro-4-(trifluoromethyl)phenyl (Compound I/114), 4-iodophenyl (Compound I/115), 3,4,5-trimetoxy-phenyl (Compound I/116), 4-azidophenyl (Compound I/117), 3-azetidinyl (Compound I/118) or 4-piperidinyl (Compound I/119); or
**if Y is 3-azidophenyl and Z is H,** then X is 2-methylphenyl (Compound I/103), 4-(trifluoromethyl)phenyl (Compound I/111), 3-fluoro-4-(trifluoromethyl)phenyl (Compound I/112), 4-iodophenyl (Compound I/113) or 4-chlorophenyl (Compound I/133); or
**if Y is 3,5-diazidophenyl and Z is H,** then X is 4-chlorophenyl (Compound I/138) or 4-(trifluromethyl)phenyl (Compound I/139); or
or a pharmaceutically acceptable salt thereof.

2. The compounds according to Claim 1, wherein
**if Y is 3-fluorophenyl and Z is H,** then X is 4-azidophenyl (Compound I/106); or
**if Y is 3,5-difluorophenyl and Z is H,** then X is 4-azidophenyl (Compound I/107); or
**if Y is 3-azidophenyl and Z is H,** then X is 4-(trifluoromethyl)phenyl (Compound I/111) or 4-chlorophenyl (Compound I/133); or
**if Y is 3,5-diazidophenyl and Z is H,** then X is 4-chlorophenyl (Compound I/138) or 4-(trifluromethyl)phenyl (Compound I/139);
or a pharmaceutically acceptable salt thereof.

3. The compounds according to Claim 2, wherein
**if Y is 3,5-difluorophenyl and Z is H,** then X is 4-azidophenyl (Compound I/107); or
**if Y is 3-azidophenyl and Z is H,** then X is 4-(trifluoromethyl)phenyl (Compound I/111) or 4-chlorophenyl (Compound I/133);
or a pharmaceutically acceptable salt thereof.

4. The compounds of formula (**I**) 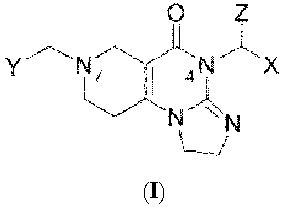 wherein
**if Y is 3,5-difluorophenyl and Z is H,** then X is 4-fluorophenyl (Compound I/7), 4-(trifluoromethyl)phenyl (Compound I/30), 4-bromophenyl (Compound I/39) or 4-chlorophenyl (Compound I/124); or
**if Y is 3,5-difluorophenyl and Z is hydroxymethyl,** then X is 4-chlorophenyl (racemic) (Compound I/128), 4-fluorophenyl (R-enantiomer) (Compound I/129 (R)) or enantiomers, mixture of enantiomers, or a pharmaceutically acceptable salt thereof for use as a medicament.

5. The compounds of formula (**I**) 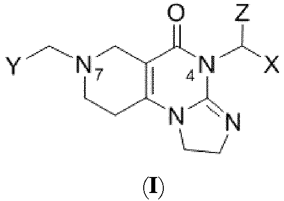 wherein
**if Y is 3,5-difluorophenyl and Z is H,** then X is 4-fluorophenyl (Compound I/7), 4-(trifluoromethyl)phenyl (Compound I/30), 4-bromophenyl (Compound I/39) or 4-chlorophenyl (Compound I/124); or
**if Y is 3,5-difluorophenyl and Z is hydroxymethyl,** then X is 4-chlorophenyl (racemic) (Compound I/128), 4-fluorophenyl (R-enantiomer) (Compound I/129 (R));
or enantiomers, mixture of enantiomers, or a pharmaceutically acceptable salt thereof for use in the treatment of cancer.

6. The compounds for use according to the Claim 5, wherein the cancer is selected form the group consisting of prostate carcinoma, pancreatic cancer, lung carcinoma, breast cancer, glioma, cancers of head and neck, colon cancer, skin cancer.

7. The compounds for use according to Claims 4 to 6, wherein
**if Y is 3,5-difluorophenyl and Z is H,** then X is 4-fluorophenyl (Compound I/7), 4-(trifluoromethyl)phenyl (Compound I/30), 4-bromophenyl (Compound I/39) or 4-chlorophenyl (Compound I/124); or
or a pharmaceutically acceptable salt thereof.

8. The compounds for use according to Claims 4 to 6, wherein
**if Y is 3,5-difluorophenyl and Z is H,** then X is 4-fluorophenyl (Compound I/7), 4-(trifluoromethyl)phenyl (Compound I/30) or 4-chlorophenyl (Compound I/124);
or a pharmaceutically acceptable salt thereof.

9. The compounds according to the Claims 1-3 for use as a medicament.

10. The compounds according to the Claims 1-3 for use in the treatment of cancer.

11. The compounds for use according to the Claim 10, wherein the cancer is selected form the group consisting of prostate carcinoma, pancreatic cancer, lung carcinoma, breast cancer, glioma, cancers of head and neck, colon cancer, skin cancer.

## Patentansprüche

1. Verbindungen der Formel (I) 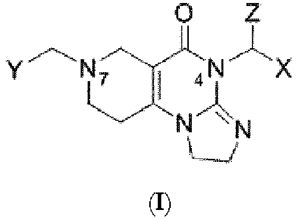 wobei,
wenn Y für Phenyl und Z für H steht, X dann für 3-Azidophenyl steht (Verbindung 1/102) oder für 4-Azidophenyl steht (Verbindung 1/104); oder,
wenn Y für 3-Fluorphenyl und Z für H steht, X dann für 3-Azidophenyl steht (Verbindung 1/105) oder für 4-Azidophenyl steht (Verbindung I/106); oder,
wenn Y für 3,5-Difluorphenyl und Z für H steht, X dann für 3-(Aminomethyl)phenyl steht (Verbindung 1/61), für 4-Azidophenyl steht (Verbindung I/107) oder für 3-Azidophenyl steht (Verbindung I/127); oder,
wenn Y für 4-Azidophenyl und Z für H steht, X dann für 2-Methylphenyl steht (Verbindung I/101), für 3-Fluorphenyl steht (Verbindung I/108), für 4-Fluorphenyl steht (Verbindung I/109), für 4-(Trifluormethyl)phenyl steht (Verbindung I/110), für 3-Fluor-4-(trifluormethyl)phenyl steht (Verbindung 1/114), für 4-Iodphenyl steht (Verbindung 1/115), für 3,4,5-Trimetoxyphenyl steht (Verbindung I/116), für 4-Azidophenyl steht (Verbindung 1/117), für 3-Azetidinyl steht (Verbindung 1/118) oder für 4-Piperidinyl steht (Verbindung 1/119); oder,
wenn Y für 3-Azidophenyl und Z für H steht, X dann für 2-Methylphenyl steht (Verbindung 1/103), für 4-(Trifluormethyl)phenyl steht (Verbindung 1/111), für 3-Fluor-4-(trifluormethyl)phenyl steht (Verbindung I/112), für 4-Iodphenyl steht (Verbindung 1/113) oder für 4-Chlorphenyl steht (Verbindung 1/133); oder,
wenn Y für 3,5-Diazidophenyl und Z für H steht, X dann für 4-Chlorphenyl steht (Verbindung 1/138) oder für 4-(Trifluormethyl)phenyl steht (Verbindung 1/139); oder oder ein pharmazeutisch unbedenkliches Salz davon.

2. Verbindungen nach Anspruch 1, wobei,
wenn Y für 3-Fluorphenyl und Z für H steht, X dann für 4-Azidophenyl steht (Verbindung 1/106); oder,
wenn Y für 3,5-Difluorphenyl und Z für H steht, X dann für 4-Azidophenyl steht (Verbindung 1/107); oder,
wenn Y für 3-Azidophenyl und Z für H steht, X dann für 4-(Trifluormethyl)phenyl steht (Verbindung 1/111) oder für 4-Chlorphenyl steht (Verbindung 1/133) steht; oder wenn Y für 3,5-Diazidophenyl und Z für H steht, X dann für 4-Chlorphenyl steht (Verbindung 1/138) oder für 4-(Trifluormethyl)phenyl steht (Verbindung 1/139);
oder ein pharmazeutisch unbedenkliches Salz davon.

3. Verbindungen nach Anspruch 2, wobei,
wenn Y für 3,5-Difluorphenyl und Z für H steht, X dann für 4-Azidophenyl steht (Verbindung 1/107); oder,
wenn Y für 3-Azidophenyl und Z für H steht, X dann für 4-(Trifluormethyl)phenyl steht (Verbindung 1/111) oder für 4-Chlorphenyl steht (Verbindung 1/133);
oder ein pharmazeutisch unbedenkliches Salz davon.

4. Verbindungen der Formel (I) 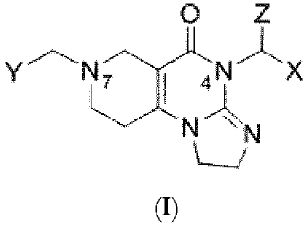 wobei,
wenn Y für 3,5-Difluorphenyl und Z für H steht, X dann für 4-Fluorphenyl (Verbindung I/7) steht, für 4-(Trifluormethyl)phenyl steht (Verbindung 1/30), für 4-Bromphenyl steht (Verbindung I/39) oder für 4-Chlorphenyl steht (Verbindung I/124); oder,
wenn Y für 3,5-Difluorphenyl und Z für Hydroxymethyl steht, X dann für 4-Chlorphenyl (racemisch) steht (Verbindung 1/128), für 4-Fluorphenyl (R-Enantiomer) steht (Verbindung I/129 (R))), oder Enantiomere, Enantiomerengemisch oder ein pharmazeutisch unbedenkliches Salz davon zur Verwendung als Arzneimittel.

5. Verbindungen der Formel (I) 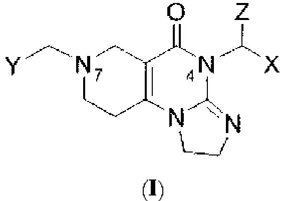 wobei,
wenn Y für 3,5-Difluorphenyl und Z für H steht, X dann für 4-Fluorphenyl (Verbindung 1/7) steht, für 4-(Trifluormethyl)phenyl steht (Verbindung 1/30), für 4-Bromphenyl steht (Verbindung I/39) oder für 4-Chlorphenyl steht (Verbindung 1/124); oder,
wenn Y für 3,5-Difluorphenyl und Z für Hydroxymethyl steht, X dann für 4-Chlorphenyl (racemisch) steht (Verbindung I/128), für 4-Fluorphenyl (R-Enantiomer) steht (Verbindung I/129 (R));
oder Enantiomere, Enantiomerengemisch oder ein pharmazeutisch unbedenkliches Salz davon zur Verwendung bei der Behandlung von Krebs.

6. Verbindungen zur Verwendung nach Anspruch 5, wobei der Krebs aus der Gruppe bestehend aus Prostatakarzinom, Bauchspeicheldrüsenkrebs, Lungenkarzinom, Brustkrebs, Gliom, Krebs im Kopf- und Halsbereich, Darmkrebs, Hautkrebs ausgewählt ist.

7. Verbindungen zur Verwendung nach Anspruch 4 bis 6, wobei,
wenn Y für 3,5-Difluorphenyl und Z für H steht, X dann für 4-Fluorphenyl (Verbindung 1/7) steht, für 4-(Trifluormethyl)phenyl steht (Verbindung 1/30), für 4-Bromphenyl steht (Verbindung I/39) oder für 4-Chlorphenyl steht (Verbindung 1/124); oder,
oder ein pharmazeutisch unbedenkliches Salz davon.

8. Verbindungen zur Verwendung nach Anspruch 4 bis 6, wobei,
wenn Y für 3,5-Difluorphenyl und Z für H steht, X dann für 4-Fluorphenyl steht (Verbindung I/7), für 4-(Trifluormethyl)phenyl steht (Verbindung 1/30) oder für 4-Chlorphenyl steht (Verbindung 1/124);
oder ein pharmazeutisch unbedenkliches Salz davon.

9. Verbindungen nach Anspruch 1-3 zur Verwendung als Arzneimittel.

10. Verbindungen nach Anspruch 1-3 zur Verwendung bei der Behandlung von Krebs.

11. Verbindungen zur Verwendung nach Anspruch 10, wobei der Krebs aus der Gruppe bestehend aus Prostatakarzinom, Bauchspeicheldrüsenkrebs, Lungenkarzinom, Brustkrebs, Gliom, Krebserkrankungen im Kopf- und Halsbereich, Darmkrebs, Hautkrebs ausgewählt ist.

## Revendications

1. Composés de formule (**I**) 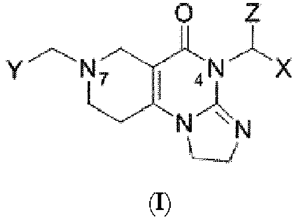 dans lesquels
**si Y est phényle et Z est H,** alors X est 3-azidophényle (Composé I/102) ou 4-azidophényle (Composé I/104) ; ou **si Y est 3-fluorophényle et Z est H,** alors X est 3-azidophényle (Composé I/105) ou 4-azidophényle (Composé I/106) ; ou
**si Y est 3,5-difluorophényle et Z est H,** alors X est 3-(aminométhyl)phényle (Composé I/61), 4-azidophényle (Composé I/107) ou 3-azidophényle (Composé I/127) ; ou **si Y est 4-azidophényle et Z est H,** alors X est 2-méthylphényle (Composé I/101) ; 3-fluorophényle (Composé I/108), 4-fluorophényle (Composé I/109), 4-(trifluorométhyl)phényle (Composé I/110), 3-fluoro-4-(trifluorométhyl)phényle (Composé I/114), 4-iodophényle (Composé I/115), 3,4,5-triméthoxy-phényle (Composé I/116), 4-azidophényle (Composé I/117), 3-azétidinyle (Composé I/118) ou 4-pipéridinyle (Composé I/119) ; ou
**si Y est 3-azidophényle et Z est H,** alors X est 2-méthylphényle (Composé I/103), 4-(trifluorométhyl)phényle (Composé I/111), 3-fluoro-4-(trifluorométhyl)phényle (Composé I/112), 4-iodophényle (Composé I/113) ou 4-chlorophényle (Composé I/133) ; ou
**si Y est 3,5-diazidophényle et Z est H,** alors X est 4-chlorophényle (Composé I/138) ou 4-(trifluorométhyl)phényle (Composé I/139) ; ou
ou sel pharmaceutiquement acceptable correspondant.

2. Composés selon la Revendication 1, dans lesquels **si Y est 3-fluorophényle et Z est H,** alors X est 4-azidophényle (Composé I/106) ; ou
**si Y est 3,5-difluorophényle et Z est H,** alors X est 4-azidophényle (Composé I/107) ; ou
**si Y est 3-azidophényle et Z est H,** alors X est 4-(trifluorométhyl)phényle (Composé I/111) ou 4-chlorophényle (Composé I/133) ; ou
**si Y est 3,5-diazidophényle et Z est H,** alors X est 4-chlorophényle (Composé I/138) ou 4-(trifluorométhyl)phényle (Composé I/139) ;
ou sel pharmaceutiquement acceptable correspondant.

3. Composés selon la Revendication 2, dans lesquels **si Y est 3,5-difluorophényle et Z est H,** alors X est 4-azidophényle (Composé I/107) ; ou
**si Y est 3-azidophényle et Z est H,** alors X est 4-(trifluorométhyl)phényle (Composé I/111) ou 4-chlorophényle (Composé I/133) ;
ou sel pharmaceutiquement acceptable correspondant.

4. Composés de formule **(I)** 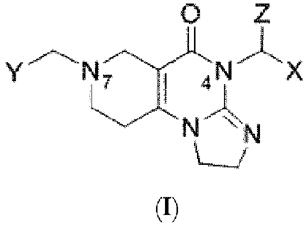 dans lesquels
**si Y est 3,5-difluorophényle et Z est H,** alors X est 4-fluorophényle (Composé I/7), 4-(trifluorométhyl)phényle (Composé I/30), 4-bromophényle (Composé I/39) ou 4-chlorophényle (Composé I/124) ; ou
**si Y est 3,5-difluorophényle et Z est hydroxyméthyle,** alors X est 4-chlorophényle (racémique) (Composé I/128), 4-fluorophényle (énantiomère R) (Composé I/129 (R)) ou énantiomères, mélange d'énantiomères, ou sel pharmaceutiquement acceptable correspondant pour une utilisation comme médicament.

5. Composés de formule (**I**) 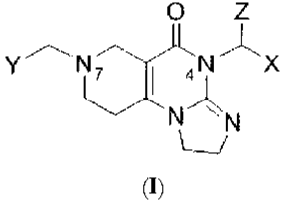 dans lesquels
**si Y est 3,5-difluorophényle et Z est H,** alors X est 4-fluorophényle (Composé I/7), 4-(trifluorométhyl)phényle (Composé I/30), 4-bromophényle (Composé I/39) ou 4-chlorophényle (Composé I/124) ; ou
**si Y est 3,5-difluorophényle et Z est hydroxyméthyle,** alors X est 4-chlorophényle (racémique) (Composé I/128), 4-fluorophényle (énantiomère R) (Composé I/129 (R)) ; ou énantiomères, mélange d'énantiomères, ou sel pharmaceutiquement acceptable correspondant pour une utilisation dans le traitement d'un cancer.

6. Composés pour une utilisation selon la Revendication 5, dans lesquels le cancer est choisi dans le groupe constitué par un carcinome de la prostate, un cancer du pancréas, un carcinome du poumon, un cancer du sein, un gliome, des cancers de la tête et du cou, un cancer du côlon, un cancer de la peau.

7. Composés pour une utilisation selon les Revendications 4 à 6, dans lesquels
**si Y est 3,5-difluorophényle et Z est H,** alors X est 4-fluorophényle (Composé I/7), 4-(trifluorométhyl)phényle (Composé I/30), 4-bromophényle (Composé I/39) ou 4-chlorophényle (Composé I/124) ; ou
ou sel pharmaceutiquement acceptable correspondant.

8. Composés pour une utilisation selon les Revendications 4 à 6, dans lesquels
**si Y est 3,5-difluorophényle et Z est H,** alors X est 4-fluorophényle (Composé I/7), 4-(trifluorométhyl)phényle (Composé I/30) ou 4-chlorophényle (Composé I/124) ;
ou sel pharmaceutiquement acceptable correspondant.

9. Composés selon les Revendications 1 à 3 pour une utilisation comme médicament.

10. Composés selon les Revendications 1 à 3 pour une utilisation dans le traitement d'un cancer.

11. Composés pour une utilisation selon la Revendication 10, dans lesquels le cancer est choisi dans le groupe constitué par un carcinome de la prostate, un cancer du pancréas, un carcinome du poumon, un cancer du sein, un gliome, des cancers de la tête et du cou, un cancer du côlon, un cancer de la peau.
